# EUROPEAN PATENT APPLICATION

(11) **EP 2 620 429 A1**
(43) Date of publication of application: **31.07.2013**
(21) Application number: 13165200.0
(22) Date of filing: 15.02.2008
(51) Int. Cl.: C07D 207/46, C07D 209/44, C07D 211/94, C07D 405/12, C07D 409/12, C07D 413/06, C07D 417/12, C07D 417/14, A61K 31/4535, A61P 17/18

(54) **Hydroxylamine compounds and methods of their use**

(30) Priority: 22.02.2007 US 891140 P
(62) Divisional of application: 08730007.5
(71) Applicant: Colby Pharmaceutical Company, San Jose, CA 95138 (US)
(72) Inventor: Patil, Ghanshyam, Deceased (US)
(74) Representative: Lienard, Benoît

(57) **Abstract**

The present disclosure provides compounds that include hydroxylamines of formula I or II, pharmaceutical compositions, and methods for their use. The methods utilize hydroxylamine compounds and/or their pharmaceutical compositions for the treatment of angiogenesis, hepatitis, complement-mediated pathologies, drusen-mediated pathologies, macular degeneration and certain other ophthalmic conditions, inflammation, arthritis, and related diseases and for the inhibition of complement activation.

## Description

### CROSS-REFERENCE TO RELATED APPLICATIONS

This application claims the benefit of U.S. Provisional Application Serial No. 60/891,140 filed February 22, 2007, the disclosure of which is incorporated herein by reference in its entirety.

### FIELD OF THE INVENTION

This invention relates to novel hydroxylamine compounds, pharmaceutical compositions containing such compounds, and uses thereof. More particularly, the present invention relates to novel hydroxylamine compounds that which may have anti-oxidant and/or free radical scavenging properties and which thus may be useful, *inter alia,* for the treatment of various disease states, disorders or conditions.

### BACKGROUND OF THE INVENTION

Various patents and other publications are referenced herein. The contents of each of these patents and publications are incorporated by reference herein, in their entireties. The entire contents of commonly-owned co-pending U.S. Publication Nos. 2004/0002461, 2005/0130906 and 2005/0131025 are incorporated by reference herein.

Angiogenesis is a complex process of new blood vessel development and formation. Angiogenesis occurs in response to specific signals and involves a complex process characterized by infiltration of the basal lamina by vascular endothelial cells in response to angiogenic growth signal(s), degradation of extracellular matrix and migration of the endothelial cells toward the source of the signal(s), and subsequent proliferation and formation of the capillary tube. Blood flow through the newly formed capillary is initiated after the endothelial cells come into contact and connect with a preexisting capillary.

Angiogenesis is highly regulated and involves a balancing between various angiogenic stimulators and inhibitors. Normally, for mature individuals, there is not much new vessel formation, which means that the naturally occurring balance between endogenous stimulators and inhibitors of angiogenesis heavily favors the inhibitors. Rastinejad et al., 1989, Cell 56:345-355. However, there are some instances in which neovascularization occurs under normal physiological conditions, such as wound healing, organ regeneration, embryonic development, and female reproductive processes, but the angiogenesis is stringently regulated and spatially and temporally delimited. On the other hand, under conditions of pathological angiogenesis, such as that characterizing solid tumor growth, these regulatory controls fail.

When the regulatory controls are compromised and unregulated angiogenesis becomes pathologic, this can lead to sustained progression of many neoplastic and non-neoplastic diseases. A number of serious diseases are dominated by abnormal neovascularization and include solid tumor growth and metastases, arthritis, some types of eye disorders, and psoriasis. See, e.g., reviews by Moses et al., 1991, Biotech. 9:630-634; Folkman et al., 1995, N. Engl. J. Med., 333:1757-1763; Auerbach et al., 1985, J. Microvasc. Res. 29:401-411; Folkman, 1985, Advances in Cancer Research, eds. Klein and Weinhouse, Academic Press, New York, pp. 175-203; Patz, 1982, Am. J. Opthalmol. 94:715-743; and Folkman et al., 1983, Science 221:719-725. As with healthy tissue, tumors require blood vessels to sustain the underlying cells. In a number of pathological conditions, the process of angiogenesis can even contribute to the disease state. Indeed, some investigators have suggested that the growth of solid tumors is dependent on angiogenesis. Folkman and Klagsbrun, 1987, Science 235:442-447.

Reactive oxygen species (ROS), such as superoxide and hydrogen peroxide, have been reported to induce angiogenesis *in vivo,* possibly through up-regulation of inducible nitric oxide synthase and increased production of endogenous nitric oxide. Polytarchou & Papadimitriou, 2005, Eur. J. Pharmacol. 510:31-38. ROS have also been reported to stimulate vascular endothelial growth factor (VEGF) release, and mediate activation of a MAP kinase (Mitogen Activated Protein Kinases) signaling pathway for VEGF. Kuroki et al., 1996, J. Clin. Invest. 98:1667-1675; Cho et al., 2001, Am. J. Physiol. Heart Circ. Physiol. 280: H2357-H2363.

Certain antioxidants have also been shown to have angiogenesis inhibiting activity, for example, superoxide dismutase and the nitroxide TEMPOL (4-hydroxy-2,2,6,6-tetramethylpiperidine-N-oxyl radical), but not the reduced product of TEMPOL, the hydroxylamine TEMPOL-H (2,2,6,6-tetramethylpiperidine-1,4-diol). Other anti-angiogenic agents include VEGF antagonists, bFGF antagonists, or nitric oxide synthase (NOS) antagonists, such as N^{ω}-nitro-L-arginine methyl ester (L-NAME) and dexamethasone.

The liver is the largest gland in the body, and plays a vital role in, among other things, digestion, metabolism of carbohydrates, lipids, and proteins, storage of vitamins, minerals, and carbohydrates, production of blood clotting factors, destruction of bacteria in the blood, and detoxification of the body from endogenous and exogenous substances. Given the liver's broad spectrum of functions, diseases and pathologies of the liver can have wide-ranging systemic effects on the body. One such pathology is hepatitis.

Hepatitis is a generalized term for liver inflammation. Liver inflammation can be chronic or acute, and affects millions of individuals worldwide. The majority of these cases are classified as infectious hepatitis, meaning that they are capable of transmission to others. Infectious hepatitis is typically caused by viruses, most commonly the hepatitis A (HAV), hepatitis B (HBV), and hepatitis C (HCV) viruses. Other sources of infectious hepatitis include the hepatitis D virus (HDV), hepatitis E virus (HEV), and the putative hepatitis F and G viruses, as well as bacteria and other common viruses such as cytomegalovirus, Epstein-Barr virus, herpes simplex virus (HSV), and Varicella-Zoster virus, among others.

Hepatitis can also be classified as non-infectious, meaning that it is not capable of transmission to others. Examples of non-infectious hepatitis include alcoholic hepatitis, toxic/drug-induced hepatitis, autoimmune hepatitis, and granulomatus hepatitis. Alcoholic hepatitis can arise from excessive consumption of alcoholic beverages. Toxic/drug-induced hepatitis is the product of exposure to a toxin, drug, or chemical. Examples of common toxins that induce toxic/drug-induced hepatitis are aflatoxin or amanitin (from poisonous mushrooms). Autoimmune hepatitis results primarily from a cell-mediated (cytotoxic T cell) attack on liver tissue. Granulomatus hepatitis is characterized by an abnormal accumulation of white blood cells in the liver.

Cirrhosis of the liver results from damage to liver cells from toxins, inflammation, metabolic derangements and other causes. Damaged and dead liver cells are replaced by fibrous tissue, i.e., scarring of the liver. Liver cells regenerate in an abnormal pattern, forming nodules that are surrounded by the fibrous tissue. Grossly abnormal liver architecture eventually ensues, and this can lead to decreased blood flow to and through the liver, resulting in biochemical and functional abnormalities.

Retinitis pigmentosa is the name given to a group of inherited eye diseases that affect the retina. Retinitis pigmentosa causes the degeneration of photoreceptor cells in the retina. As the disease progresses and more rod cells degenerate, patients lose their peripheral vision. Patients with Retinitis Pigmentosa often experience a ring of vision loss in their mid-periphery with small islands of vision in their very far periphery. Others report the sensation of tunnel vision, as though they see the world through a straw. Many patients with Retinitis Pigmentosa retain a small degree of central vision throughout their life.

Oxidative stress is a pathology associated with both infectious and non-infectious hepatitis and can contribute to disease progression (Emerit I et al. (2005) Hepatogastroenterology 552:530-6; Pemberton PW et al. (2004) Biochim. Biophys. Acta. 1689:182-9, and Loguercio C et al. (2003) Free Radic. Biol. Med. 34:1-10). Antioxidants are a dietary means for combating oxidative stress. In fact, antioxidants have been demonstrated to exert a hepatoprotective effect (Amin A et al. (2005) Life Sci. 77:266-78), and have been proposed as a supplementary treatment for hepatitis (Dikici I et al. (2005) Clin. Biochem.. 38:1141-4; Medina J et al. (2005) Drugs 65:2445-61; Melham A et al. (2005) J. Clin. Gastroenterol. 39:737-42; and, Peterhans E (1997) J. Nutr. 127:962S-965S).

The various forms of hepatitis are typically treated with various chemotherapeutic regimens. However, many drugs currently used to treat hepatitis can exhibit undesirable side effects. Thus, newer drugs and methods of treatment with fewer or less severe side effects are desirable. Moreover it is also desirable to obtain drugs that can work synergistically with existing therapies to enhance their efficacy, or that can target the underlying molecular, biochemical, or physiological basis for hepatitis.

The complement system is an important weapon in the body's arsenal for immunological defense against foreign pathogens. Complement proteins are activated in an enzyme cascade that can be triggered by various signals, and proceed through one of three main pathways, termed the classical, alternative or lectin pathways. These pathways result in the generation of anaphylatoxic peptides, including C3a and C5a, and can culminate in the formation of the C5b-9 membrane attack complex (MAC), which functions to lyse invading cells. The anaphylatoxins can exert their effects on blood vessels, facilitating inflammation as well as the contraction of smooth muscle and an increase in vascular permeability.

In certain situations, the complement system can produce deleterious effects. For example, inappropriate activation of complement may result in damage to endogenous cells. Complement can exacerbate damage to tissues in antibody-mediated autoimmune diseases such as myasthenia gravis and systemic lupus erythematosus, especially when immune complexes are produced, and can exacerbate tissue damage following ischemia (Liszewski MK et al. (1998) Expert Opin. Investig. Drugs. 7:323-31). Complement has also been implicated in facilitating or exacerbating various disease states, including glomerulonephritis, adult respiratory syndrome, and rejection of transplantated tissues (Glovsky MM et al. (2004) Ann. Allergy Asthma Immunol. 93:513-22; Colvin RB et al. (2005) Nat Rev Immunol. 5:807-17). Complement-mediated tissue injury has also been found to result from bioincompatibility situations, such as those encountered in patients undergoing dialysis or cardiopulmonary bypass (Mollnes TE (1998) Vox Sang. 74 Suppl 2:303-307).

Complement-mediated tissue injuries are directly mediated by the MAC, and indirectly by the generation of the anaphylatoxins C3a and C5a. These peptides induce damage through their effects on neutrophils and mast cells. Regulation of complement at the C3 and C5 activation steps is provided by both plasma and membrane proteins. The plasma protein inhibitors include factor H and C4-binding protein, and the regulatory membrane proteins located on cell surfaces include complement receptors 1 (CR1), decay-accelerating factor (DAF), and membrane cofactor protein (MCP). These proteins inhibit the C3 and C5 convertases (multisubunit proteases), by promoting dissociation of the multisubunit complexes and/or by inactivating the complexes through proteolysis (catalyzed by factor I).

Complement has also been implicated in drusen formation. Drusen is the name given to extracellular deposits localized to the area of the eye between the retinal pigmented epithelium (RPE) and Bruch's membrane, and sometimes localized to the retinal periphery (Lewis HB et al. (1986) Ophthalmology 93:1098-1111). Drusen contains various lipids, proteins, polysaccharides, and glycosaminoglycans, and drusen proteins are often found oxidatively modified (Crabb JW et al. (2002) Proc. Natl. Acad. Sci. USA 99:14682-7). Drusen deposition occurs primarily in aged individuals, and is a primary factor in the pathogenesis of age related macular degeneration (AMD) (Abdelsalam A et al. (1999) Surv. Opthalmol. 44:1-29).

Although the precise mechanisms that lead to drusen formation and depositions have only been partially characterized, there has been speculation that cellular debris from the RPE serves as a stimulus for inflammation and in turn provides a potential nucleation site for the accumulation of drusen (Johnson LV et al. (2000) Exp. Eye Res. 70:441-9; and, Johnson LV et al. (2001) Exp. Eye. Res. 73:887-96). In support of this hypothesis, various inflammatory mediators, including the complement constituents C3a, C5a, and the MAC have been observed in drusen (Luibl V et al. (2006) J. Clin. Invest. 116:378-85), and such components have been found to be colocalized with a complement-activating protein, amyloid-beta protein, in substructural vesicles within drusen (Johnson LV et al. (2002) Proc. Natl. Acad. Sci. USA 99:11830-5). In addition, recent work has demonstrated neutralization of C3a or C5a or their respective receptors reduced neovascularization in AMD (Nozaki M et al. (2006) Proc. Natl. Acad. Sci. USA 2006 Feb 1; [electronic publication ahead of print]. These observations indicate that complement may play a role in the initiation or progression of drusen formation and deposition. As such, complement is an attractive target for inhibiting drusen deposition.

To date, there are no clinically viable inhibitors of complement activation although certain candidates for clinical use exist. Such candidates include a recombinant form of complement receptor 1 known as soluble complement receptor 1 (sCR1) and a humanized monoclonal anti-C5 antibody (5G1.1-scFv). Both of these substances have been shown to suppress complement activation in *in vivo* animal models (Kalli KR et al. (1994) Springer Semin. Immunopathol. 15:417-31; and, Wang et al. (1996) Proc. Natl. Acad. Sci. U S A. 93:8563-8). However, each substance possesses the disadvantage of being large molecular weight proteins (240 kDa and 26,000 kDa, respectively) that are difficult to manufacture and must be administered by infusion. CD59, which blocks assembly of the MAC, has also been proposed as a potential therapeutic agent, but has shown limited activity *in vitro* (Song H et al. (2003) J. Clin. Invest. 111:1875-85). Accordingly, recent research has emphasized the development of smaller active agents that are easier to deliver, more stable, and less toxic to the patient to which they are administered.

The eye can experience numerous diseases and other deleterious conditions that affect its ability to function normally. Many such conditions can be found in the interior and most particularly at the rear of the eye, where lies the optic nerve and the retina, seven layers of alternating cells and processes that convert a light signal into a neural signal. Diseases and degenerative conditions of the optic nerve and retina are the leading causes of blindness throughout the world.

A significant degenerative condition of the retina is macular degeneration, also referred to as age-related macular degeneration (AMD). AMD is the most common cause of vision loss in the United States in those 50 or older, and its prevalence increases with age. AMD is classified as either wet (neovascular) or dry (non-neovascular). The dry form of the disease is most common. It occurs when the central retina has become distorted, pigmented, or most commonly, thinned. The wet form of the disease is responsible for most severe loss of vision. The wet form of macular degeneration is usually associated with aging, but other diseases that can cause wet macular degeneration include severe myopia and some intraocular infections like histoplasmosis, which may be exacerbated in individuals with AIDS. A variety of elements may contribute to macular degeneration, including genetic makeup, age, nutrition, smoking and exposure to sunlight.

Retinopathy associated with diabetes is a leading cause of blindness in type 1 diabetes, and is also common in type 2 diabetes. The degree of retinopathy depends on the duration of the diabetes, and generally begins to occur ten or more years after onset of diabetes. Diabetic retinopathy may be classified as (1) non-proliferative or background retinopathy, characterized by increased capillary permeability, edema, hemorrhage, microaneurysms, and exudates, or 2) proliferative retinopathy, characterized by neovascularization extending from the retina to the vitreous, scarring, fibrous tissue formation, and potential for retinal detachment. Diabetic retinopathy is believed to be caused, at least in part, by the development of glycosylated proteins due to high blood glucose. Glycosylated proteins generate free radicals, resulting in oxidative tissue damage and depletion of cellular reactive oxygen species (ROS) scavengers, such as glutathione.

Several other less common, but nonetheless debilitating retinopathies include choroidal neovascular membrane (CNVM), cystoid macular edema (CME, also referred to as macular edema or macular swelling), epi-retinal membrane (ERM) (macular pucker) and macular hole. In CNVM, abnormal blood vessels stemming from the choroid grow up through the retinal layers. The fragile new vessels break easily, causing blood and fluid to pool within the layers of the retina. In CME, which can occur as a result of disease, injury or surgery, fluid collects within the layers of the macula, causing blurred, distorted central vision. ERM (macular pucker) is a cellophane-like membrane that forms over the macula, affecting the central vision by causing blur and distortion. As it progresses, the traction of the membrane on the macula may cause swelling. ERM is seen most often in people over 75 years of age. Its etiology is unknown, but may be associated with diabetic retinopathy, posterior vitreous detachment, retinal detachment or trauma, among other conditions.

Retinal phototoxicity is induced by exposure of the eye to retinal illumination from an operating microscope positioned for temporal approach eye surgery or from lasers used by the military. These light sources have the potential for light-induced injury to the fovea (M.A. Pavilack and R.D. Brod "Site of Potential Operating Microscope Light-induced Phototoxicity on the Human Retina during Temporal Approach Eye Surgery" Ophthalmol. 2001, 108(2):381-385; H.F. McDonald and M.J. Harris "Operating microscope-induced retinal phototoxicity during pars plana vitrectomy" Arch. Ophthalmol. 1988 106:521-523; Harris M.D. et al. "Laser eye injuries in military occupations" Aviat. Space Environ. Med. 2003, 74(9):947-952). Damage may also occur upon treatment of ablated surface of corneas after excimer laser phototherapy (Seiji Hayashi et al. "Oxygen free radical damage in the cornea after excimer laser therapy" Br. J. Ophthalmol. 1997, 81:141-144).

Retinitis pigmentosa is another such condition of the eye which threatens blindness.

Oxidative stress has been implicated in the development or acceleration of numerous ocular diseases or disorders, including AMD and the various retinopathies described above (see, e.g., Ambati et al., 2003, Survey of Ophthalmology 48: 257-293; Berra et al., 2002, Arch. Gerontol. Geriatrics 34: 371-377), as well as uveitis (e.g., Zamir et al., 1999, Free Rad. Biol. Med. 27: 7-15), cataract (e.g., M. Lou, 2003, Prog. Retinal & Eye Res. 22: 657-682), glaucoma (e.g., Babizhayev & Bunin, 2002, Curr. Op. Ophthalmol. 13: 61-67), corneal and conjuctival inflammations, various corneal dystrophies, post-surgical or UV-associated corneal damage (e.g., Cejkova et al., 2001, Histol. Histopathol. 16: 523-533; Kasetsuwan et al., 1999, Arch. Ophthalmol. 117: 649-652), and presbyopia (Moffat et al., 1999, Exp. Eye Res. 69: 663-669). For this reason, agents with anti-oxidative properties have been investigated as potential therapeutic agents for the treatment of such disorders. Many investigations have focused on the biochemical pathways that generate reducing power in cells, for example, glutathione synthesis and cycling. Enzymes, such as superoxide dismutase, that reduce activated oxygen species have also been studied to determine whether they diminish cellular oxidative stress. Compounds for inhibiting lipid oxidation in cell membranes by direct radical scavenging have also been considered to be promising therapeutic interventions.

Other studies have focused on the administration of elevated doses of common, orally administered antioxidants. For example, the Age-related Eye Disease Study Research Group reported on the outcome of a randomized, placebo-controlled, clinical trial of high-dose supplementation with vitamins C and E, beta carotene, and zinc for treatment of age-related macular degeneration and loss of visual acuity (AREDS Report No.8, reprinted in Arch. Ophthalmol. 2001; 119: 1417-1436).

It has been reported that tissue factor (TF) may be implicated in pathophysiological processes, such as intracellular signaling, cell proliferation, and inflammation. Experimental studies have demonstrated that inhibition of tissue factor:factor VIIa procoagulant activity provides powerful inhibition of in vivo thrombosis and that this approach usually results in less pronounced bleeding tendency, as compared to other "more classical" antithrombotic interventions. (Paolo Golino, Thrombosis Research, Volume 106, Issue 3 , 1 May 2002, Pages V257-V265).

Researchers have disclosed that antibody mediated inhibition of tissue factor (TF) function reduces thrombus size in ex vivo perfusion of human blood suggesting that TF might be involved in the mechanism of deep vein thrombosis. The results suggested that blocking the TF activity could inhibit thrombus propagation. (J. Himber et al., Journal of Thrombosis and Haemostasis 1 (5), 889-895 (2003)). Szotowski et al. disclosed that tissue factor was downregulated in the myocardium of dilated cardiomyopathy (DCM) patients, suggesting that the reduction in TF expression and change in localization may influence cell-to-cell contact stability and contractility, thereby contributing to cardiac dysfunction in DCM. (Björn Szotowski et al; J Am Coll Cardiol 2005;45:1081-9).

The extrinsic pathway of clotting cascade is activated in chronic urticaria (CU). Disease severity is associated with the activation of the coagulation cascade. The extrinsic pathway of the coagulation cascade is activated in chronic urticaria and this activation appears to lead to thrombin generation.

Nitroxides such as TEMPOL have been of greater interest because of their radical scavenging properties and exertion of an anti-inflammatory effect in various animal models of oxidative damage and inflammation. Nilsson et al. disclosed, in WO 88/05044, that nitroxides and their corresponding hydroxylamines are useful in prophylaxis and treatment of ischemic cell damage, presumably due to antioxidant effects. Paolini et al. (U.S. Patent 5,981,548) disclosed N-hydroxylpiperidine compounds and their potential general utility in the treatment of pathologies arising from oxygen radicals and as foodstuff and cosmetic additives. Hsia et al. (U.S. Patents 6,458,758, 5,840,701, 5,824,781, 5,817,632, 5,807,831, 5,804,561, 5,767,089, 5,741,893, 5,725,839 and 5,591,710) disclosed the use of stable nitroxides and hydroxylamines (e.g., TEMPOL and its hydroxylamine counterpart, TEMPOL-H), in combination with a variety of biocompatible macromolecules, to alleviate free radical toxicity in blood and blood components. Hahn et al. (1998, Int. J. Radiat. Oncol. Biol. Physics 42: 839-842; 2000, Free Rad. Biol. Med. 28: 953-958) reported on the *in vivo* radioprotection and effects on blood pressure of the stable free radical nitroxides and certain hydroxylamine counterparts.

Due to their comparative lack of toxicity, hydroxylamines are preferable to nitroxides as therapeutic agents. Published United States Patent Applications 2004/0002461, 2005/0130906 and 2005/0131025 to Matier and Patil disclose hydroxylamines and related compounds and their use in the treatment of a variety of ophthalmic conditions in which oxidative damage or inflammation are involved. Such compounds possess numerous advantageous qualities, including robust anti-inflammatory and antioxidant activities, as well as ocular permeability in some instances. However, while some nitroxides, e.g., TEMPOL, have demonstrated some anti-angiogenic activity, hydroxylamines heretofore have not been reported as possessing any anti-angiogenic activity, any efficacy to treat liver inflammation, any efficacy to inhibit complement activation, or to treat complement- or drusen-mediated pathologies such as AMD.

The claimed invention herein provides new hydroxylamine compounds directed to these and other important ends.

### SUMMARY OF THE INVENTION

Accordingly, the present invention is directed, in part, to novel hydroxyl amine compounds which may have anti-oxidant, anti-inflammatory, and/or free radical scavenging properties and which thus may be useful, *inter alia,* for inhibiting angiogenesis, treating disease states that involve angiogenesis, treating hepatitis, inhibiting complement activation or treating a pathology mediated by complement activation, inhibiting drusen formation, treating macular degeneration or retinopathy, treating inflammation, treating thrombosis including cancer-related, and/or reducing or reversing drug resisistance, including chemoresistance, in a cell demonstrating said drug resistance or chemoresistance.

Specifically, in certain embodiments, the present invention relates to compounds of formula I: 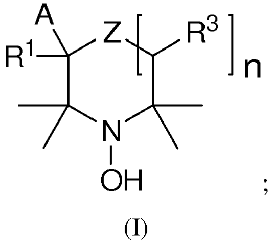 or a pharmaceutically acceptable salt thereof
wherein:
A and B are each H, or taken together form a double bond between the ring atoms to which they are attached, provided that when A and B form a double bond, R⁴ is other than H;
Z is -O- or -C(B)(R²)-, provided that when n is 0, then Z is -C(B)(R²)-;
R¹ and R³ are each independently H, alkyl, or halo;
R² is halo, -OR⁴, -N(R⁵)R⁶, -CN, -(C=O)NH₂, or -C[(R⁷)(R⁸)]ₘR⁹, or when A is H, B and R² taken together form =O; or when A and B taken together form a double bond between the ring atoms to which they are attached, R¹ and R² taken together with the atoms through which they are attached, form an optionally substituted C₆aromatic ring;
m is 1 or 2;
n is 0, 1, or 2;
R⁴ is H, alkyl, or 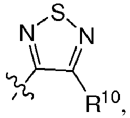
R⁵ is H or alkyl;
R⁶ is alkyl, 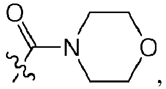 -C(=O)-R¹¹, or -S(=O)₂-R¹¹; or R⁵ and R⁶ taken together with the nitrogen atom to which they are attached form a morpholine ring;
R⁷ and R⁸ are each H or alkyl;
R⁹ is H, alkyl, -OH, -CH₂OCH₂-cycloalkyl, -O-alkyl, furanyl, tetrahydrofuranyl, -C(=O)-furanyl, -CH₂-C(=O)-morpholin-4-yl; -CN, or -N(R⁵)R⁶;
R¹⁰ is H, alkyl, aralkyl, heterocycle, heteroaryl, -NH₂, alkylamino, dialkylamino, halo, or 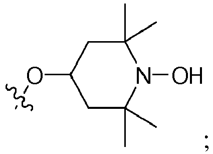 and
R¹¹ is alkyl, cycloalkyl, -NH(3,5-di-*tertiary* butyl-4-hydroxyphenyl), -NH-(4,5-dihydroxy-2-methylphenyl), or

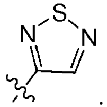

In certain other embodiments, the present invention relates to compounds of Formula II: 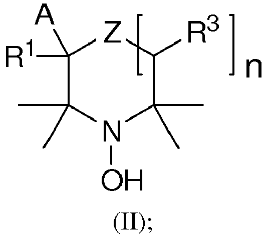 or a pharmaceutically acceptable salt thereof; wherein:
A is H;
Z is -O- or -C(B)(R²)-, provided that when n is 0, then Z is -C(B)(R²)-;
B is H, alkyl, aryl, or heteroaralkyl, or A and B taken together form a double bond between the ring atoms through which they are connected, provided that when A and B form a double bond, R⁴ is other than H;
R¹ is H, alkyl, aryl, hydroxy, or halo; or A and R¹ taken together form =O, provided that when A and R¹ taken together form =O, then Z is -O-;
R³ is H, alkyl, or halo;
R² is H, halo, aryl, aralkyl, heteroaryl, -OR⁴, -SR⁴, -N(R⁵)R⁶, -ONO₂, -CN,_C(=O)-aryl, -C(=O)NH₂, -(C=O)N(R⁵)R⁶, or -C[(R⁷)(R⁸)]ₘR⁹, or R¹ and R² taken together with the atoms through which they are connected form an aryl ring, provided that:
   when R¹ and R² taken together with the atoms through which they are connected form an aryl ring, then A and B are absent;
   when R² is other than-OH, then B is other than alkyl, aryl, or heteroaralkyl;
   when R² is H, then R¹ is H, and A and B taken together form a double bond between the ring atoms through which they are connected;
   when R² is -C(=O)NH₂, then A and B are H, and n is 0; and
   when A is H, B and R² taken together form =O or =CH(R¹²);
m is 1, 2, or 3;
n is 0, 1, or 2;
R⁴ is H, alkyl, aryl, aralkyl, heteroaryl, 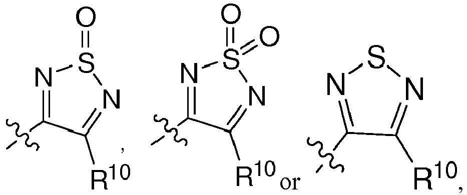
R⁵ is H, alkyl, aryl, or aralkyl;
R⁶ is alkyl, aralkyl, heteroaryl, 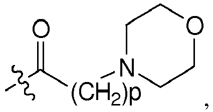 -C(=O)-R¹¹, -C(=NH)-alkyl, or -S(=O)₂-R¹¹; or R⁵ and R⁶ taken together with the nitrogen atom to which they are attached form a heterocycloalkyl ring;
p is 0, 1, or 2;
R⁷ and R⁸ are each H or alkyl;
R⁹ is H, alkyl, -OH, -CH₂OCH₂-cycloalkyl, -O-alkyl, -O-aryl, -ONO₂, heterocycloalkyl, heteroaryl, -C(=O)-aryl,- C(=O)-heteroaryl, -CH₂-C(=O)-heterocycloalkyl; alkylheteroaryloxy, - CN, or -N(R⁵)R⁶;
R¹⁰ is H, alkyl, aryl, aralkyl, arylheterocycloalkyl, heterocycloalkyl, heteroaryl, -NH₂, cyano, carboxy, alkoxycarbonyl, alkylamino, dialkylamino, halo, haloarylheterocycloalkyl, heteroaroylheterocycloalkyl, heteroarylheterocycloalkyl, , C(=O)-heterocycloalkyl, 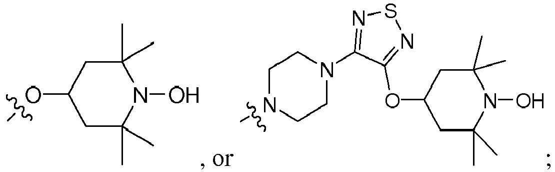
R¹¹ is alkyl, cycloalkyl, aryl, aralkenyl, heterocycloalkyl, halobenzo[1,2,5]oxadiazolyl, heteroarylheterocycloalkyl, heterocycloalkylalkyl -(3,5-di-*tertiary* butyl-4-hydroxyphenyl), - (4,5-dihydroxy-2-methylphenyl), or 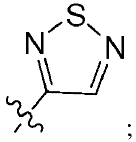 and
R¹² is -C(=O)-heterocycloalkylaryl or C(=O)-heterocycloalkyl.

The present invention is directed, in part, to pharmaceutical compositions, comprising:
a pharmaceutically acceptable carrier; and a compound of formula I or II as defined hereinabove.

The present invention is also directed, in part, to methods of inhibiting angiogenesis in a patient in need thereof, comprising administering to the patient in need thereof a compound of formula I or II as defined hereinabove in a therapeutically sufficient amount to inhibit the angiogenesis.

The present invention provides methods for the treatment of a number of diseases and disorders in which pathogenic angiogenesis is an underlying causal factor. The methods comprise administration of compositions comprising a pharmaceutically acceptable carrier or diluent and a hydroxylamine compound of formula I or II as defined hereinabove in a therapeutically sufficient amount to prevent, retard the development of or reduce the symptoms of one or more angiogenesis-associated diseases or conditions.

The present invention is also directed, in part, to methods of treating a patient having a disease state that involves angiogenesis, comprising administering to the patient in need thereof a compound of formula I or II as defined hereinabove in a therapeutically sufficient amount to inhibit pathological angiogenesis.

The present invention is also directed, in part, to methods for treating or inhibiting hepatitis in a patient, comprising administering to the patient in need thereof a compound a therapeutically sufficient amount of formula I or II as defined hereinabove.

The present invention is also directed, in part, to methods for inhibiting complement activation in a patient, comprising administering to the patient in need thereof a compound of formula I or II as defined hereinabove in an amount effective to inhibit complement activation in the patient.

The present invention is also directed, in part, to methods for treating a patient having a pathology mediated by complement activation comprising administering to the patient in need thereof a compound of formula I or II as defined hereinabove in amount effective to inhibit complement activation in the patient.

The present invention is also directed, in part, to methods of inhibiting drusen formation in a patient, comprising administering to the patient in need thereof a compound of formula I or II as defined hereinabove in a therapeutically sufficient amount to inhibit drusen formation.

The present invention is also directed, in part, to methods for treating macular degeneration or retinopathy in a patient, comprising administering to the patient in need thereof a compound a therapeutically sufficient amount of formula I or II as defined hereinabove.

The present invention is also directed, in part, to methods for treating inflammation in a patient, comprising administering to the patient in need thereof a compound a therapeutically sufficient amount of formula I or II.

The present invention is also directed, in part, to methods for treating thrombosis in a patient, comprising administering to the patient in need thereof a compound a therapeutically sufficient amount of formula I or II as defined hereinabove.

The present invention is also directed, in part, to methods of reducing or reversing chemoresistance in a cell demonstrating said chemoresistance to chemotherapy treatment in a patient, comprising administering to the patient in need thereof a compound a therapeutically sufficient amount of formula I or II as defined hereinabove.

Other features and advantages of the invention will be understood by reference to the drawings, detailed description, and examples that follow.

### BRIEF DESCRIPTION OF THE DRAWINGS

FIG. 1 depicts the dose dependent effect of H₂O₂ in the CAM model observed for TEMPOL-H.
FIG. 2 depicts the anti-angiogenesis efficacy of TEMPOL-H inhibiting oxidative stress, b-FGF, and VEG-F induced angiogenesis in the CAM model.

### DETAILED DESCRIPTION OF ILLUSTRATIVE EMBODIMENTS

The present invention is generally directed to hydroxylamine compounds, pharmaceutical compositions containing these compounds, and methods of their pharmaceutical use.

As employed above and throughout the disclosure, the following terms, unless otherwise indicated, shall be understood to have the following meanings.

As used herein, the term "alkyl" refers to an optionally substituted, saturated, straight or branched hydrocarbon having from about 1 to about 20 carbon atoms (and all combinations and subcombinations of ranges and specific numbers of carbon atoms therein), preferably 1 to about 10, yet more preferably, 1 to about 6, with from 1 to about 3 being even more preferred. Alkyl groups include, but are not limited to, methyl, ethyl, n-propyl, isopropyl, n-butyl, isobutyl, sec-butyl, t-butyl, n-pentyl, isopentyl, neopentyl, n-hexyl, isohexyl, 3-methylpentyl, 2,2-dimethylbutyl, and 2,3-dimethylbutyl. As the context may admit, such groups may be functionalized such as with one or more hydroxy, alkoxy, alkylthio, alkylamino, dialkylamino, aryloxy, arylamino, benzyloxy, benzylamino, heterocycle, or YCO-Z, where Y is O, N, or S and Z is alkyl, cycloalkyl, heterocycle, or aryl substituent.

As used herein, the term "alkenyl" refers to an optionally substituted alkyl group having from about 2 to about 10 carbon atoms and one or more double bonds (and all combinations and subcombinations of ranges and specific numbers of carbon atoms therein), wherein alkyl is as previously defined.

As used herein, the term "cycloalkyl" or "carbocyclic ring" each refers to an optionally substituted, mono-, di-, tri-, or other multicyclic alicyclic ring system having from about 3 to about 20 carbon atoms (and all combinations and subcombinations of ranges and specific numbers of carbon atoms therein). In some preferred embodiments, the cycloalkyl groups have from about 3 to about 8 carbon atoms. Multi-ring structures may be bridged or fused ring structures, wherein the additional groups fused or bridged to the cycloalkyl ring may include optionally substituted cycloalkyl, aryl, heterocycloalkyl, or heteroaryl rings. Exemplary cycloalkyl groups include, but are not limited to, cyclopropyl, cyclobutyl, cyclopentyl, cyclohexyl, cyclooctyl, adamantyl, 2-[4-isopropyl-1-methyl-7-oxa-bicyclo[2.2.1]heptanyl], and 2-[1,2,3,4-tetrahydro-naphthalenyl].

As used herein, the term "heterocycloalkyl" and "heterocyclic ring" each refers to an optionally substituted ring system composed of a cycloalkyl radical wherein in at least one of the rings, one or more of the carbon atom ring members is independently replaced by a heteroatom group selected from the group consisting of O, S, N, and NH, wherein cycloalkyl is as previously defined. Heterocycloalkyl ring systems having a total of from about 5 to about 14 carbon atom ring members and heteroatom ring members (and all combinations and subcombinations of ranges and specific numbers of carbon and heteroatom ring members) are preferred. In other preferred embodiments, the heterocyclic groups may be fused to one or more aromatic rings. In certain preferred embodiments, heterocycloalkyl moieties are attached via a ring carbon atom to the rest of the molecule. Exemplary heterocycloalkyl groups include, but are not limited to, azepanyl, tetrahydrofuranyl, hexahydropyrimidinyl, tetrahydrothienyl, piperidinyl, pyrrolidinyl, isoxazolidinyl, isothiazolidinyl, pyrazolidinyl, oxazolidinyl, thiazolidinyl, piperazinyl, 2-oxo-morpholinyl, morpholinyl, 2-oxo-piperidinyl, piperadinyl, decahydroquinolyl, octahydrochromenyl, octahydro-cyclopenta[c]pyranyl, 1,2,3,4,-tetrahydroquinolyl, 1,2,3,4-tetrahydroquinazolinyl, octahydro-[2]pyridinyl, decahydro-cycloocta[c]furanyl, 1,2,3,4-tetrahydroisoquinolyl, 2-oxo-imidazolidinyl, and imidazolidinyl. In some embodiments, two moieties attached to a heteroatom may be taken together to form a heterocycloalkyl ring, such as when R² and R³, taken together with the nitrogen atom to which they are attached, form a heterocycloalkyl ring. In certain of these embodiments, 1 or 2 of the heterocycloalkyl ring carbon atoms may be replaced by other moieties which contain either one (-O-, -S-, -N(R⁹)-) or two (-N(R¹⁰)-C(=O)-, or -C(=O)-N(R¹⁰)-) ring replacement atoms. When a moiety containing one ring replacement atom replaces a ring carbon atom, the resultant ring, after replacement of a ring atom by the moiety, will contain the same number of ring atoms as the ring before ring atom replacement. When a moiety containing two ring replacement atoms replaces a ring carbon atom, the resultant ring after replacement will contain one more ring atom than the ring prior to replacement by the moiety. For example, when a piperidine ring has one of its ring carbon atoms replaced by -N(R¹⁰)-C(=O)-, the resultant ring is a 7-membered ring containing 2 ring nitrogen atoms and the carbon of a carbonyl group in addition to 4 other carbon ring atoms (CH₂ groups) from the original piperidine ring. In certain alternatively preferred embodiments, five, six and seven membered rings with at least one oxygen or nitrogen atom in the ring are preferred heterocycles, furanyl and tetrahydrofuranyl species are among those still more preferred. In certain other preferred embodiments, heterocycloalkyl is

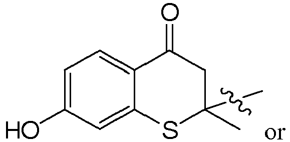

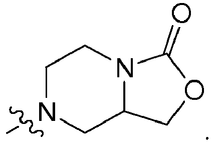

As used herein, the term "aryl" refers to an optionally substituted, mono-, di-, tri-, or other multicyclic aromatic ring system having from about 5 to about 50 carbon atoms (and all combinations and subcombinations of ranges and specific numbers of carbon atoms therein), with from about 6 to about 10 carbons being preferred. Non-limiting examples include, for example, phenyl, naphthyl, anthracenyl, and phenanthrenyl, optionally substituted. In certain preferred embodiments, aryl is 2-hydroxy-5-acetylphenyl, 2-hydroxy-3-methoxy-5-acetylphenyl, 3-hydroxy-2-methoxy-5-acetylphenyl, 3,5-di-*tert*-butyl-4-hydroxyphenyl, or 4,5-dihdroxy-2-methylphenyl. In other preferred embodiments, aryl is substituted with at least one of hydroxy, alkoxy, and aralkenyl.

As used herein, the term "aralkyl" refers to an optionally substituted ring system comprising an alkyl radical bearing an aryl substituent and having from about 6 to about 50 carbon atoms (and all combinations and subcombinations of ranges and specific numbers of carbon atoms therein), with from about 6 to about 10 carbon atoms being preferred. Non-limiting examples include, for example, benzyl, diphenylmethyl, triphenylmethyl, phenylethyl, and diphenylethyl.

As used herein, the term "alkoxyl" refers to an optionally substituted alkyl-O- group wherein alkyl is as previously defined. In some preferred embodiments, the alkyl moieties of the alkoxy groups have from about 1 to about 4 carbon atoms. Exemplary alkoxy groups include, but are not limited to, methoxy, ethoxy, n-propoxy, i-propoxy, n-butoxy, and heptoxy.

As used herein, the term "aryloxyl" refers to an optionally substituted aryl-O- group wherein aryl is as previously defined. Exemplary aryloxy groups include, but are not limited to, phenoxy and naphthoxy.

As used herein, the term "aralkoxyl" refers to an optionally substituted aralkyl-O- group wherein aralkyl is as previously defined. Exemplary aralkoxy groups include, but are not limited to, benzyloxy, 1-phenylethoxy, 2-phenylethoxy, and 3-naphthylheptoxy.

As used herein, the term "halo" refers to a fluoro, chloro, bromo, or iodo moiety, preferably fluoro, chloro, or bromo, with fluoro, chloro, or bromo moieties being more preferred.

As used herein, the term "heteroaryl" refers to an optionally substituted aryl ring system wherein in at least one of the rings, one or more of the carbon atom ring members is independently replaced by a heteroatom group selected from the group consisting of S, O, N, and NH, wherein aryl is as previously defined. Heteroaryl groups having a total of from about 5 to about 14 carbon atom ring members and heteroatom ring members(and all combinations and subcombinations of ranges and specific numbers of carbon and heteroatom ring members) are preferred. Exemplary heteroaryl groups include, but are not limited to, pyrryl, furyl, pyridyl, pyridine-N-oxide, 1,2,4-thiadiazolyl, pyrimidyl, thienyl, isothiazolyl, imidazolyl, tetrazolyl, pyrazinyl, pyrimidyl, quinolyl, isoquinolyl, thiophenyl, benzothienyl, dibenzothienyl, benzthiazolyl, dibenzofuranyl, 9H-carbazolyl (preferably 9H-carbazol-3-yl),isobenzofuryl, pyrazolyl, indolyl, indazolyl, purinyl, carbazolyl, benzimidazolyl, pyrrolo[2,3-b]pyridine, isoxazolyl, 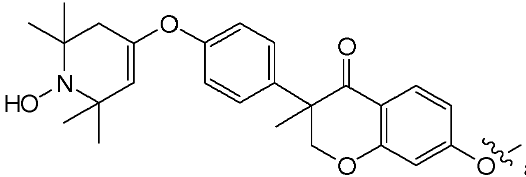 and 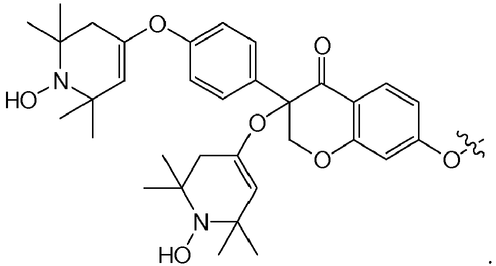 In some embodiments, heteroaryl is preferably tetrazolyl. Heteroaryl may be attached via a carbon or a heteroatom to the rest of the molecule.

As used herein, the term "alkylheteroaryloxy" refers to an alkyl substituted heteroaryl-O- ring system, optionally further substituted, wherein in at least one of the rings, one or more of the carbon atom ring members is independently replaced by a heteroatom group selected from the group consisting of S, O, N, and NH, wherein heteroaryl and alkyl are each as previously defined. Heteroaryloxy groups having a total of from about 5 to about 14 carbon atom ring members and heteroatom ring members(and all combinations and subcombinations of ranges and specific numbers of carbon and heteroatom ring members) are preferred. Exemplary heteroaryloxy groups include, but are not limited to, pyrryloxy, furyloxy, pyridyloxy, 1,2,4-thiadiazolyloxy, pyrimidyloxy, thienyloxy, isothiazolyloxy, imidazolyloxy, tetrazolyloxy, pyrazinyloxy, pyrimidyloxy, quinolyloxy, isoquinolyloxy, thiophenyloxy, benzothienyloxy, isobenzofuryloxy, pyrazolyloxy, indolyloxy, purinyloxy, carbazolyloxy, benzimidazolyloxy, and isoxazolyloxy. Alkylheteroaryloxy may be attached via a carbon or a heteroatom to the rest of the molecule. In certain preferred embodiments, alkylheteroaryloxy is alkyl-[1,2,5]thiadiazol-3-oxy.

As used herein, the term "arylheterocycloalkyl" refers to an aryl substituted ring system optionally further substituted, which is composed of a cycloalkyl radical wherein in at least one of the rings, one or more of the carbon atom ring members is independently replaced by a heteroatom group selected from the group consisting of O, S, N, and NH, wherein cycloalkyl and aryl are each as previously defined. Arylheterocycloalkyl ring systems having a total of from about 11 to about 29 carbon atom ring members and heteroatom ring members (and all combinations and subcombinations of ranges and specific numbers of carbon and heteroatom ring members) are preferred. In other preferred embodiments, the heterocycloalkyl groups may be fused to one or more aromatic rings. In certain preferred embodiments, heterocycloalkyl moieties are attached via a ring carbon atom to the rest of the molecule. Exemplary heterocycloalkyl groups include, but are not limited to, azepanyl, tetrahydrofuranyl, hexahydropyrimidinyl, tetrahydrothienyl, piperidinyl, pyrrolidinyl, isoxazolidinyl, isothiazolidinyl, pyrazolidinyl, oxazolidinyl, thiazolidinyl, piperazinyl, 2-oxo-morpholinyl, morpholinyl, 2-oxo-piperidinyl, piperadinyl, decahydroquinolyl, octahydrochromenyl, octahydro-cyclopenta[c]pyranyl, 1,2,3,4,-tetrahydroquinolyl, 1,2,3,4-tetrahydroquinazolinyl, octahydro-[2]pyridinyl, decahydro-cycloocta[c]furanyl, 1,2,3,4-tetrahydroisoquinolyl, 2-oxo-imidazolidinyl, and imidazolidinyl each of which is substituted with an optionally substituted phenyl, naphthyl, anthracenyl, phenanthrenyl,or pyrenyl. In certain of these embodiments, 1 or 2 of the heterocycloalkyl ring carbon atoms may be replaced by other moieties which contain either one (-O-, -S-, -N(R⁹)-) or two (-N(R¹⁰)-C(=O)-, or -C(=O)-N(R¹⁰)-) ring replacement atoms. When a moiety containing one ring replacement atom replaces a ring carbon atom, the resultant ring, after replacement of a ring atom by the moiety, will contain the same number of ring atoms as the ring before ring atom replacement. When a moiety containing two ring replacement atoms replaces a ring carbon atom, the resultant ring after replacement will contain one more ring atom than the ring prior to replacement by the moiety. For example, when a piperidine ring has one of its ring carbon atoms replaced by -N(R¹⁰)-C(=O)-, the resultant ring is a 7-membered ring containing 2 ring nitrogen atoms and the carbon of a carbonyl group in addition to 4 other carbon ring atoms (CH₂ groups) from the original piperidine ring. In certain alternatively preferred embodiments, five, six and seven membered rings with at least one oxygen or nitrogen atom in the ring are preferred heterocycles, optionally substituted furanyl and tetrahydrofuranyl species are among those still more preferred.

As used herein, the term "haloarylheterocycloalkyl" refers to a haloaryl substituted ring system optionally further substituted, wherein halo and arylheterocycloalkyl are as previously defined. Exemplary halo aryl groups include optionally substituted halophenyl, dihalophenyl, halonaphthyl and the like, wherein at least one halo of the haloaryl is fluoro, chloro, or bromo, more preferably fluoro. More preferred in some embodiments, haloarylheterocycloalkyl is optionally substituted haloarylpiperazinyl, still more preferably fluorophenylpiperazinyl.

As used herein, the term "heteroarylheterocycloalkyl" refers to a heteroaryl substituted heterocycloalkyl ring system optionally further substituted, wherein heteroaryl and heterocycloalkyl are as previously defined. Exemplary embodiments include optionally substituted pyridylpiperazinyl, pyrimidinylpiperazinyl, and thiadiazolinylpiperidinyl.

As used herein, the term "heteroaroylheterocycloalkyl" refers to a heteroaryl-C(=O)-substituted heterocycloalkyl ring system optionally further substituted, wherein heteroaryl and heterocycloalkyl are as previously defined. Exemplary embodiments include optionally substituted furanoylpiperazinyl.

As used herein, the term "aralkenyl" refers to an aryl substituted alkenyl group further optionally substituted, wherein aryl and alkenyl are as previously defined. Exemplary aralkenyl groups include optionally substituted styryl(phenyl substituted ethenyl) groups such as 4-hydroxy-3-methoxyphenethenyl, 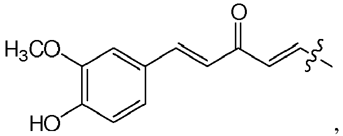 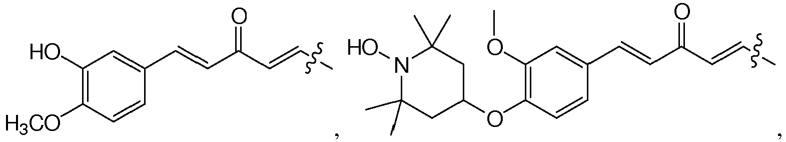 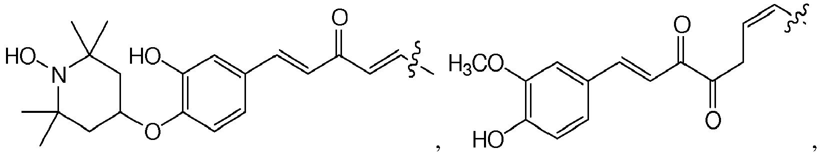 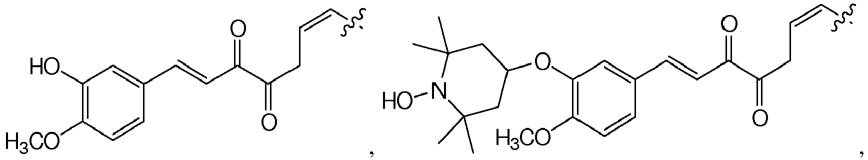 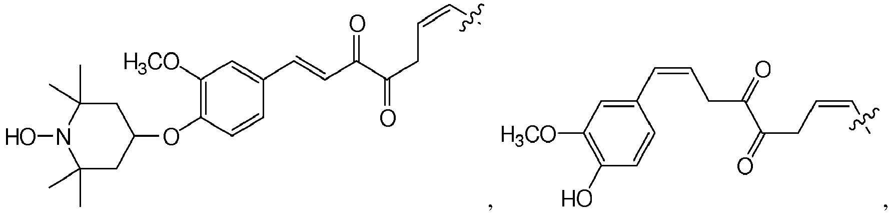 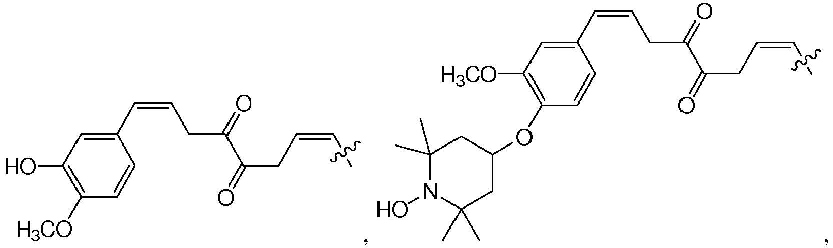 and

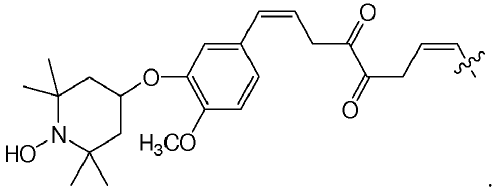

As used herein, the term "heterocycloalkylaryl" refers to a heterocycloalkyl substituted aryl group optionally further substituted, wherein aryl and heterocycloalkyl are as previously defined.

As used herein, the term "heterocycloalkylalkyl" refers to an optionally substituted ring system composed of an alkyl radical having one or more heterocycloalkyl substituents, wherein heterocycloalkyl and alkyl are as previously defined. In some preferred embodiments, the alkyl moieties of the heterocycloalkylalkyl groups have from about 1 to about 3 carbon atoms. Exemplary heterocycloalkyl groups include, but are not limited to, optionally substituted azepanylmethyl, tetrahydrofuranylethyl, hexahydropyrimidinylisobutyl, tetrahydrothienylpropyl, piperidinyl-2,2-dimethylethyl, pyrrolidinylmethyl , isoxazolidinylethyl, isothiazolidinylpropyl, pyrazolidinylmethyl, oxazolidinylbutyl, thiazolidinylisopropyl, piperazinylmethyl, 2-oxo-morpholinylmethyl, morpholinylethyl, 2-oxo-piperidinylethyl, piperadinylmethyl, decahydroquinolylethyl, octahydrochromenylpropyl, octahydro-cyclopenta[c]pyranylbutyl, 1,2,3,4,-tetrahydroquinolylethyl, 1,2,3,4-tetrahydroquinazolinylmethyl, octahydro-[2]pyridinylethyl, decahydro-cycloocta[c]furanylmethyl, 1,2,3,4-tetrahydroisoquinolylmethyl, 2-oxo-imidazolidinylethyl, and imidazolidinylmethyl.

Typically, substituted chemical moieties include one or more substituents that replace hydrogen. Exemplary substituents include, for example, halo (e.g., F, Cl, Br, I), alkyl, alkenyl, cycloalkyl, aralkyl, aryl, aralkenyl, heteroaryl, heterocycloalkyl, hydroxyl (-OH), oxo (=O), alkoxyl, aryloxyl, aralkoxyl, nitro (-NO₂), nitrooxy(-ONO₂), cyano (-CN), amino (-NH₂), N-substituted amino (-NHR"), *N,N-*disubstituted amino (-N(R")R"), carboxyl (-COOH), -C(=O)R", -OR", -P(=O)(alkoxy)₂, -C(=O)OR", -C(=O)NHSO₂R", -NHC(=O)R", aminocarbonyl (-C(=O)NH₂), N-substituted aminocarbonyl (-C(=O)NHR"), *N,N*-disubstituted aminocarbonyl (-C(=O)N(R")R"), -alkylene-NH-C(=NH)(alkyl), -C(=NH)alkyl, -NHC(=NH)alkyl, thiolato (-SR"), -S(=O)₂R", -S(=O)₂NH₂, -S(=O)₂NHR", -S(=O)₂NR"R", -SO₂NHC(=O)R", - NHS(=O)₂R", -NR"S(=O)₂R", -CF₃, -CF₂CF₃, -NHC(=O)NHR", -NHC(=O)NR"R", - NR"C(=O)NHR", -NR"C(=O)NR"R", -NR"C(=O)R" and the like. In relation to the aforementioned substituents, each moiety R" can be, independently, any of H, alkyl, cycloalkyl, alkenyl, aryl, aralkyl, heteroaryl, or heterocycloalkyl, or when (R"(R")) is attached to a nitrogen atom, R" and R" can be taken together with the nitrogen atom to which they are attached to form a 4- to 8-membered nitrogen heterocycle, wherein the heterocycloalkyl ring is optionally interrupted by one or more additional -O-, -S-, -SO, -SO₂-, -NH-, -N(alkyl)-, or -N(aryl)-groups, for example. In certain embodiments, chemical moieties are substituted by at least one optional substituent, such as those provided hereinabove. In the present invention, when chemical moieties are substituted with optional substituents, the optional substituents are not further substituted. For example, when R¹ is an alkyl moiety, it is optionally substituted, based on the definiton of "alkyl" as set forth herein. Specifically, when R¹ is alkyl substituted with optional aryl, the optional aryl substituent is not further substituted. To further clarify, 2-(alpha-naphthyl)ethyl (wherein ethyl is the alkyl moiety and alpha-naphthyl is the optional aryl substituent) is within the scope of optionally substituted alkyl. In contrast, 2-(3-chlorophenyl)ethyl (wherein ethyl is the alkyl moiety and 3-chlorophenyl is the optional substituent) is not within the scope of optionally substituted alkyl because the optional aryl substituent cannot be further substituted by a further chemical group.

As used herein, the term "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, cyclohexylsulfamic acid, and quinic acid, and the like. These physiologically acceptable salts are prepared by methods known in the art, e.g., by dissolving the free amine bases with an excess of the acid in aqueous alcohol, or neutralizing a free carboxylic acid with an alkali metal base such as a hydroxide, or with an amine.

Compounds described herein throughout, can be used or prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

As used herein, the term "N-oxide" refers to compounds wherein the basic nitrogen atom of either a heteroaromatic ring or tertiary amine is oxidized to give a quaternary nitrogen bearing a positive formal charge and an attached oxygen atom bearing a negative formal charge.

As used herein, the term "hydrate" refers to a compound of the present invention which is associated with water in the molecular form, i.e., in which the H-OH bond is not split, and may be represented, for example, by the formula R·H₂O, where R is a compound of the invention. A given compound may form more than one hydrate including, for example, monohydrates (R·H₂O) or polyhydrates (R·nH₂O wherein n is an integer > 1) including, for example, dihydrates (R·2H₂O), trihydrates (R·3H₂O), and the like, or hemihydrates, such as, for example, R·n_{/2}H₂O, R·n_{/3}H₂O, R·n_{/4}H₂O and the like wherein n is an integer.

As used herein, the term "solvate" refers to a compound of the present invention which is associated with solvent in the molecular form, i.e., in which the solvent is coordinatively bound, and may be represented, for example, by the formula R· (solvent), where R is a compound of the invention. A given compound may form more than one solvate including, for example, monosolvates (R· (solvent)) or polysolvates (R· n(solvent)) wherein n is an integer > 1) including, for example, disolvates (R· 2(solvent)), trisolvates (R· 3(solvent)), and the like, or hemisolvates, such as, for example, R· n_{/2}(solvent), R· n_{/3}(solvent), R·n_{/4}(solvent) and the like wherein n is an integer. Solvents herein include mixed solvents, for example, methanol/water, and as such, the solvates may incorporate one or more solvents within the solvate.

As used herein, the term "acid hydrate" refers to a complex that may be formed through association of a compound having one or more base moieties with at least one compound having one or more acid moieties or through association of a compound having one or more acid moieties with at least one compound having one or more base moieties, said complex being further associated with water molecules so as to form a hydrate, wherein said hydrate is as previously defined and R represents the complex herein described above.

Certain acidic or basic compounds of the present invention may exist as zwitterions. All forms of the compounds, including free acid, free base and zwitterions, are contemplated to be within the scope of the present invention. It is well known in the art that compounds containing both basic nitrogen atom and acidic groups often exist in equilibrium with their zwitterionic forms. Thus, any of the compounds described herein throughout that contain, for example, both basic nitrogen and acidic groups, also include reference to their corresponding zwitterions.

As used herein, the term "therapeutically sufficient amount" refers to an amount of a compound as described herein that may be therapeutically sufficient to inhibit, prevent or treat the symptoms of particular disease, disorder or side effect. Thus, for example, for treating a subject afflicted with hepatitis, a therapeutically sufficient amount of a composition comprising a pharmaceutically acceptable carrier and at least one hydroxylamine compound or ester derivative thereof is administered to the subject. A therapeutically sufficient amount will provide a clinically significant decrease in localized or systemic inflammation of the liver or biliary tissue, or the inhibition of the onset or progression of hepatitis, and the like. The compositions are effective to treat chronic and acute hepatitis, as well as infectious and non-infectious hepatitis, and can be administered to any animal, particularly mammals such as dogs, cats, rats, mice, rabbits, horses, pigs, cows, sheep, and donkeys, and are preferably administered to humans.

The therapeutically sufficient amount of the composition may be dependent on any number of variables, including without limitation, the species, breed, size, height, weight, age, overall health of the subject, the type of formulation, the mode or manner or administration, or the severity of the hepatitis or other related condition. The therapeutically sufficient amount can be routinely determined by those of skill in the art using routine optimization techniques and the skilled and informed judgment of the practitioner and other factors evident to those skilled in the art. Preferably, a therapeutically sufficient dose of the compounds described herein will provide therapeutic benefit without causing substantial toxicity to the subject.

Toxicity and therapeutic efficacy of agents or compounds can be determined by standard pharmaceutical procedures in cell cultures or experimental animals, e.g., for determining the LD50 (the dose lethal to 50% of the population) and the ED50 (the dose therapeutically sufficient in 50% of the population). The dose ratio between toxic and therapeutic effects is the therapeutic index and it can be expressed as the ratio LD50/ED50. Agents or compositions which exhibit large therapeutic indices are preferred. The dosage of such agents or compositions lies preferably within a range of circulating concentrations that include the ED50 with little or no toxicity. The dosage may vary within this range depending upon the dosage form employed and the route of administration utilized.

For the compositions used in the inventive methods, the therapeutically sufficient dose can be estimated initially from *in vitro* assays such as cell culture assays. For example, a dose can be formulated in animal models to achieve a circulating plasma concentration range that includes the IC50 as determined in cell culture (*i.e.,* the concentration of the composition which achieves a half-maximal inhibition of the osteoclast formation or activation). Such information can be used to more accurately determine useful doses in a specified subject such as a human. The treating physician can terminate, interrupt, or adjust administration due to toxicity, or to organ dysfunctions, and can also adjust treatment as necessary if the clinical response was not adequate in order to improve the clinical response.

As used herein, the term "pharmaceutically acceptable" refers to those compounds, materials, compositions, and/or dosage forms that are, within the scope of sound medical judgment, suitable for contact with the tissues of human beings and animals without excessive toxicity, irritation, allergic response, or other problems or complications commensurate with a reasonable benefit/risk ratio.

As used herein, the term "in combination with", "combination therapy" and "combination products" refer, in certain embodiments, to the concurrent administration to a patient of one or more additional anti-angiogenic agents and the compounds of the formula I or II. When administered in combination, each component may be administered at the same time or sequentially in any order at different points in time. Thus, each component may be administered separately but sufficiently closely in time so as to provide the desired therapeutic effect.

As used herein, the term "dosage unit" refers to physically discrete units suited as unitary dosages for the particular individual to be treated. Each unit may contain a predetermined quantity of active compound(s) calculated to produce the desired therapeutic effect(s) in association with the required pharmaceutical carrier. The specification for the dosage unit forms of the invention may be dictated by (a) the unique characteristics of the active compound(s) and the particular therapeutic effect(s) to be achieved, and (b) the limitations inherent in the art of compounding such active compound(s).

As used herein, the term "angiogenesis" means the generation of new blood vessels into a tissue or organ. Under normal physiological conditions, humans or animals undergo angiogenesis only in very specific restricted situations. For example, angiogenesis is normally observed in wound healing, fetal and embryonal development and formation of the corpus luteum, endometrium and placenta. The term "endothelium" is defined herein as a thin layer of flat cells that lines serous cavities, lymph vessels, and blood vessels. These cells are defined herein as "endothelial cells". The term "endothelial inhibiting activity" means the capability of a molecule to inhibit angiogenesis in general. The inhibition of endothelial cell proliferation at various stages also results in an inhibition of angiogenesis (Albo, et al., 2004, Curr Pharm Des. 10(1):27-37).

Many diseases or adverse conditions are associated with angiogenesis. Examples of such diseases or disorders include, but are not limited to, (1) neoplastic diseases, such as cancers of the breast, head, rectum, gastrointestinal tract, lung, bronchii, pancreas, thyroid, testicles or ovaries, leukemia (e.g., acute myelogenous leukemia), sinonasal natural killer/T-cell lymphoma, malignant melanoma, adenoid cystic carcinoma, angiosarcoma, anaplastic large cell lymphoma, endometrial carcinoma,or prostate carcinoma (2) hyperproliferative disorders, e.g., disorders caused by non-cancerous (i.e. non-neoplastic) cells that overproduce in response to a particular growth factor, such as psoriasis, endometriosis, atherosclerosis, systemic lupus and benign growth disorders such as prostate enlargement and lipomas; (3) cell proliferation as a result of infectious diseases, such as Herpes simplex infections, Herpes zoster infections, protozoan infections and Bartonellosis (a bacterial infection found in South America); (4) arthritis, including rheumatoid arthritis and osteoarthritis; (5) chronic inflammatory disease, including ulcerative colitis and Crohn's disease; and (6) other conditions, including the childhood disease, hemangioma, as well as hereditary diseases such as Osler-Weber-Rendu disease, or hereditary hemorrhagic telangiectasia.

The present inventors have determined that angiogenesis, and the diseases or disorders involving angiogenesis, can be ameliorated through the administration of hydroxylamine compounds of formula I or II. This determination was made in part through the use of the chick chorioallantoic membrane (CAM) model of angiogenesis, the protocols of which are set forth in the examples.

The following abbreviations may be used in the specification and examples: HAV, hepatitis A virus; HBV, hepatitis B virus; HCV, hepatitis C virus, HDV, hepatitis D virus; HEV, hepatitis E virus; HFV, hepatitis F virus; HGV, hepatitis G virus.

The terms "treating" or "treatment" refer to any success or indicia of success in the attenuation or amelioration of an injury, pathology or condition, including any objective or subjective parameter such as abatement, remission, diminishing of symptoms or making the injury, pathology, or condition more tolerable to the patient, slowing in the rate of degeneration or decline, making the final point of degeneration less debilitating, improving a subject's physical or mental well-being, or prolonging the length of survival. The treatment or amelioration of symptoms can be based on objective or subjective parameters; including the results of a physical examination, neurological examination, and/or psychiatric evaluations. The term "treatment" as used herein includes preventative (e.g., prophylactic), curative or palliative treatment and "treating" as used herein also includes preventative, curative and palliative treatment.

"Hepatitis" refers to any clinically significant inflammation of the liver or biliary system, regardless of etiology. "Acute hepatitis" refers to any short term (less than six months) or initial-stage liver inflammation, such as the initial stages of hepatitis virus infection. "Chronic hepatitis" refers to any inflammation of the liver persisting six months or longer. "Infectious hepatitis" refers to any inflammation of the liver that can be transmitted to others. Typically, infectious hepatitis is caused by a microorganism such as a virus (e.g., HAV, HBV, HCV, HDV, HEV, HFV, HGV, cytomegalovirus, Epstein-Barr virus, herpes simplex virus (HSV), and Varicella-Zoster virus, etc.), bacteria, protozoan, or yeast. "Non-infectious hepatitis" refers to any inflammation of the liver that cannot be transmitted to others, such as alcoholic hepatitis, autoimmune hepatitis, toxic/drug induced hepatitis, and granulomatus hepatitis, and the like. Biological results or treatments may include, but are not limited to, the treatment of both infectious and non-infectious hepatitis in a subject, as determined by any means suitable in the art.

The terms "biliary system" or "biliary tissue" refer to the organs and duct system that create, transport, store, and release bile into the small intestine. The term encompasses the liver, gallbladder, and bile ducts: the cystic duct, hepatic duct, common hepatic duct, common bile duct, and pancreatic duct.

"Etiology" means the cause or origin of a disease, disorder, or pathology.

"Pathology" refers to the structural and functional deviations from a normal state that constitute the inception or progression of a disorder, disease, or disease state, or characterize a particular disorder or disease.

"Drusen" refers to any extracellular deposits that accumulate beneath the basement membrane of the retinal pigmented epithelium (RPE) and the inner collagenous layer of the Bruch membrane.

As used herein, the term "patient" refers to animals, preferably mammals, more preferably humans.

When any variable occurs more than one time in any constituent or in any formula, its definition in each occurrence is independent of its definition at every other occurrence. Combinations of substituents and/or variables are permissible only if such combinations result in stable compounds.

It is believed the chemical formulas and names used herein correctly and accurately reflect the underlying chemical compounds. However, the nature and value of the present invention does not depend upon the theoretical correctness of these formulae, in whole or in part. Thus it is understood that the formulas used herein, as well as the chemical names attributed to the correspondingly indicated compounds, are not intended to limit the invention in any way, including restricting it to any specific tautomeric form or to any specific optical or geometric isomer, except where such stereochemistry is clearly defined.

Accordingly, the present invention is directed, in part, to compounds of formula I: 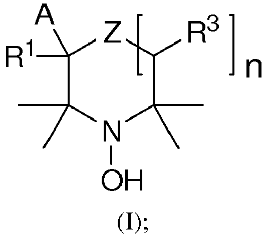 or a pharmaceutically acceptable salt thereof
wherein:
A and B are each H, or taken together form a double bond between the ring atoms to which they are attached, provided that when A and B form a double bond, R⁴ is other than H;
Z is -O- or -C(B)(R²)-, provided that when n is 0, then Z is -C(B)(R²)-;
R¹ and R³ are each independently H, alkyl, or halo;
R² is halo, -OR⁴, -N(R⁵)R⁶, -CN, -(C=O)NH₂, or -C[(R⁷)(R⁸)]ₘR⁹, or when A is H, B and R² taken together form =O; or when A and B taken together form a double bond between the ring atoms to which they are attached, R¹ and R² taken together with the atoms through which they are attached, form an optionally substituted C₆aromatic ring;
m is 1 or 2;
n is 0, 1, or 2;
R⁴ is H, alkyl, or 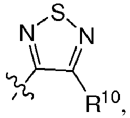
R⁵ is H or alkyl;
R⁶ is alkyl, 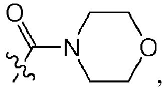 -C(=O)-R¹¹, or -S(=O)₂-R¹¹; or R⁵ and R⁶ taken together with the nitrogen atom to which they are attached form a morpholine ring;
R⁷ and R⁸ are each H or alkyl;
R⁹ is H, alkyl, -OH, -CH₂OCH₂-cycloalkyl, -O-alkyl, furanyl, tetrahydrofuranyl, -C(=O)-furanyl, -CH₂-C(=O)-morpholin-4-yl; -CN, or -N(R⁵)R⁶;
R¹⁰ is H, alkyl, aralkyl, heterocycle, heteroaryl, -NH₂, alkylamino, dialkylamino, halo, or 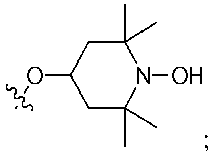 and
R¹¹ is alkyl, cycloalkyl, -NH(3,5-di-*tertiary* butyl-4-hydroxyphenyl), -NH-(4,5-dihydroxy-2-methylphenyl), or

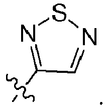

The present invention is also directed, in part, to compounds of formula II: 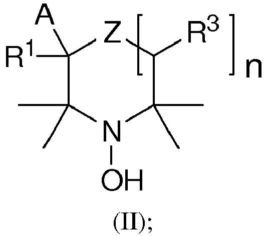 or a pharmaceutically acceptable salt thereof;
wherein:
A is H;
Z is -O- or -C(B)(R²)-, provided that when n is 0, then Z is -C(B)(R²)-;
B is H, alkyl, aryl, or heteroaralkyl, or A and B taken together form a double bond between the ring atoms through which they are connected, provided that when A and B form a double bond, R⁴ is other than H;
R¹ is H, alkyl, aryl, or halo; or A and R¹ taken together form =O, provided that when A and R¹ taken together form =O, then Z is -O-;
R³ is H, alkyl, or halo;
R² is H, halo, aryl, aralkyl, heteroaryl, -OR⁴, -SR⁴, -N(R⁵)R⁶, -ONO₂, -CN,_-C(=O)-aralkyl, -C(=O)NH₂, -(C=O)N(R⁵)R⁶, or -C[(R⁷)(R⁸)]ₘR⁹, or R¹ and R² taken together with the atoms through which they are connected form an aryl ring, provided that:
   when R¹ and R² taken together with the atoms through which they are connected form an aryl ring, then A and B are absent;
   when R² is other than-OH, then B is other than alkyl, aryl, or heteroaralkyl;
   when R² is H, then R¹ is H, and A and B taken together form a double bond between the ring atoms through which they are connected;
   when R² is -C(=O)NH₂, then A and B are H, and n is 0; and
   when A is H, B and R² taken together form =O or =CH(R¹²);
m is 1, 2, or 3;
n is 0, 1, or 2;
R⁴ is H, alkyl, aryl, aralkyl, heteroaryl, 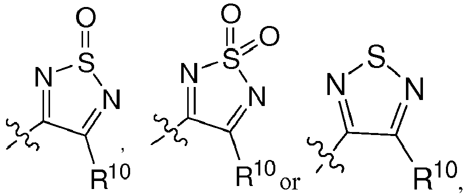
R⁵ is H, alkyl, aryl, or aralkyl;
R⁶ is alkyl, aralkyl, heteroaryl, 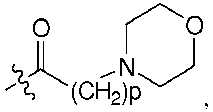 -C(=O)-R¹¹, -C(=NH)-alkyl, or -S(=O)₂-R¹¹; or R⁵ and R⁶ taken together with the nitrogen atom to which they are attached form a heterocycloalkyl ring;
p is 0, 1, or 2;
R⁷ and R⁸ are each H or alkyl;
R⁹ is H, alkyl, -OH, -CH₂OCH₂-cycloalkyl, -O-alkyl, -O-aryl, -ONO₂, heterocycloalkyl, heteroaryl, -C(=O)-aryl,- C(=O)-heteroaryl, -CH₂-C(=O)-heterocycloalkyl; alkylheteroaryloxy, -CN, or -N(R⁵)R⁶;
R¹⁰ is H, alkyl, aryl, aralkyl, arylheterocycloalkyl, heterocycloalkyl, heteroaryl, -NH₂, cyano, carboxy, alkoxycarbonyl, alkylamino, dialkylamino, halo, haloarylheterocycloalkyl, heteroaroylheterocycloalkyl, heteroarylheterocycloalkyl, , C(=O)-heterocycloalkyl, 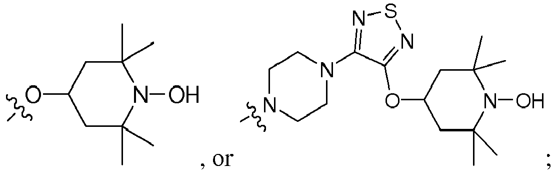
R¹¹ is alkyl, cycloalkyl, aryl, aralkenyl, heterocycloalkyl, halobenzo[1,2,5]oxadiazolyl, heteroarylheterocycloalkyl, heterocycloalkylalkyl -(3,5-di-*tertiary* butyl-4-hydroxyphenyl), -(4,5-dihydroxy-2-methylphenyl), or 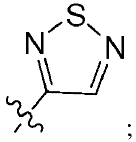 and
R¹² is -C(=O)-heterocycloalkylaryl or C(=O)-heterocycloalkyl.

In certain preferred embodiments, the compounds of the invention have the formula I or II described hereinabove with the proviso that the compounds of formula I or II are other than 1,4-dihydroxy-2,2,6,6-tetramethylpiperidine, 1-hydroxy-4-methoxy-2,2,6,6-tetramethylpiperidine, 1-hydroxy-4-ethoxy-2,2,6,6-tetramethylpiperidine, 1-hydroxy-4-amino-2,2,6,6-tetramethylpiperidine, 1-hydroxy-4-acetamido-2,2,6,6-tetramethylpiperidine, 1-hydroxy-2,2,6,6-tetramethyl-piperidin-4-one, 1-hydroxy-2,2,5,5-tetramethyl- pyrrolidin-3-one, 2,2,5,5-tetramethyl-pyrrolidine-1,3-diol, 3-hydroxymethyl-2,2,5,5-tetramethyl-2,5-dihydro-pyrrol-1-ol, 3-bromo-2,2,6,6-tetramethyl-piperidine-1,4-diol, 3-bromo-4-methoxy-2,2,6,6-tetramethylpiperidine-1-ol, 3-chloro-2,2,6,6-tetramethyl-piperidine-1,4-diol, 1-hydroxy-4-methanesulfonamido-2,2,6,6-tetramethylpiperidine, 4-chloro-1-hydroxy-2,2,6,6-tetramethylpiperidine, 3-bromo-1-hydroxy-2,2,6,6-tetramethyl-piperidin-4-one, 3-chloro-1-hydroxy-2,2,6,6-tetramethyl-piperidin-4-one, 1-Hydroxy-2,2,5,5-tetramethyl-pyrrolidin-3-one, 4-bromo-1-hydroxy-2,2,6,6-tetramethylpiperidine, 2,2,6,6-tetramethyl-4-morpholin-4-yl-3,6-dihydro-2H-pyridin-1-ol, 3-ethoxymethyl-2,2,5,5-tetramethyl-2,5-dihydro-pyrrol-1-ol, 3-(N,N-dimethylaminomethyl)-2,2,5,5-tetramethyl-2,5-dihydro-pyrrol-1-ol, 3-(N,N-diethylaminomethyl)-2,2,5,5-tetramethyl-2,5-dihydro-pyrrol-1-ol, 3-(N-tertiarybutylaminomethyl)-2,2,5,5-tetramethyl-2,5-dihydro-pyrrol-1-ol, 3-(N-pyrrolidinomethyl)-2,2,5,5-tetramethyl-2,5-dihydro-pyrrol-1-ol, 3-(N-piperidinomethyl)-2,2,5,5-tetramethyl-2,5-dihydro-pyrrol-1-ol, or 2,5-dihydro-2,2,5,5-tetramethyl-1H-pyrrole-1-hydroxy-3-carboxamide.

In certain other preferred embodiments, the compounds of the invention have the formula I or II described hereinabove with the proviso that the compounds of formula I or II are other than 1,4-dihydroxy-2,2,6,6-tetramethylpiperidine, 1-hydroxy-4-methoxy-2,2,6,6-tetramethylpiperidine, 1-hydroxy-4-ethoxy-2,2,6,6-tetramethylpiperidine, 1-hydroxy-4-amino-2,2,6,6-tetramethylpiperidine, 1-hydroxy-4-acetamido-2,2,6,6-tetramethylpiperidine, 1-hydroxy-2,2,6,6-tetramethyl-piperidin-4-one, 1-hydroxy-2,2,5,5-tetramethyl- pyrrolidin-3-one, 2,2,5,5-tetramethyl-pyrrolidine-1,3-diol, 3-hydroxymethyl-2,2,5,5-tetramethyl-2,5-dihydro-pyrrol-1-ol, 3-bromo-2,2,6,6-tetramethyl-piperidine-1,4-diol, 3-bromo-4-methoxy-2,2,6,6-tetramethylpiperidine-1-ol, 3-chloro-2,2,6,6-tetramethyl-piperidine-1,4-diol, 1-hydroxy-4-methanesulfonamido-2,2,6,6-tetramethylpiperidine, 4-chloro-1-hydroxy-2,2,6,6-tetramethylpiperidine, 3-bromo-1-hydroxy-2,2,6,6-tetramethyl-piperidin-4-one, 3-chloro-1-hydroxy-2,2,6,6-tetramethyl-piperidin-4-one, 4-bromo-1-hydroxy-2,2,6,6-tetramethylpiperidine, 2,2,6,6-tetramethyl-4-morpholin-4-yl-3,6-dihydro-2H-pyridin-1-ol, 3-ethoxymethyl-2,2,5,5-tetramethyl-2,5-dihydro-pyrrol-1-ol, 3-(N,N-dimethylaminomethyl)-2,2,5,5-tetramethyl-2,5-dihydro-pyrrol-1-ol, 3-(N,N-diethylaminomethyl)-2,2,5,5-tetramethyl-2,5-dihydro-pyrrol-1-ol, 3-(N-tertiarybutylaminomethyl)-2,2,5,5-tetramethyl-2,5-dihydro-pyrrol-1-ol, 3-(N-pyrrolidinomethyl)-2,2,5,5-tetramethyl-2,5-dihydro-pyrrol-1-ol, 3-(N-piperidinomethyl)-2,2,5,5-tetramethyl-2,5-dihydro-pyrrol-1-ol, 1-hydroxy-2,2,5,5-tetramethylpyrrolidine-3-carboxamide, or 2,5-dihydro-2,2,5,5-tetramethyl-1H-pyrrole-1-hydroxy-3-carboxamide.

In certain preferred embodiments of formula I or II compounds, A and B are each H. In other preferred embodiments, A and B taken together form a double bond between the ring atoms to which they are attached, provided that when A and B form a double bond, R⁴ is other than H.

In some preferred embodiments of formula I or II compounds, Z is -C(B)(R²)-.

In other preferred embodiments of formula I or II compounds, R¹ and R³ are each H. In other preferred embodiments, at least one of R¹ and R³ is alkyl or halo.

In certain preferred embodiments of formula I or II compounds, R² is halo, -OR⁴, - N(R⁵)R⁶, -(C=O) N(R⁵)R⁶,or -C[(R⁷)(R⁸)]ₘR⁹, more preferably -OR⁴, -N(R⁵)R⁶, -(C=O) N(R⁵)R⁶, or -C[(R⁷)(R⁸)]ₘR⁹, still more preferably -OR⁴, -N(R⁵)R⁶, -(C=O) N(R⁵)R⁶, or -C[(R⁷)(R⁸)]ₘR⁹, with -OR⁴, -N(R⁵)R⁶, or -C[(R⁷)(R⁸)]ₘR⁹ being most preferred.

In other preferred embodiments of compounds of formula I or II, R² is C(=O)NH₂.

In some preferred embodiments of compounds of formula I or II, R¹ and R² taken together with the atoms through which they are attached, form an optionally substituted C₆aromatic ring.

In other preferred embodiments of compounds of formula I or II, m is 1.

In certain preferred embodiments of compounds of formula I or II, n is 0 or 1, more preferably wherein n is 1. Alternatively preferred in some embodiments of compounds of formula I or II, n is 0.

Representative compounds of Formula I or II when n is 1 include:

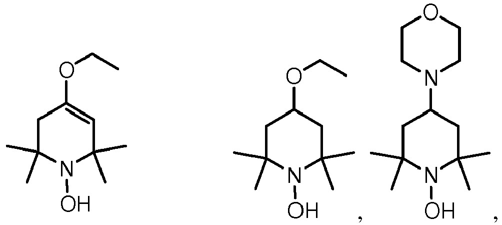

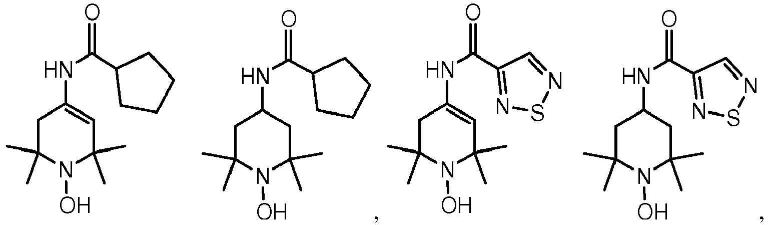

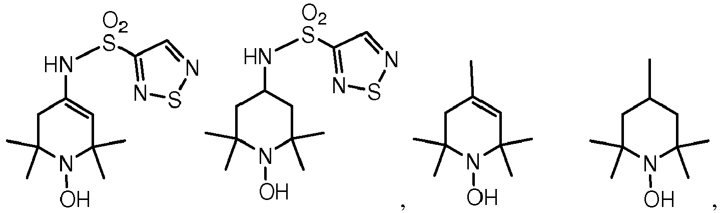

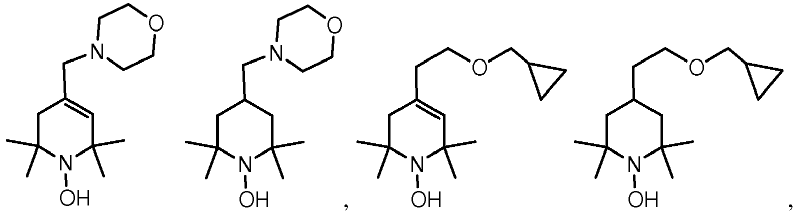

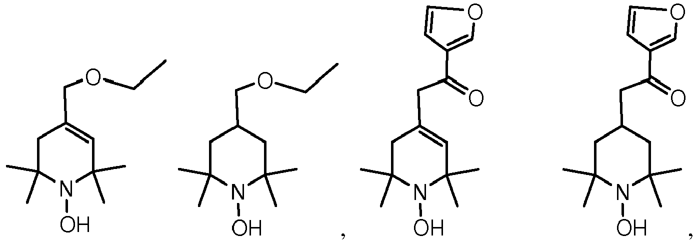

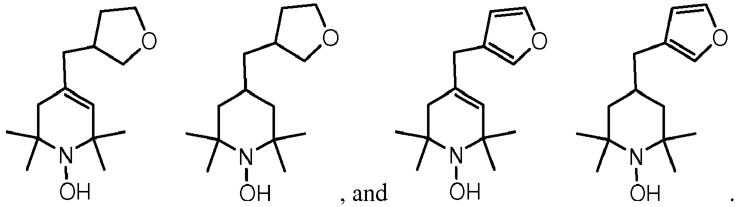

Representative compounds of Formula I or II when n is 0 include:

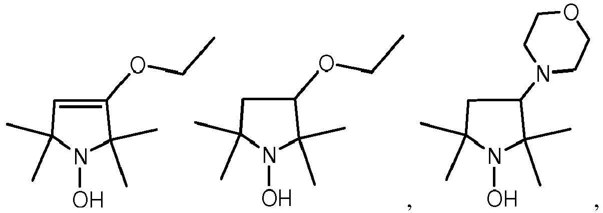

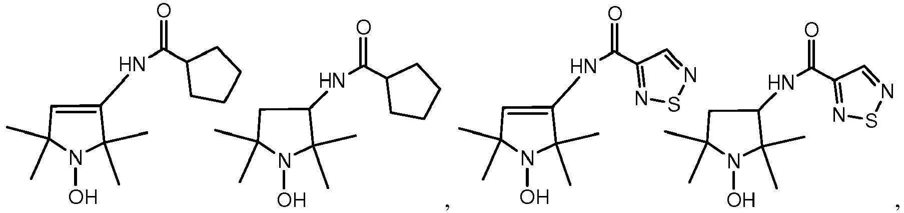

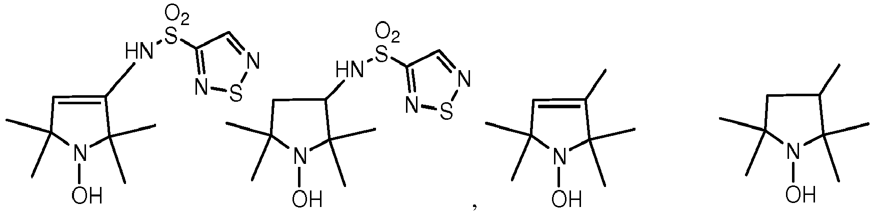

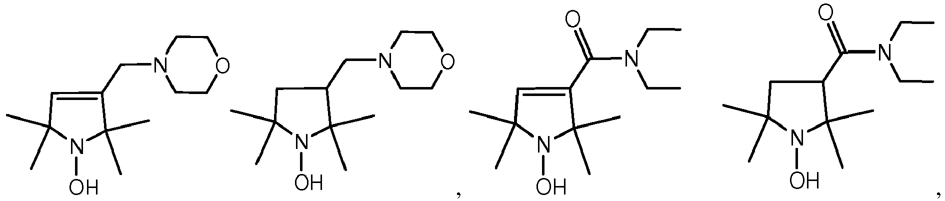

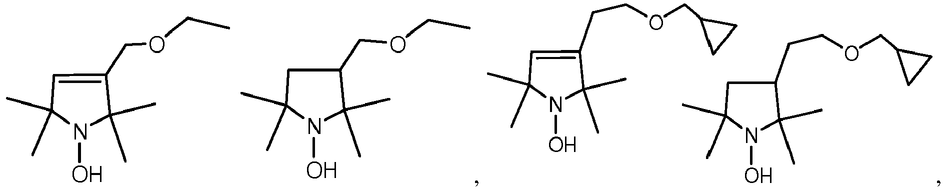

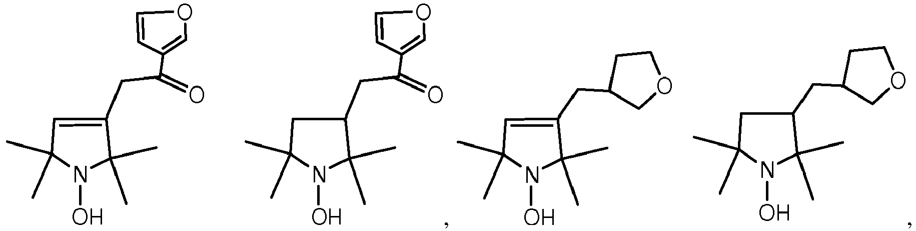

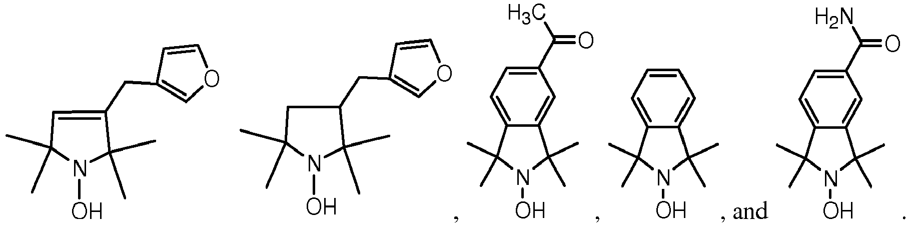

In certain preferred embodiments of compounds of formula I or II, R⁴ is alkyl, or 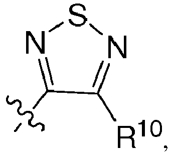 more preferably

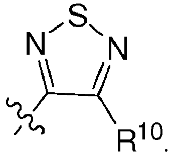

In some preferred embodiments of compounds of formula I or II, R⁵ is H.

In other preferred embodiments of compounds of formula I or II, R⁶ is alkyl, 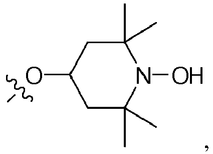 -C(=O)-R¹¹, or -S(=O)₂-R¹¹; more preferably 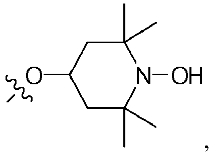 -C(=O)-R¹¹, or -S(=O)₂-R¹¹_{.}

In certain preferred embodiments of compounds of formula I or II, R⁵ and R⁶ taken together with the nitrogen atom to which they are attached form a morpholine ring.

In some preferred embodiments of compounds of formula I or II, at least one of R⁷ and R⁸ is H.

In certain preferred embodiments of compounds of formula I or II, R⁹ is -OH, -CH₂-C(=O)-morpholin-4-yl; or -N(R⁵)R⁶.

In some preferred embodiments of compounds of formula I or II, R¹⁰ is H, morpholinyl, halo, or

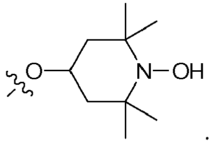

In other preferred embodiments of compounds of formula I or II, R¹¹ is alkyl, cycloalkyl, -NH(3,5-di-*tertiary* butyl-4-hydroxyphenyl), -NH-(4,5-dihydroxy-2-methylphenyl), or 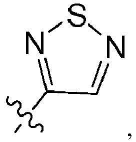 more preferably alkyl, cycloalkyl, or 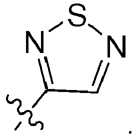 Alternatively preferred in some embodiments of compounds of formula I or II, R¹¹ is -NH(3,5-di-*tertiary* butyl-4-hydroxyphenyl) or -NH-(4,5-dihydroxy-2-methylphenyl).

In certain preferred embodiments of compounds of formula I or II, the compound of formula I or II is:
4-(2,2,6,6-tetramethylpiperidin-1-hydroxy-4-yl)morpholine;
4-(4-(2,2,6,6-tetramethylpiperidin-1-hydroxyl-4-yloxy)-1,2,5-thiazol-3-yl)morpholine;
2,2,3,5,6,6-Hexamethyl-piperidine-1,4-diol;
N-(2,2,6,6-tetramethylpiperidin-1-hydroxyl-4-yl)morpholine-4-carboxamide;
4-cyano-1-hydroxyl-2,2,6,6-tetramethylpiperidine;
4-(4-chloro-1,2,5-thiadiazol-3-yloxyl)-1-hydroxyl-2,2,6,6-tetramethylpiperidine;
1-hydroxyl-4-(4-(2,2,6,6-tetramethylpiperidin-1-hydroxyl-4-yloxy)-1,2,5-thiadiazol-3-yloxy)-2,2,6,6-tetramethylpiperidine;
1,1,3,3-tetramethylisoindolin-2-hydroxyl-5-carboxylic acid;
3,3,5,5-1-hydroxy-tetramethylmorpholine;
1-hydroxy-2,2,5,5-tetramethylpyrrolidine-3-carboxamide;
1-hydroxy-1,2,3,6-tetrahydro-2,2,6,6-tetramethylpyridin-4-yl)methanol;
N-(1-hydroxy-2,2,6,6-tetramethylpiperidin-4-yl)-3-morpholinopropanamide;
4,5-dihydroxy-2-methyl-N-(1-hydroxy-2,2,6,6-tetramethylpiperidin-4-yl)benzamide;
N-(3,5-di-tert-butyl-4-hydroxyphenyl)-1-hydroxy-2,2,6,6-tetramethylpiperidine-4-carboxamide;
1-hydroxy-2,2,6,6-tetramethyl-4-(2H-tetrazol-5-yl)piperidine;
N-(1-hydroxy-2,2,6,6-tetramethylpiperidin-4-yl)cyclopropanecarboxamide;
4-(4-(1-hydroxy-2,2,5,5-tetramethylpyrrolidin-3-yloxy)-1,2,5-thiadiazol-3-yl)morpholine;
1-hydroxy-2,2,5,5-tetramethylpyrrolidin-3-yl)methanol;
(1-hydroxy-2,2,6,6-tetramethylpiperidin-4-yl)methanol;
1-hydroxy-1,2,3,6-tetrahydro-2,2,6,6-tetramethylpyridine;
((1-hydroxy-2,5-dihydro-2,2,5,5-tetramethyl-1H-pyrrol-3-yl)methyl)morpholine;
1-hydroxy-2,2,5,5-tetramethylpyrrolidin-3-one; or
N-(1-hydroxy-2,2,5,5-tetramethylpyrrolidin-3-yl)cyclopropanecarboxamide;
or a pharmaceutically acceptable salt thereof.

In some preferred embodiments of compounds of formula I or II, the compound of formula I or II is present as a hydrochloride salt thereof.

In other preferred embodiments of compounds of formula I or II, the compound is 4-(4-(2,2,6,6-tetramethylpiperidin-1-hydroxyl-4-yloxy)-1,2,5-thiazol-3-yl)morpholine or a pharmaceutically acceptable salt thereof.

In certain preferred embodiments of formula II compounds, A is H.

In some preferred embodiments of formula II compounds, Z is -O-. In other preferred embodiments of formula II compounds, Z is -C(B)(R²)-.

In other preferred embodiments of formula II compounds, B is H, alkyl, aryl, or heteroaralkyl, more preferably H, alkyl, or aryl, still more preferably H or aryl, with H being even more preferred. In other preferred embodiments, B is alkyl or aryl.

In still other preferred embodiments of formula II compounds, A and B taken together form a double bond between the ring atoms through which they are connected, more preferably provided that when A and B form a double bond, R⁴ is other than H.

In other preferred embodiments of formula II compounds, R¹ is H, alkyl, aryl, or halo, more preferably H, alkyl, or aryl, still more preferably H or aryl, with H being even more preferred.

In other preferred embodiments, A and R¹ taken together form =O, more preferably provided that when A and R¹ taken together form =O, then Z is -O-.

In certain embodiments of formula II compounds, R³ is H, alkyl, aryl, or halo, preferably H, aryl, or alkyl, more preferably H or aryl, with H being even more preferred.

In some preferred embodiments of formula II compounds, R² is heteroaryl, -OR⁴, - N(R⁵)R⁶, -ONO₂, -(C=O) N(R⁵)R⁶, or -C[(R⁷)(R⁸)]ₘR⁹. In other preferred embodiments, R² is aryl, -OR⁴, -N(R⁵)R⁶, -C(=O)-aralkyl or -C[(R⁷)(R⁸)]ₘR⁹, more preferably aryl, -OR⁴, -N(R⁵)R⁶, or -C[(R⁷)(R⁸)]ₘR⁹, yet more preferably -OR⁴, -N(R⁵)R⁶, or -C[(R⁷)(R⁸)]ₘR⁹, still more preferably -OR⁴, or -N(R⁵)R⁶, with -OR⁴ being even more preferred. In certain preferred alternative embodiments, at least one of R¹ and R² is aryl, more preferably both are independently aryl.

In still other preferred embodiments of formula II compounds, R¹ and R² taken together with the atoms through which they are connected form an aryl ring. In certain more preferred embodiments, when R¹ and R² taken together with the atoms through which they are connected form an aryl ring, then A and B are absent; or when R² is other than-OH, then B is other than alkyl, aryl, or heteroaralkyl; or when R² is H, then R¹ is H, and A and B taken together form a double bond between the ring atoms through which they are connected; or when R² is - C(=O)NH₂, then A and B are H, and n is 0; or when A is H, B and R² taken together form =O or =CH(R¹²).

In some embodiments of formula II compounds, m is 1, 2, or 3, preferably 1 or 2.

In certain embodiments of formula II compounds, n is 0, 1, or 2; preferably 0 or 1, more preferably 1. Alternatively, n is preferably 0.

In other embodiments of formula II compounds, p is preferably 1 or 2. Alternatively p is preferably O.

In other preferred embodiments of formula II compounds, R⁴ is H, alkyl, aryl, or heteroaryl, more preferably H or alkyl, with H even more preferred. Alternatively preferred, R⁴ is 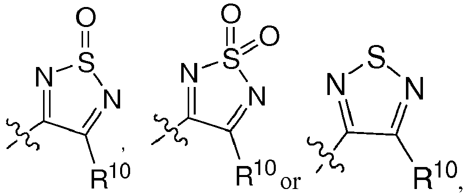 more preferably 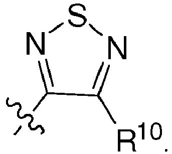 In other embodiments, R⁴ is H, alkyl, aralkyl, heteroaryl, or

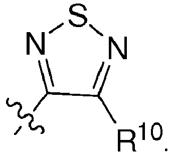

In still other preferred embodiments of formula II compounds, R⁵ is H.

In certain preferred embodiments of formula II compounds, R⁶ is aralkyl, 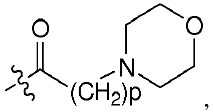 -C(=O)-R¹¹, -C(=NH)-alkyl, or -S(=O)₂-R¹¹, more preferably -C(=O)-R¹¹, - C(=NH)-alkyl, or -S(=O)₂-R¹¹, still more preferably -C(=O)-R¹¹ or -S(=O)₂-R¹¹. In other embodiments, R⁶ is 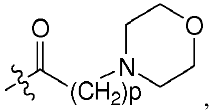 -C(=O)-R¹¹ or -S(=O)₂-R¹¹.

In other preferred embodiments of formula II compounds, R⁵ and R⁶ taken together with the nitrogen atom to which they are attached form a heterocycloalkyl ring, preferably a 5 or 6 membered heterocycloalkyl in which 1 of the heterocycloalkyl ring carbon atoms independently is optionally replaced by -O-, -S-, -NH-, or N-alkyl.

In still other preferred embodiments of formula II compounds, R⁷ and R⁸ are each H or alkyl provided that at least one of R⁷ and R⁸ is H, more preferably wherein both R⁷ and R⁸ are H.

In some preferred embodiments of formula II compounds, R⁹ is -OH, -O-aryl, alkylheteroaryloxy; more preferably -OH.

In certain preferred embodiments of formula II compounds, R¹⁰ is alkyl, aryl, arylheterocycloalkyl, heterocycloalkyl, cyano, carboxy, alkoxycarbonyl, halo, haloarylheterocycloalkyl, heteroaroylheterocycloalkyl, heteroarylheterocycloalkyl, , C(=O)-heterocycloalkyl, 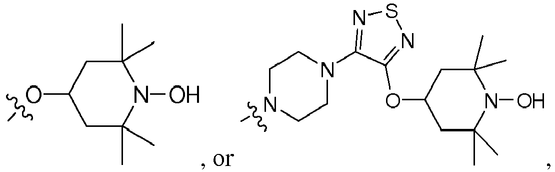 more preferably heterocycloalkyl,

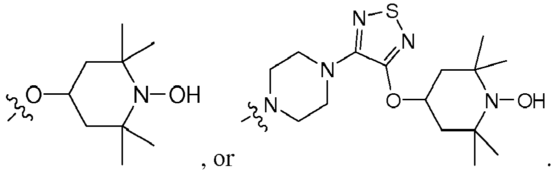

In other preferred embodiments of formula II compounds, R¹¹ is alkyl, aryl, aralkenyl, heterocycloalkyl, halobenzo[1,2,5]oxadiazolyl, heteroarylheterocycloalkyl, heterocycloalkylalkyl -(3,5-di-*tertiary* butyl-4-hydroxyphenyl), -(4,5-dihydroxy-2-methylphenyl), or 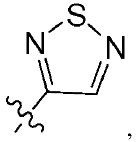 more preferably alkyl, aryl, heterocycloalkyl, aralkenyl or heteroaryl, still more preferably alkyl. In certain preferred embodiments, R¹¹ is alkyl, aryl, aralkenyl, heteroaryl, heterocycloalkyl, -(3,5-di-*tertiary* butyl-4-hydroxyphenyl), -(4,5-dihydroxy-2-methylphenyl), or 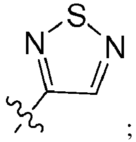 more preferably alkyl, aralkenyl, or

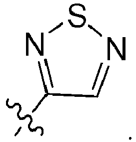

In still other preferred embodiments of formula II compounds, R¹² is -C(=O)- or heterocycloalkylaryl.

In some preferred embodiments of formula II compounds or compositions containing those compounds, the compounds are:
4-(4-(2,2,6,6-tetramethylpiperidin-1-hydroxyl-4-yloxy)-1,2,5-thiadiazol-3-yl)morpholine;
1,3-Dihydroxy-2,2,5,5-Tetramethyl-pyrrolidine;
2,5-dihydro-2,2,5,5-tetramethyl-1-hydroxyl-1H-pyrrol-3-yl)methanol;
4,5-dihydroxy-2-methyl-N-(1-hydroxy-2,2,6,6-tetramethylpiperidin-4-yl)benzamide;
N-(3,5-di-t-butyl-4-hydroxyphenyl)-1-hydroxy-2,2,6,6-tetramethylpiperidine-4-carboxamide;
1-hydroxy-2,2,6,6-tetramethyl-4-(2H-tetrazol-5-yl)piperidine;
N-Hydroxyl-3,3,5,5-tetramethylmorpholin-2-one;
1,4-dihydroxy-4-n-butyl-2,2,6,6-tetramethylpiperidine;
1,4-Dihydroxy-4-phenyl-2,2,6,6-tetrmethylpiperidine;
4-Benzyloxy-1-hydroxy-2,2,6,6-tetramethylpiperidine;
5-(2,5,-dihydro-4-(3,4,5-trimethoxyphenyl)-1-hydroxy-2,2,5,5-tetramethyl-1H-pyrrol-3-yl)-2-methoxybenzaldehyde;
1-Hydroxy-2,3,6-trihydro-4-(3,4,5-trimethoxyphenyl)-2,2,6,6-tetramethylpiperidine;
4-[(4-methylpiperazin-1-yl)]-3-[(2,2,6,6-tetramethyl-1-Hydroxy piperidinyl)]-1,2,5-thiadiazole;
4-(4-(1-hydroxy 2,2,6,6-tetramethylpiperidin-4-yloxy)-1,2,5-thiadiazol-3-yl)thiomorpholine;
4-(4-Fluorophenyl)-1-hydroxyl-2,2,6,6-tetramethylpiperidin-4-ol;
4-O-nitro-1-hydroxy-2,2,6,6- tetramethylpiperidine;
1,4-bis(1-hydroxy-2,26,6-tetramethylpipendin-4-yloxy)-1,2,5-thiadiazol-3-yl)piperazine; or
3,4-dihydro-6-hydroxy-2,5,7,8-tetramethyl-N-(1-hydroxy-2,2,6,6-tetramethylpiperidin-4-yl)-2H-chromene-2-carboxamide;
or a pharmaceutically acceptable salt, preferably a hydrochloride salt, thereof. More preferably, the compounds or compositions containing those compounds are:
4-(4-(2,2,6,6-tetramethylpiperidin-1-hydroxyl-4-yloxy)-1,2,5-thiadiazol-3-yl)morpholine;
1,3-Dihydroxy-2,2,5,5-tetramethyl-pyrrolidine;
2,5-dihydro-2,2,5,5-tetramethyl-1-hydroxyl-1H-pyrrol-3-yl)methanol;
1,4-dihydroxy-4-n-butyl-2,2,6,6-tetramethylpiperidine;
1,4-Dihydroxy-4-phenyl-2,2,6,6-tetrmethylpiperidine;
5-(2,5,-dihydro-4-(3,4,5-trimethoxyphenyl)-1-hydroxy-2,2,5,5-tetramethyl-1H-pyrrol-3-yl)-2-methoxybenzaldehyde; and
4-[(4-methylpiperazin-1-yl)]-3-[(2,2,6,6-tetramethyl-1-Hydroxy piperidinyl)]-1,2,5-thiadiazole;
or a pharmaceutically acceptable salt, preferably a hydrochloride salt, thereof. Still more preferably, the compounds or compositions containing those compounds are:
4-(4-(2,2,6,6-tetramethylpiperidin-1-hydroxyl-4-yloxy)-1,2,5-thiadiazol-3-yl)morpholine; 1,4-dihydroxy-4-n-butyl-2,2,6,6-tetramethylpiperidine;
1,4-Dihydroxy-4-phenyl-2,2,6,6-tetrmethylpiperidine; or
4-[(4-methylpiperazin-1-yl)]-3-[(2,2,6,6-tetramethyl-1-Hydroxy piperidinyl)]-1,2,5-thiadiazole;
or a pharmaceutically acceptable salt, preferably a hydrochloride salt, thereof.

In other preferred embodiments of formula II compounds or compositions containing those compounds, the compounds are:
1-hydroxy-4-methoxy-2,2,6,6-tetramethylpiperidine;
4-(4-(2,2,6,6-tetramethylpiperidin-1-hydroxyl-4-yloxy)-1,2,5-thiadiazol-3-yl)morpholine;
1,3-Dihydroxy-2,2,5,5-tetramethyl-pyrrolidine;
2,5-dihydro-2,2,5,5-tetramethyl-1-hydroxyl-1H-pyrrol-3-yl)methanol;
1,4-dihydroxy-3-bromo-2,2,6,6-tetramethylpiperidine;
1,4-dihydroxy-4-n-butyl-2,2,6,6-tetramethylpiperidine;
1,4-Dihydroxy-4-phenyl-2,2,6,6-tetrmethylpiperidine;
5-(2,5,-dihydro-4-(3,4,5-trimethoxyphenyl)-1-hydroxy-2,2,5,5-tetramethyl-1H-pyrrol-3-yl)-2-methoxybenzaldehyde; or
4-[(4-methylpiperazin-1-yl)]-3-[(2,2,6,6-tetramethyl-1-Hydroxy piperidinyl)]-1,2,5-thiadiazole;
or a pharmaceutically acceptable salt, preferably a hydrochloride salt, thereof. More preferably, the compounds or compositions containing those compounds are:
1-hydroxy-4-methoxy-2,2,6,6-tetramethylpiperidine;
4-(4-(2,2,6,6-tetramethylpiperidin-1-hydroxyl-4-yloxy)-1,2,5-thiadiazol-3-yl)morpholine;
1,4-dihydroxy-4-n-butyl-2,2,6,6-tetramethylpiperidine;
1,4-Dihydroxy-4-phenyl-2,2,6,6-tetrmethylpiperidine; or
4-[(4-methylpiperazin-1-yl)]-3-[(2,2,6,6-tetramethyl-1-Hydroxy piperidinyl)]-1,2,5-thiadiazole;
or a pharmaceutically acceptable salt, preferably a hydrochloride salt, thereof. thereof.

The compounds employed in the methods of the present invention may exist in prodrug form. As used herein, "prodrug" is intended to include any covalently bonded carriers which release the active parent drug, for example, as according to Formula I or II, or other formulas or compounds employed in the methods of the present invention *in vivo* when such prodrug is administered to a mammalian subject. Since prodrugs are known to enhance numerous desirable qualities of pharmaceuticals (e.g., solubility, bioavailability, manufacturing, etc.) the compounds employed in the present methods may, if desired, be delivered in prodrug form. Thus, the present invention contemplates methods of delivering prodrugs. Prodrugs of the compounds employed in the present invention, for example Formula I or II, may be prepared by modifying functional groups present in the compound in such a way that the modifications are cleaved, either in routine manipulation or *in vivo,* to the parent compound.

Accordingly, prodrugs include, for example, compounds described herein in which a hydroxy, amino, or carboxy group is bonded to any group that, when the prodrug is administered to a mammalian subject, cleaves to form a free hydroxyl, free amino, or carboxylic acid, respectively. Examples include, but are not limited to, acetate, formate and benzoate derivatives of alcohol and amine functional groups; and alkyl, carbocyclic, aryl, and alkylaryl esters such as methyl, ethyl, propyl, *iso*-propyl, butyl, isobutyl, *sec*-butyl, *tert*-butyl, cyclopropyl, phenyl, benzyl, and phenethyl esters, and the like.

The compounds employed in the methods of the present invention may be prepared in a number of ways well known to those skilled in the art. The compounds can be synthesized, for example, by the methods described below, or variations thereon as appreciated by the skilled artisan. All processes disclosed in association with the present invention are contemplated to be practiced on any scale, including milligram, gram, multigram, kilogram, multikilogram or commercial industrial scale.

As discussed in detail above, compounds employed in the present methods may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

As will be readily understood, functional groups present may contain protecting groups during the course of synthesis. Protecting groups are known *per se* as chemical functional groups that can be selectively appended to and removed from functionalities, such as hydroxyl groups and carboxy groups. These groups are present in a chemical compound to render such functionality inert to chemical reaction conditions to which the compound is exposed. Any of a variety of protecting groups may be employed with the present invention. Preferred protecting groups include the benzyloxycarbonyl group and the tert-butyloxycarbonyl groups. Preferred hydroxyl protecting groups include the benzyl and the tertiary-butyldimethylsilyl groups. Other preferred protecting groups that may be employed in accordance with the present invention may be described in Greene, T.W. and Wuts, P.G.M., Protective Groups in Organic Synthesis 3d. Ed., Wiley & Sons, 1991**.**

The compounds are preferably combined with a pharmaceutical carrier selected on the basis of the chosen route of administration and standard pharmaceutical practice as described, for example, in Remington's Pharmaceutical Sciences (Mack Pub. Co., Easton, PA, 1980), the disclosure of which is hereby incorporated herein by reference, in its entirety.

Although the compounds of the present invention may be administered as the pure chemicals, it is preferable to present the active ingredient as a pharmaceutical composition. The invention thus further provides pharmaceutical compositions comprising one or more of the cannabinoid receptor modulator compounds of the present invention, for example compounds of formula Formula I or II, together with one or more pharmaceutically acceptable carriers therefore and, optionally, other therapeutic and/or prophylactic ingredients. The carrier(s) must be acceptable in the sense of being compatible with the other ingredients of the composition and not deleterious to the recipient thereof.

In accordance with certain embodiments of the present invention, the compositions and methods of the invention may further comprise an anti-angiogenic agent, preferably wherein the anti-angiogenic agent is an antioxidant, VEGF antagonist, bFGF antagonist, NOS antagonist, or a combination thereof. In accordance with other embodiments of the present invention, the compositions and methods of the invention may further comprise a chemotherapeutic agent, preferably wherein the chemotherapeutic agent is doxorubicin.

In accordance with other embodiments of the present invention, the compositions and methods for treating or inhibiting hepatitis in a patient, comprise administering to the patient in need thereof a therapeutically sufficient amount of hydroxylamine compound of formula I or II. The amount administered is sufficient to treat, inhibit, or slow the progression of hepatitis. In some aspects of the invention, the compositions of the invention are used synergistically with other anti-hepatic or anti-inflammatory agents, or with antioxidants.

The methods of the present invention may also utilize compositions comprising a pharmaceutically acceptable carrier or diluent and a hydroxylamine compound having an N-hydroxy piperidine portion corresponding to a compound of formula I or II bound to a solubility modifying portion, the compound having a solubility in water at 25°C of at least about 0.25% by weight and a water/n-octanol partition coefficient at 25°C of at least about 5. The composition may have the N-hydroxy piperidine portion cleavable from the compound under conditions found in biological tissues, such as found in the eye. The portion that substantially contains the hydroxylamine moiety of formula I or II may be cleaved enzymatically.

In accordance with some embodiments of the present invention, the compositions and methods of the invention for treating pathologies mediated by complement activation in a subject and/or methods for inhibiting drusen formation in a subject by administering to the subject a composition comprise a pharmaceutically acceptable carrier and a hydroxylamine compound of formula I or II in an amount therapeutically sufficient to treat pathologies mediated by complement activation and/or to inhibit drusen formation, respectively. Examples of ophthalmic pathologies that can be treated by the compositions and methods of the invention include retinopathy and age-related macular degeneration (AMD). Moreover, it is becoming apparent that chronic inflammation, such as results from complement activation, is factor in many of the important diseases of aging. Accordingly, other pathologies that can be treated by the compositions and methods of the invention include age-related disorders, such as Alzheimer's disease (AD) , Parkinson's disease (PD) , ALS and multiple sclerosis, atherosclerosis, heart disease, skin elastosis, glomerular basement membrane disease and numerous amyloidoses, to name a few. The compositions and methods can be used in any animal, and preferably are used in mammals, and most preferably are used in humans.

The compounds of the invention may be administered in an effective amount by any of the conventional techniques well-established in the medical field. The compounds employed in the methods of the present invention including, for example, the compounds of Formula I or II, may be administered by any means that results in the contact of the active agents with the agents' site or site(s)of action in the body of a patient. The compounds may be administered by any conventional means available for use in conjunction with pharmaceuticals, either as individual therapeutic agents or in a combination of therapeutic agents. For example, they may be administered as the sole active agents in a pharmaceutical composition, or they can be used in combination with other therapeutically active ingredients.

Compounds of the present invention can be administered to a mammalian host in a variety of forms adapted to the chosen route of administration, *e.g.*, orally or parenterally. Parenteral administration in this respect includes administration by the following routes: intravenous, intramuscular, subcutaneous, intraocular, intrasynovial, transepithelial including transdermal, ophthalmic, sublingual and buccal; topically including ophthalmic, dermal, ocular, rectal and nasal inhalation via insufflation, aerosol and rectal systemic.

Solid forms can be prepared according to any means suitable in the art. For example, capsules are prepared by mixing the composition with a suitable diluent and filling the proper amount of the mixture in capsules. Tablets are prepared by direct compression, by wet granulation, or by dry granulation. Their formulations usually incorporate diluents, binders, lubricants and disintegrators as well as the compound. Nonlimiting examples of diluents include various types of starch, cellulose, crystalline cellulose, microcrystalline cellulose, lactose, fructose, sucrose, mannitol, kaolin, calcium phosphate or sulfate, inorganic salts such as sodium chloride and powdered sugar. Powdered cellulose derivatives are also useful. Nonlimiting examples of tablet binders include starch, gelatin and sugars such as lactose, fructose, glucose and the like. Natural and synthetic gums can also be used, including acacia, alginates, methylcellulose, polyvinylpyrrolidine and the like. Polyethylene glycol, ethylcellulose and waxes can also serve as binders.

A lubricant can be used in a tablet formulation to prevent the tablet and punches from sticking in the die. The lubricant can be chosen from such slippery solids as talc, magnesium and calcium stearate, stearic acid and hydrogenated vegetable oils. Tablet disintegrators are substances which swell when-wetted to break up the tablet and release the compound, and include starches such as corn and potato starches, clays, celluloses, algins and gums, methylcellulose, agar, bentonite, wood cellulose, powdered natural sponge, cation-exchange resins, alginic acid, guar gum, citrus pulp, carboxymethyl cellulose, and sodium lauryl sulfate. Tablets can be coated with sugar as a flavor and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

Also included are liquid formulations and solid form preparations which are intended to be converted, shortly before use, to liquid form preparations. Such liquid forms include solutions, suspensions, syrups, slurries, and emulsions. Liquid preparations may be prepared by conventional means with pharmaceutically acceptable additives such as suspending agents (*e.g*., sorbitol syrup, cellulose derivatives or hydrogenated edible fats or oils); emulsifying agents (*e.g.*, lecithin or acacia); non-aqueous vehicles (*e.g.*, almond oil, oily esters, or fractionated vegetable oils); and preservatives (*e.g.*, methyl or propyl-p-hydroxybenzoates or sorbic acid). These preparations may contain, in addition to the active agent, colorants, flavors, stabilizers, buffers, artificial and natural sweeteners, dispersants, thickeners, solubilizing agents, and the like. The compositions may be in powder form for constitution with a suitable vehicle such as sterile water, saline solution, or alcohol, before use.

Compositions for use in topical administration include, *e.g.*, liquid or gel preparations suitable for penetration through the skin such as creams, liniments, lotions, ointments or pastes, and drops suitable for delivery to the eye, ear or nose.

In some embodiments, the present compositions include creams, drops, liniments, lotions, ointments and pastes are liquid or semi-solid compositions for external application. Such compositions may be prepared by mixing the active ingredient(s) in powdered form, alone or in solution or suspension in an aqueous or non-aqueous fluid with a greasy or non-greasy base. The base may comprise complex hydrocarbons such as glycerol, various forms of paraffin, beeswax; a mucilage; a mineral or edible oil or fatty acids; or a macrogel. Such compositions may additionally comprise suitable surface active agents such as surfactants, and suspending agents such as agar, vegetable gums, cellulose derivatives, and other ingredients such as preservatives, antioxidants, and the like.

The compositions can also be formulated for injection into the subject. For injection, the compositions of the invention can be formulated in aqueous solutions such as water or alcohol, or in physiologically compatible buffers such as Hanks's solution, Ringer's solution, or physiological saline buffer. The solution may contain formulatory agents such as suspending, stabilizing and/or dispersing agents. Injection formulations may also be prepared as solid form preparations which are intended to be converted, shortly before use, to liquid form preparations suitable for injection, for example, by constitution with a suitable vehicle, such as sterile water, saline solution, or alcohol, before use.

The compositions may also be formulated in sustained release vehicles or depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt. Liposomes and emulsions are well-known examples of delivery vehicles suitable for use as carriers for hydrophobic vehicles or carriers for hydrophilic drugs.

The compositions may further include one or more antioxidants. Exemplary reducing agents include mercaptopropionyl glycine, N-acetylcysteine, β-mercaptoethylamine, glutathione, ascorbic acid and its salts, sulfite, or sodium metabisulfite, or similar species. In addition. antioxidants can also include natural antioxidants such as vitamin E, C, leutein, xanthine, beta carotene and minerals such as zinc and selenium.

The administration of these additional compounds may be simultaneous with the administration of the hydroxylamine compounds, or may be administered in tandem, either before or after the administration of the hydroxylamine compounds, as necessary. Any suitable protocol may be devised whereby the various compounds to be included in the combination treatment are administered within minutes, hours, days, or weeks of each other. Repeated administration in a cyclic protocol is also contemplated to be within the scope of the present invention.

Further, the methods of the invention include combination therapy. In some embodiments of the invention, the hydroxylamines or derivatives are administered with another compound known in the art that is useful for treating a disease or disorder associated with pathogenic angiogenesis. The other compound(s) known in the art may be administered simultaneously with the hydroxylamine compounds, or may be administered sequentially.

For example, the hydroxylamine compounds can be administered in combination with one or more additional anti-angiogenic agents. In general, anti-angiogenic agents can be any known inhibitor or down regulator of an angiogenic agent or an inhibitor of the cell signaling pathway promoted by an angiogenic agent, including, but not limited to, cartilage-derived factors, angiostatic steroids, angiostatic vitamin D analogs, angiostatin, endostatin, and verostatin. There are some anti-angiogenic agents that are thought to affect a specific angiogenic factor, e.g., the angiogenic factor angiogenin. Anti-angiogenic agents specific for angiogenin include monoclonal antibodies that bind angiogenin, human placental ribonuclease inhibitor, actin, and synthetic peptides corresponding to the C-terminal region of angiogenin. Anti-angiogenic agents of microbial origin are also contemplated herein. Such agents include anthracycline, 15-deoxyspergualin, D-penicillamine, eponemycin, fumagillin, herbimycin A, rapamycin and neomycin. The term "neomycin" refers to an antibiotic complex composed of neomycins A, B and C, which together is also known as Mycifradin, Myacyne, Fradiomycin, Neomin, Neolate, Neomas, Nivemycin, Pimavecort, Vonamycin Powder V, and analogs thereof.

The pharmaceutical compositions of the invention may optionally comprise one or more anti-neoplastic agents, which include, but are not limited to, alkaloids such as docetaxel, etoposide, trontecan, paclitaxel, teniposide, topotecan, vinblastine, vincristine, and vindesine; alkylating agents such as busulfan, improsulfan, piposulfan, aziridines, benzodepa, carboquone, meturedepa, uredepa, altretamine, triethylenemelamine, triethylenephosphoramide, triethylenethiophosphoramide, chlorambucil, chloraphazine, cyclophosphamide, estramustine, ifosfamide, mechlorethamine, mechlorethamine oxide hydrochloride, melphalan, novembichin, perfosfamide, phenesterine, prednimustine, trofosfamide, uracil mustard, carmustine, chlorozotocin, fotemustine, lomustine, nimustine, ranimustine, dacarbazine, mannomustine, mitobronitol, mitolactol, pipobroman, temozolomide; antibiotics and analogues such as aclacinomycinsa actinomycin F₁, anthramycin, azaserine, bleomycins, cactinomycin, carubicin, carzinophilin, chromomycins, dactinomycin, daunorubicin, 6-diazo-5-oxo-L-norleucine, doxorubicin, epirubicin, idarubicin, menogaril, mitomycins, mycophenolic acid, nogalamycin, olivomycins, peplomycin, pirarubicin, plicamycin, porfiromycin, puromycin, streptonigrin, streptozocin, tubercidin, zinostatin, zorubicin; antimetabolites such as denopterin, edatrexate, methotrexate, piritrexim, pteropterin, Tomudex^{®}, trimetrexate, cladribine, fludarabine, 6-mercaptopurine, thiamiprine, thioguanine, ancitabine, azacitidine, 6-azauridine, carnofur, cytarabine, doxifluridine, emitefur, enocitabune, floxuridine, fluorouracil, gemcitabine, tegafur; L-Asparaginase; immunomodulators such as interferon-.alpha., interferon-.beta., interferon-.gamma., interleukin-2, lentinan, propagermanium, PSK, roquinimex, sizofican, ubenimex; platimum complexes such as carboplatin, cisplatin, miboplatin, oxaliplatin; aceglarone; amsacrine; bisantrene; defosfamide; demecolcine; diaziquone; eflornithine; elliptinium acetate; etoglucid; fenretinide; gallium nitrate; hydroxyurea; lonidamine; miltefosine; mitoguazone; mitoxantrone; mopidamol; nitracine; pentostain; phenamet; podophyllinic acid 2-ethyl-hydrazide; procabazine; razoxane; sobuzoxane; spirogermanium; tenuzonic acid; triaziquone; 2,2',2"trichlorotriethylamine; urethan; antineoplastic hormone or analogues such as calusterone, dromostanolone, epitiostanol, mepitiostane, testolacone, aminoglutethimide, mitotane, trilostane, bicalutamide, flutamide, nilutamide, droloxifene, tamoxifen, toremifene, aminoglutethimide, anastrozole, fadrozole, formestane, letrozole, fosfestrol, hexestrol, polyestradiol phosphate, buserelin, goserelin, leuprolide, triptorelin, chlormadinone acetate, medroxyprogesterone, megestrol acetate, melengestrol; porfimer sodium; batimastar; and folinic acid. For a description of these and other antineoplastic agents that may comprise the pharmaceutical composition of the invention, see The Merck Index, 12th ed.

The therapeutic compounds of this invention may be administered to a patient alone or in combination with a pharmaceutically acceptable carrier. As noted above, the relative proportions of active ingredient and carrier may be determined, for example, by the solubility and chemical nature of the compounds, chosen route of administration and standard pharmaceutical practice.

Pathological angiogenesis or proliferation of endothelial cells has been associated with many diseases or conditions, including hyperproliferative and neoplastic diseases and inflammatory diseases and disorders, as listed in detail above. The methods of the invention may be adapted for the treatment of any condition in which angiogenesis is a causal factor. Compositions can be administered by any of the routes conventionally used for drug administration. Such routes include, but are not limited to, oral, topical parenteral and by inhalation. Parenteral delivery may be intraperitoneal, intravenous, perioral, subcutaneous, intramuscular, intraarterial, etc. The disclosed compositions can be administered in conventional dosage forms prepared by combining with standard pharmaceutically acceptable carriers according to procedures known in the art. Such combinations may involve procedures such as mixing, granulating, compressing and dissolving the appropriate ingredients.

The form and nature of the pharmaceutically acceptable carrier is controlled by the amounts of the active ingredient to which it is combined, the route of the administration, and other well-known variables. The active ingredient can be one of the present compounds, i.e., hydroxylamines or the ester derivatives thereof. As used herein, the term "carrier" refers to diluents, excipients and the like for use in preparing admixtures of a pharmaceutical composition. The term "pharmaceutically acceptable" means approved by a regulatory agency of the Federal or a state government or listed in the U.S. Pharmacopeia or other generally recognized pharmacopeia for use in animals, and more particularly in humans. Such pharmaceutically acceptable carriers or diluents and methods for preparing are well known in the art (see, e.g., Remington's Pharmaceutical Sciences, Meade Publishing Col., Easton, Pa., latest edition; the Handbook of Pharmaceutical Excipients, APhA publications, 1986).

Pharmaceutically acceptable carriers may be, for example, a liquid or solid. Liquid carriers include, but are not limited, to water, saline, buffered saline, dextrose solution, preferably such physiologically compatible buffers as Hank's or Ringer's solution, physiological saline, a mixture consisting of saline and glucose, and heparinized sodium-citrate-citric acid-dextrose solution and the like, preferably in sterile form. Exemplary solid carrier include agar, acacia, gelatin, lactose, magnesium stearate, pectin, talc and like.

Administration of the compositions can be by infusion or injection (intravenously, intraarterially, intramuscularly, intracutaneously, subcutaneously, intrathecal, intraduodenally, intraperitoneally, and the like). The compositions can also be administered intranasally, vaginally, rectally, orally, topically, or transdermally. Preferably, the compositions are administered orally. Administration can be at the direction of a physician.

For buccal administration, the compositions may take the form of tablets, troche or lozenge formulated in conventional manner. Compositions for oral or buccal administration, may be formulated to give controlled release of the active compound. Such formulations may include one or more sustained-release agents known in the art, such as glyceryl mono-stearate, glyceryl distearate and wax.

Various alternative pharmaceutical delivery systems may be employed. Non-limiting examples of such systems include liposomes and emulsions. Certain organic solvents such as dimethylsulfoxide also may be employed. Additionally, the compounds may be delivered using a sustained-release system, such as semipermeable matrices of solid polymers containing the therapeutic agent. The various sustained-release materials available are well known by those skilled in the art. Sustained-release capsules may, depending on their chemical nature, release the compounds over a range of several days to several weeks to several months.

The compositions utilized in accordance with the inventive methods may contain more than one hydroxylamine compound. In some embodiments, two or more hydroxylamines are administered simultaneously. In other embodiments, they are administered sequentially.

The compositions of the invention for treating hepatitis may also be co-administered with other well known therapeutic agents that are selected for their particular usefulness against the condition that is being treated. For example, such therapeutic agents can be pain relievers, anti-inflammatory agents, antibiotics, anti-viral agents, anti-cirrhotic, or other known agents that treat or inhibit hepatitis.

In general, anti-hepatic agents can be any known inhibitor or down regulator of inflammation and the inflammatory response. Non-limiting examples of anti-hepatic agents include COX-2 inhibitors, lamivudine, interferon alpha-2a, interferon alpha-2b, interferon-n3, interferon-alfacon, peginterferon alpha-2a, peginterferon alpha-2b, and ribavirus. Non-limiting examples of anti-inflammatory agents include non-steroidal anti-inflammatory drugs (NSAIDs), corticosteroids, lactoferrin, and herbs and herbal extracts such as *Silybum marianum* (milk thistle), Phyllanthus, glycyrrhizin (licorice root extract), *Picrorhiza kurroa, Cudrania cochinchinensis var. gerontogea, Bidens pilosa,* Glossogyne tenuifolia, scoparone from *Artemisia capillaries,* and *Sargassum polycystum,* among others. (See, Ishikado A et al. (2005) Biol. Pharm. Bull. 28:1717-21; Dhiman RK et al. (2005) Dig. Dis. Sci. 50:1807-12; We MJ et al. (2005) Life Sci. 76:1135-46; Jang S et al. (2005) Arch. Pharm. Res. 28:203-8; Raghavendran HR (2005) Mol. Cell Biochem. 276:89-96; Abajo C et al. (2004) J. Ethnopharmacol. 93:319-23; Lin CC et al. (1999) Am. J. Chin. Med. 27:227-39; and, Luper S (1998) Altern. Med. Rev. 3:410-21).

In the inventive methods, the compositions comprise a concentration of a hydroxylamine compound in a range of about 0.01% to about 90% of the dry matter weight of the composition. A daily dose range of about 0.01 mg/kg to about 100 mg/kg of the weight of the subject is preferred. Preferably, the daily dose ranges from about 0.1 mg/kg to about 50 mg/kg of the weight of the subject. More preferably, the daily dose ranges from about 1 mg/kg to about 10 mg/kg of the weight of the subject.

In preferred embodiments, administration of the compositions comprising hydroxylamine compounds to a subject will achieve a concentration of the hydroxylamine component in the range of about 0.1 µM to about 10 mM in the tissues and fluids of the subject, preferably in the liver or biliary tissue. In some embodiments, the range is from 1 µm to 5 mM, in other embodiments the range is about 10 µM to 2.5 mM. In still other embodiments, the range is about 50 µM to 1 mM. Most preferably the range of hydroxylamine concentration will be from 1 to 100 µM in the tissues and fluids of the subject, preferably in the liver. In embodiments that include a reducing agent, either within the formulation or administered separately, the concentration of the reducing agent will be from 1 µM to 5 mM in the tissues and fluids of the subject to which the composition is administered, particularly in the liver, preferably in the range of 10 µM to 2 mM. The concentrations of the components of the composition are adjusted appropriately to the route of administration, by typical pharmacokinetic and dilution calculations, to achieve such local concentrations.

Treatment can be initiated with smaller dosages that are less than the optimum dose of the hydroxylamine compound, followed by an increase in dosage over the course of the treatment until the optimum effect under the circumstances is reached. If needed, the total daily dosage may be divided and administered in portions throughout the day.

For effective treatment of hepatitis, one skilled in the art may recommend a dosage schedule and dosage amount adequate for the subject being treated. It may be preferred that dosing occur one to four times daily for as long as needed. The dosing may occur less frequently if the compositions are formulated in sustained delivery vehicles. The dosage schedule may also vary depending on the active drug concentration, which may depend on the needs of the subject.

Techniques and formulations for administering above-described compositions may be found in Remington's Pharmaceutical Sciences, Meade Publishing Col., Easton, Pa., 20th edition (2003).

In order to minimize contact, one embodiment of this invention where the product is orally administered provides for a combination product wherein one active ingredient is enteric coated. By enteric coating one or more of the active ingredients, it is possible not only to minimize the contact between the combined active ingredients, but also, it is possible to control the release of one of these components in the gastrointestinal tract such that one of these components is not released in the stomach but rather is released in the intestines. Another embodiment of this invention where oral administration is desired provides for a combination product wherein one of the active ingredients is coated with a sustained-release material that effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low-viscosity grade of hydroxypropyl methylcellulose (HPMC) or other appropriate materials as known in the art, in order to further separate the active components. The polymer coating serves to form an additional barrier to interaction with the other component.

Dosage forms of the combination products of the present invention wherein one active ingredient is enteric coated can be in the form of tablets such that the enteric coated component and the other active ingredient are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present invention can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

These as well as other ways of minimizing contact between the components of combination products of the present invention, whether administered in a single dosage form or administered in separate forms but at the same time by the same manner, will be readily apparent to those skilled in the art, once armed with the present disclosure.

It will be further appreciated that the amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The dose may also be provided by controlled release of the compound, by techniques well known to those in the art.

In certain preferred embodiments of the methods of treating a patient having a disease state that involves angiogenesis, the methods further include co-administering an additional agent, such as an antioxidant, a reducing agent, an additional anti-angiogenic agent, or an antineoplastic agent.

The ophthalmic compounds are formulated into compositions for application to the eye of patients in need of therapy. Thus, such compositions are adapted for pharmaceutical use as an eye drop or in contact lenses, inserts or the like, as described in greater detail below. Accordingly, formulation of compound into sterile water containing any desired diluents, salts, pH modifying materials and the like as are known to persons skilled in the pharmaceutical formulations art may be performed in order to achieve a solution compatible with administration to the eye. It may be that eye drops, inserts, contact lenses, gels and other topical liquid forms may require somewhat different formulations. All such formulations consistent with direct administration to the eye are comprehended hereby.

The ophthalmic compositions may also have antioxidants, in addition to the oral antioxidants of the combination therapy, in ranges that vary depending on the kind of antioxidant used. The usage also depends on the amount of antioxidant needed to allow at least 2 years shelf-life for the pharmaceutical composition. One or more antioxidants may be included in the formulation. Certain commonly used antioxidants have maximum levels allowed by regulatory authorities. As such, the amount of antioxidant(s) to be administered should be enough to be effective while not causing any untoward effect. Such doses may be adjusted by a physician as needed, within the maximum levels set by regulatory authorities, and is well within the purview of the skilled artisan to determine the proper and effective dose. Reasonable ranges are about 0.01% to about 0.15% weight by volume of EDTA, about 0.01% to about 2.0% weight volume of sodium sulfite, and about 0.01% to about 2.0% weight by volume of sodium metabisulfite. One skilled in the art may use a concentration of about 0.1 % weight by volume for each of the above. N-Acetylcysteine may be present in a range of about 0.1% to about 5.0% weight by volume, with about 0.1% to about 10% of hydroxylamine concentration being preferred. Ascorbic acid or salt may also be present in a range of about 0.1% to about 5.0% weight by volume with about 0.1% to about 10% weight by volume of hydroxylamine concentration preferred. Other sulfhydryls, if included, may be the same range as for N-acetylcysteine. Other exemplary compounds include mercaptopropionyl glycine, N-acetyl cysteine, β-mercaptoethylamine, glutathione and similar species, although other anti-oxidant agents suitable for ocular administration, e.g. ascorbic acid and its salts or sulfite or sodium metabisulfite may also be employed.

A buffering agent may be used to maintain the pH of eye drop formulations in the range of about 4.0 to about 8.0; this is necessary to prevent corneal irritation. Because the compounds of this invention are esters, the pH is preferably maintained at about 3.5 to about 6.0, preferably about 4.0 to about 5.5, in order to prevent hydrolysis of the ester bond and to ensure at least a 2-year shelf life, for the product. This pH also ensures that most of the hydroxylamine is in its protonated form for highest aqueous solubility. The buffer may be any weak acid and its conjugate base with a pKa of about 4.0 to about 5.5; e.g. acetic acid/sodium acetate; citric acid/sodium citrate. The pKa of the hydroxylamines is about 6.0. For direct intravitreal or intraocular injection, formulations should be at pH 7.2 to 7.5, preferably at pH 7.3-7.4.

The ophthalmic compositions may also include tonicity agents suitable for administration to the eye. Among those suitable is sodium chloride to make formulations of the present invention approximately isotonic with 0.9% saline solution.

In certain embodiments, the ophthalmic compounds are formulated with viscosity enhancing agents. Exemplary agents are hydroxyethylcellulose, hydroxypropylcellulose, methylcellulose, and polyvinylpyrrolidone. The viscosity agents may exists in the compounds up to about 2.0% weight by volume. It may be preferred that the agents are present in a range from about 0.2% to about 0.5% weight by volume. A preferred range for polyvinylpyrrolidone may be from about 0.1% to about 2.0%weight by volume. One skilled in the art may prefer any range established as acceptable by the Food and Drug Administration.

The ophthalmic compositions may have co-solvents added if needed. Suitable cosolvents may include glycerin, polyethylene glycol (PEG), polysorbate, propylene glycol, mannitol and polyvinyl alcohol. The presence of the co-solvents may exist in a range of about 0.2% to about 4.0% weight by volume. It may be preferred that mannitol may be formulated in the ophthalmic compounds in a range of about 0.5% to about 4.0% weight by volume. It may also be preferred that polyvinyl alcohol may be formulated in the ophthalmic compounds in a range of about 0.1% to about 4.0% weight by volume. One skilled in the art may prefer ranges established as acceptable by the Food and Drug Administration.

Preservatives may be used in the invention within particular ranges. Among those preferred are up to 0.013% weight by volume of benzalkonium chloride, up to 0.013% weight by volume of benzethonium chloride, up to 0.5% weight by volume of chlorobutanol, up to 0.004% weight by volume or phenylmercuric acetate or nitrate, up to 0.01 % weight by volume of thimerosal, and from about 0.01% to about 0.2% weight by volume of methyl or propylparabens.

Formulations for injection are preferably designed for single-use administration and do not contain preservatives. Injectable solutions should have isotonicity equivalent to 0.9% sodium chloride solution (osmolality of 290-300 mOsmoles). This may be attained by addition of sodium chloride or other co-solvents as listed above, or excipients such as buffering agents and antioxidants, as listed above. Injectable formulations are sterilized and, in one embodiment, supplied in single-use vials or ampules. In another embodiment, injectable products may be supplied as sterile, freeze-dried solids for reconstitution and subsequent injection.

The ophthalmic compositions may contain more than one ophthalmic compound. In some embodiments, the ophthalmic compounds are administered simultaneously. In other embodiments, the ophthalmic compounds are administered sequentially.

In some embodiments of the invention, the compound(s) of the invention are administered with another compound known in the art that is useful for treating a disease or disorder that is the target of the ophthalmic compounds. Thus the composition of the invention may further contain at least one other compound known in the art for treating the disease or disorder to be treated. The other compound(s) known in the art may be administered simultaneously with the compound(s) of the invention, or may be administered sequentially. Similarly, the methods of the invention include using such combination therapy, as described in greater detail below.

Because the aqueous and vitreous humors exists in a highly reducing redox state, particularly nearest to the lens, it may be advantageous to include at least one reducing agent in ophthalmologic formulations in accordance with the invention, or to dose separately with a reducing agent to maintain the hydroxylamine in its reduced form. Preferred reducing agents may be N-acetylcysteine, ascorbic acid or a salt form, and sodium sulfite or metabisulfite, with ascorbic acid and/or N-acetylcysteine or glutathione being particularly suitable for injectable solutions. A combination of N-acetylcysteine and sodium ascorbate may be used in various formulations. A metal chelator antioxidant, such as EDTA (ethylenediaminetetraacetic acid) or possibly DTPA (diethylenetriaminepentaacetic acid) may also be added to keep the hydroxylamine in the reduced form.

Compositions comprising the ophthalmic compounds may be delivered to the eye of a patient in one or more of several delivery modes known in the art. In a preferred embodiment, the compositions are topically delivered to the eye in eye drops or washes. In another embodiment, the compositions are delivered in a topical ophthalmic ointment. In another embodiment, the compositions may be delivered to various locations within the eye *via* periodic subconjunctival or intraocular injection, or by infusion in an irrigating solution such as BSS® or BSS PLUS® (Alcon USA, Fort Worth, TX) or by using pre-formulated solutions of the hydroxylamines in compositions such as BSS® or BSS PLUS®. In one embodiment, the use of the ophthalmic compounds in vitrectomy may be effective in reducing or preventing the development of vitrectomy-associated cataracts.

Alternatively, the compositions may be applied in other ophthalmologic dosage forms known to those skilled in the art, such as pre-formed or *in situ*-formed gels or liposomes, for example as disclosed in U.S. Patent 5,718,922 to Herrero-Vanrell. A direct injection of drugs into the vitreous body used for treating diseases has been used, in which microspheres or liposomes were used to release drugs slowly (Moritera, T. et al. "Microspheres of biodegradable polymers as a drug-delivery system in the vitreous" Invest. Ophthalmol. Vis. Sci. 1991 32(6): 1785-90).

In another embodiment, the composition may be delivered to or through the lens of an eye in need of treatment *via* a contact lens (e.g. Lidofilcon B, Bausch & Lomb CW79 or DELTACON (Deltafilcon A) or other object temporarily resident upon the surface of the eye. For example, U.S. Pat. No. 6,410,045 describes a contact lens-type drug delivery device comprising a polymeric hydrogel contact lens containing drug substance in a concentration of between 0.05% and 0.25% by weight absorbed in said contact lens which is capable of being delivered into the ocular fluid.

In other embodiments, supports such as a collagen corneal shield (e.g. BIO-COR dissolvable corneal shields, Summit Technology, Watertown, Mass.) can be employed. The compositions can also be administered by infusion into the eyeball, either through a cannula from an osmotic pump (ALZET®, Alza Corp., Palo Alto, Calif.) or by implantation of timed-release capsules (OCCUSENT®) or biodegradable disks (OCULEX®, OCUSERT®) which contain the compositions. These routes of administration have the advantage of providing a continuous supply of the composition to the eye.

Many types of drug delivery systems are known in the art and can be used for delivery of compositions of the present invention. Non-limiting examples have been set forth above, and more are listed below.

A preferred method to treat dry eye symptoms utilizes aqueous based solutions or gels, which may be formulated to contain one or more compounds of the present invention. The "active" ingredients in these artificial tear formulations are common water soluble or dispersable polymers such as hydroxyethylcellulose, hydroxypropylmethylcellulose, methylcellulose, carboxymethylcellulose, polyvinyl alcohol, polyvinyl pyrrolidone, polyethylene glycol, carbomers and poloxamers.

U.S. Patent 6,429,194 describes aqueous ophthalmic preparations for instillation into the eye, or in which to pre soak or store an object to be inserted into the eye, such as a contact lens, an ointment, or a solid device to be inserted into the conjunctival sac. The ophthalmic preparation includes a mucin component, similar to that found at the normal human ocular surface. U.S. patent No. 6,281,192 also describes the ophthalmic applications of mucin.

Several other types of delivery systems are available that are particularly suitable for delivering pharmaceutical compositions to the interior or posterior of the eye. For instance, U.S. Patent 6,154,671 to Parel et al. discloses a device for transferring a medicament into the eyeball by iontophoresis. The device utilizes a reservoir for holding the active agent, which contains at least one active surface electrode facing the eye tissue lying at the periphery of the cornea. The reservoir also has a return electrode in contact with the patient's partly closed eyelids. U.S. Patent 5,869,079 to Wong et al. discloses combinations of hydrophilic and hydrophobic entities in a biodegradable sustained release ocular implant. In addition, U.S. Patent 6,375,972 to Guo et al., U.S. Patent 5,902,598 to Chen et al., U.S. Patent 6,331,313 to Wong et al., U.S. Patent 5,707,643 to Ogura et al., U.S. Patent 5,466,233 to Weiner et al. and U.S. Patent 6,251,090 to Avery et al. each describes intraocular implant devices and systems that may be used to deliver pharmaceutical compositions comprising compounds of the present invention.

U.S. Pat. No. 4,014,335 describes an ocular drug delivery device placed in the cul-de-sac between the sclera and lower eyelid for administering the drug and acting as a reservoir. The device comprises a three-layered laminate of polymeric materials holding the drug in a central reservoir region of the laminate. The drug diffuses from the reservoir through at least one of the polymeric layers of the laminate.

Solid devices, in the form of ocular inserts, have been utilized for longer term symptomatic relief of dry eye. These devices are placed in the eye and slowly dissolve or erode to provide a thickened tear film. Examples of this technology are given in U.S. Pat. Nos. 5,518,732; 4,343,787, and 4,287,175.

For effective treatment of macular degeneration or any of the other retinopathies described herein, one skilled in the art may recommend a dosage schedule and dosage amount adequate for the subject being treated. The dosing may occur less frequently if the compositions are formulated in sustained delivery vehicles, or are delivered *via* implant. For topical delivery, it may be preferred that dosing occur one to four times daily for as long as needed. The dosage amount may be one or two drops per dose. The dosage schedule may also vary depending on the active drug concentration, which may depend on the hydroxylamine used and on the needs of the patient. It may be preferred that the active amount be from about 0.1% to about 10.0% weight by volume in the formulation. In some embodiments, it is preferable that the active drug concentration be 0.25% to about 10.0% weight by volume. The concentration of the hydroxylamine component will preferably be in the range of about 0.1 µM to about 10 mM in the tissues and fluids. In some embodiments, the range is from 1 µm to 5 mM, in other embodiments the range is about 10 µM to 2.5 mM. In other embodiments, the range is about 50 µM to 1 mM. Most preferably the range of hydroxylamine concentration will be from 1 to 100 µM. The concentration of the reducing agent will be from 1 µM to 5 mM in the tissues or fluids, preferably in the range of 10 µM to 2 mM. The concentrations of the components of the composition are adjusted appropriately to the route of administration, by typical pharmacokinetic and dilution calculations, to achieve such local concentrations. Alternatively, penetration of cornea and absorption into other tissues in the interior of the eye is demonstrated using radiolabeled hydroxylamine.

An ophthalmologist or one similarly skilled in the art will have a variety of means to monitor the effectiveness of the dosage scheme and adjust dosages accordingly. For example, effectiveness in the treatment of macular degeneration or other retinopathies may be determined by improvement of visual acuity and evaluation for abnormalities and grading of stereoscopic color fundus photographs. (Age-Related Eye Disease Study Research Group, NEI, NIH, AREDS Report No. 8, 2001, Arch. Ophthalmol. 119: 1417-1436). Following such evaluation, the ophthalmologist may adjust the frequency and/or concentration of the dose, if needed.

Any currently used or subsequently developed antioxidant may be used in the optional antioxidant component of the non-ophthalmic treatment regimen, as would be understood by one of skill in the art. Suitable antioxidants may include water-soluble or fat-soluble compounds. Water soluble antioxidants include, but are not limited to, vitamins C, polyphenols from various berries (cranberry, blueberry, bilberry and the like), proanthocyanidins and anthocyanins from grape seeds and bark of the European coastal pine and *Pinus maritime,* bioflavonoids (taxifolin, naringenin, hesperetin, 6-hydroxyflavanone, 2'- hydroxyflavanone, 4'- hydroxyflavanone) from fruits (especially citrus fruits) and vegetables, L-selenomethionine, alpha-Lipoic Acid, glutathione, catechin, epicatechin, epigallocatechin, epigallocatechin gallate, epicatechin gallate and cysteine. Fat soluble antioxidants include, but are not limited to, vitamin E (alpha-tocopherol acetate), gamma-tocopherol, alpha-carotene, beta-carotene (vitamin A), lutein, zeaxanthin, retinal, astaxanthin, cryptoxanthin, natural mixed carotenoids, lycopene and resveratrol, to name a few. In some embodiments, a formulation may include a combination of all of these antioxidants. In an exemplary embodiment, a formulation includes vitamin A, vitamin C and vitamin E.

Suitable dosage ranges for any of the foregoing antioxidants are well known to the skilled artisan. For the exemplary formulation, vitamin A may be supplied from beta-carotene in a daily dosage of the latter of at least about 6 mg, more specifically at least about 9 mg, even more specifically at least about 12 mg, and even more specifically at least about 15 mg. Vitamin C may be supplied in a daily dosage of at least about 200 mg, more specifically at least about 300 mg, even more specifically at least about 400 mg, yet more specifically at least about 500 mg. Vitamin E may be supplied in a daily dosage of at least about 200 IU, more specifically at least about 300 IU, even more specifically at least about 400 IU. In a particular embodiment, the optional antioxidants are combined into an orally deliverable dosage unit. In another particular embodiment, zinc, copper and the optional antioxidants are combined into an orally deliverable dosage unit.

An ophthalmologist or one similarly skilled in the art will have a variety of means to monitor the effectiveness of the combination dosage scheme and adjust dosages accordingly. For example, as mentioned above effectiveness in the treatment of macular degeneration or other retinopathies may be determined by improvement of visual acuity and evaluation for abnormalities and grading of stereoscopic color fundus photographs. (AREDS Report No. 8, *2001, supra*). Following such evaluation, the ophthalmologist may adjust the frequency and/or concentration of either the ophthalmic or the non-opththalmic dose, if needed.

Pharmaceutical kits useful in, for example, the treatment of pain, which comprise a therapeutically sufficient amount of a chemotherapeutic agents or anti-angiogenic agent along with a therapeutically sufficient amount of a hydroxylamine of the invention, in one or more sterile containers, are also within the ambit of the present invention. Sterilization of the container may be carried out using conventional sterilization methodology well known to those skilled in the art. The sterile containers of materials may comprise separate containers, or one or more multi-part containers, as exemplified by the UNIVIAL™ two-part container (available from Abbott Labs, Chicago, Illinois), as desired. The opioid or cannabinoid compound and the compound of Formula I or II may be separate, or combined into a single dosage form as described above. Such kits may further include, if desired, one or more of various conventional pharmaceutical kit components, such as for example, one or more pharmaceutically acceptable carriers, additional vials for mixing the components, etc., as will be readily apparent to those skilled in the art. Instructions, either as inserts or as labels, indicating quantities of the components to be administered, guidelines for administration, and/or guidelines for mixing the components, may also be included in the kit.

### EXPERIMENTAL SECTION

### PREPARATION OF COMPOUNDS

### General procedure A: General procedure for the synthesis of 3-hydroxy-4-alkyl-1,2,5-thiadiazole or 3-chloro-4-alkyl-1,2,5-thiadiazole or 3-Chloro-4-aryl-1,2,5-thiadiazole

Amino amide hydrochloride (20mmol) was dissolved in 20ml of DMF. Sulfur monochloride (7.6g, 56mmol) was added at 0-5 °C in 20 min. the reaction was stirred at room temperature for 6 hrs. Yellow sulfur precipitate was filtered after quenching the reaction with ice water. The aqueous was extracted with CH₂Cl₂ (3x20ml). The combined organic was dried over MgSO4. The solvent was removed and the residue was purified on silica gel column using CH₂Cl₂( 1L) as an eleunt. A pale yellow crystalline solid, 4-(2-methyl-alkyl)-3-hydroxy-1,2,5-thiadiazole was obtained.

When an amino-nitrile was used instead of amino-amide, 3-chloro-4-alkyl-1,2,5-thiadiazole or 3-chloro-4-aryl-1,2,5-thiadiazole was obtained. The chemical structure was confirmed by ¹H NMR.

### General procedure B: General Procedure for the synthesis of 4-(4-cyclic-amino-1-yl)-3-chloro-1,2,5-thiadiazole

3,4-Dichloro-1,2,5-thiadiazole (4.65g, 30mmol) was added over a 30min period at 105-110°C to 120mmol of cyclic amine. After addition, the reaction mixture was stirred for 2hr at 105-110°C (monitored by TLC, Hex/EtOAc 1/3). The mixture was cooled to room temperature , aqueous ammonium (20 mL) was added and the mixture was extracted with CH₂Cl₂ (5x20 mL). The combined organic phase was washed with ammonia (10 mL), water (2x10 mL) and dried over MgSO₄. The solvent was removed and the residue was purified (silica gel, EtOAC). 5.9g of 4-(4-cyclic-amino-1-yl)-3-chloro-1,2,5-thiadiazole was obtained. The yield was 90.2%.

### Example 1

### General Procedure for the preparation of nitroxides:

To a solution of t-BuOK in t-BuOH (30mL), TEMPOL was added in one portion, and followed by thiadiazole. The reaction mixture was stirred at room temperature overnight. Water (50mL) was then added into the reaction mixture. The mixture was extracted with EtOAc (3×50mL). The organic phase was washed with brine (20mL) and dried over MgSO₄. The solvent was removed in vacuum. The crude product was purified by silica gel column (EtOAc/Hexane = 1/10). After removal of the solvent *in vacuo,* product (3.0g) was obtained.

### Example 2

### General Procedure for the preparation of hydroxylamine HCl salts:

To a solution of the nitroxide compound (∼1g, 5.4mmol) in 2-propanol (∼10mL)) was added a saturated solution containing hydrogen chloride in 2-propanol (∼20mL) in one portion, and the reaction mixture was stirred at room temperature (1h to 20h) or heated to reflux (∼2h) until it became colorless. The solvent was removed in vacuum to give an off-white solid. The crude product was recrystallized from 2-propanol. White solid (∼0.72g, 3.2mmol) was obtained. Product was identified by ¹H NMR analysis, elemental analysis, IR and mp.

### Example 3 - Experimental Data for Hydroxylamine Preparation

### Preparation of Compound 1: (1-hydroxy-4-methoxy-2,2,6,6-tetramethylpiperidine hydrogen chloride)

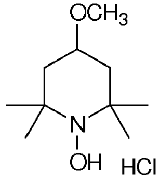

To a solution of 4-methoxy-2,2,6,6-tetramethylpiperidine-1-oxyl (1g, 5.4 mmol) in 2-propanol (10mL) was added a saturated solution containing hydrogen chloride in 2-propanol (20ml) in one portion, and the reaction mixture was stirred at room temperature until it became colorless. The solvent was removed in vacuum to give an off-white solid. The crude product was recrystallized from 2-propanol to give a white solid (0.72g). Yield is 59%. ¹H NMR (300MHz, MeOD), δ3.79(m, 1H), 3.35(s, 3H), 2.34(d, 2H), 1.75(t, 2H), 1.49(s, 6H), 1.47(s, 6H). ¹³C NMR (75MHz, MeOD), δ68.93, 68.54, 55.07, 41.43, 27.40, 19.29. M.P.: 198.5°C (dec)., Elemental analysis: Calcd. (C₁₀H₂₁NO₂.HCl) C 53.68%, H 9.91%, N 6.26% (Found C 53.74%, H 9.94%, N 6.18%).

### Preparation of Compound 2: (1-hydroxy-4-acetamido-2,2,6,6-tetramethylpiperidine)

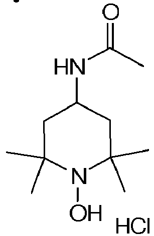

Compound 2 was prepared according to the preparation described in M. C. Krishna et al, J. Med. Chem., 41(18), 3477-3492(1998). Mp. 180.5°C (dec). ¹H NMR (300MHz, MeOD), δ4.30(m, 1H), 2.14(d, 2H), 1.99(s, 3H), 1.94(t, 2H), 1.50(s, 12H). ¹³C NMR (75MHz, MeOD), δ171.87, 68.42, 41.29, 39.42, 26.81, 20.95, 18.94. Elemental analysis: Calcd. (C₁₁H₂₂N₂O₂.2HCl.2.5H₂O) C 39.76%, H 8.80%, N 8.43% (Found C 39.99%, H8.64%, N 8.46%).

### Preparation of Compound 3: (4-(2,2,6,6-tetramethylpiperidin-1-hydroxy-4-yl)morpholine hydrogen chloride)

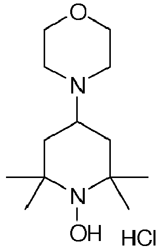

To a solution of 4-(2,2,6,6-tetramethylpiperidin-1-oxyl-4-yl)morpholine (0.3g, 1.2 mmol) in 2-propanol (25mL) was added a saturated solution containing hydrogen chloride in 2-propanol (5mL) in one portion. The solution was heated to reflux until it became colorless. The solvent was concentrated in vacuum to about 2mL of liquid remaining. The solid was collected by filtration, and dried in vacuum. White solid (0.24g) was obtained. Yield is 72%. ¹H NMR (300MHz, D₂O), δ4.06 (m, 5H), 3.51 (m, 4H), 2.62 (m, 2H), 2.17 (m, 2H), 1.58 (s, 6H), 1.56 (s, 6H). ¹³C NMR (75MHz, D₂O DEPT), δ 63.96, 56.25, 49.38, 36.25, 27.23, 19.13. Mp. 229.2°C (dec).

### Preparation of Compound 4: (4-(4-(2,2,6,6-tetramethylpiperidin-1-hydroxyl-4-yloxy)-1,2,5-thiazol-3-yl)morpholine hydrogen chloride)

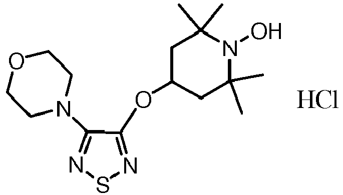

### Step a.

### Preparation of 4-(4-(2,2,6,6-tetramethylpiperidin-1-oxyl-4-yloxy)-1,2,5-thiazol-3-yl)morpholine

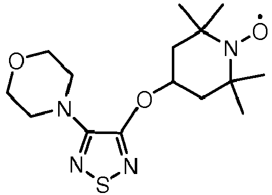

To a solution of t-BuOK(3.37g) in t-BuOH (30mL), tempol (4.31g) was added in one portion, and followed by addition of thiadiazole (4.11g). The reaction mixture was stirred at room temperature overnight. Water (50mL) was added into the reaction mixture. The mixture was extracted with EtOAC (3×50mL). The organic phase was washed with brine (20mL) and dried over MgSO₄. The solvent was removed in vacuum and the crude product was purified by silica gel column (EtOAc/Hexane = 1/10). After removal of the solvent in vacuum, product (3.0g) was obtained.

### Step b:

To a solution of the 4-(4-(2,2,6,6-tetramethylpiperidin-1-oxyl-4-yloxy)-1,2,5-thiazol-3-yl)morpholine (2g, 5.9 mmol) in 2-propanol (20mL) was added a saturated solution containing hydrogen chloride in 2-propanol (20ml) in one portion, and the reaction mixture was stirred at room temperature until it became colorless. The solvent was removed in vacuum to give an off-white solid. The crude product was recrystallized from 2-propanol to give a white solid (1.92 g) was obtained. The yield is 88%. ¹H NMR (300MHz, D₂O), δ 5.38 (m, 1H), 3.80 (m, 4H), 3.42 (m, 4H), 2.59 (d, 2H, J=12.9), 1.99 (t, 2H, J=12.3), 1.49 (s, 6H), 1.45 (s, 6H). ¹³C NMR (75MHz, D₂O ), δ 152.70, 150.99, 68.53, 70.32, 66.12, 66.07, 47.90, 47.85, 40.97, 27.38, 19.63. Mp. 203.3°C (dec). Elemental analysis: Calcd. (C₁₅H₂₆N₄O₃S.HCl) C 47.55%, H 7.18%, N 14.79% (Found C 47.56%, H 7.38%, N 14.52%).

### Preparation of Compound 5: (1-Hydroxy-2,2,6,6-tetramethyl-piperidin-4-one)

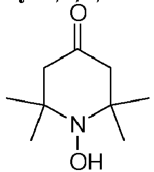

Compound 5 was prepared according to the preparation described in G. Sosnovsky et al, J. Org. Chem.,60(11), 3414-3418(1995). ¹H NMR (300MHz, CDCl₃), δ 12.30 (s, 1H), 11.31 (s, 1H), 3.67 (d, 2H, J=13.7), 2.47 (d, 2H, J=13.8) 1.80 (s, 6H), 1.42 (s, 6H). ¹³C NMR (75MHz, MeOD-D₄), δ 201.71, 71.01, 51.19, 27.78, 22.06. mp. 166.0°C (dec) Elemental analysis: Calcd. (C₉H₁₇NO₂.HCl) C 52.04%, H 8.74%, N 6.74% (Found C 55.32%, H9.44%, N 7.18%).

### Preparation of Compound 6: (2,2,3,5,6,6-Hexamethyl-piperidine-1,4-diol hydrogen chloride salt)

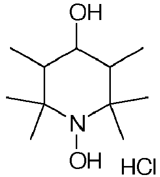

To a solution of 2,2,3,5,6,6-hexamethyl-4-hydroxypiperidin-1-oxyl (0.17g, 0.85 mmol) in isopropanol (10 mL) was added 5 mL of saturated HCl-isopropanol solution. The resulting solution was refluxed until it became colorless. After the solution was cooled to room temperature, a white solid (0.15g) precipitated and was collected via filtration. Yield was 68%. ¹H NMR (300MHz, MeOD-D₄), δ 4.04 (m, 1H), 2.17 (m, 1H), 1.93 (m, 1H), 1.40 (s, 3H), 1.38 (s, 3H), 1.32 (s, 3H), 1.26 (s, 3H), 0.99 (d, 3H, J=7.2), 0.98 (d, 3H, J=6). ¹³C NMR (75MHz, MeOD-D₄), δ 66.77, 43.15, 39.66, 25.27, 21.26, 15.41, 11.88, 6.61. mp. 191.5°C (dec) Elemental analysis: Calcd. (C₁₁H₂₃NO₂.HCl) C 55.57%, H 10.17%, N5.89% (Found C 55.63%, H10.26%,N5.85%).

### Preparation of Compound 7: (2,2,5,5-Tetramethyl-pyrrolidine-1,3-diol)

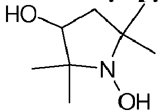

Compound 7 was prepared according to the preparation described in A. D. Malievskii, et al, Russ. Chem. B1.,47(7),1287-1291(1998). ¹H NMR (300MHz, DMSO), δ 11.32 (s, 1H), 11.02 (s, 1H), 5.69 (s, 1H), 3.98 (m, 2H), 2.33 (m, 2H), 1.80 (m, 2H), 1.44 (s, 3H), 1.31 (s, 3H), 1.25 (s, 3H), 1.18 (s, 3H). mp. 155.5°C (dec). Elemental analysis: Calcd. (C₈H₁₇NO₂.HCl.0.08H₂O) C48.74%, H 9.29%, N7.11% (Found C 48.59%, H9.18%, N 7.44%).

### Preparation of Compound 8: (2,5-dihydro-2,2,5,5-tetramethyl-1-hydroxyl-1H-pyrrol-3-yl)methanol hydrogen chloride)

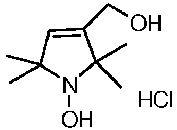

To a solution of 2,5-dihydro-2,2,5,5-tetramethyl-1-oxyl-1H-pyrrol-3-yl)methanol (0.3g, 1.8 mmol) in 2-propanol (10mL) was added a saturated solution containing hydrogen chloride in 2-propanol (10ml) in one portion, and the reaction mixture was stirred at room temperature for 1h. Then the mixture was heated to 50 °C for another 0.5h. The mixture was turned to light color and allowed to cool off to room temperature. The solvent was removed in vacuum to give an off-white solid. The crude product was recrystallized from 2-propanol (2mL) to give a white solid (0.21g). The yield is 56%. ¹H NMR (300MHz, MeOD-D₄), δ 5.83 (m, 2H), 4.17 (s, 2H), 1.60 (s, 3H), 1.59 (s, 3H), 1.55 (s, 3H), 1.50 (s, 3H). ¹³C NMR (300MHz, MeOD-D₄ ), δ 145.35, 127.89, 79.49, 77.39, 58.58, 25.46, 24.14, 23.06, 22.85. mp. 141.9°C (dec). Elemental analysis: Calcd. (C₉H₁₈ClNO₂.HCl.0.2C₃H₈O) C52.48%, H 8.99%, N6.37% (Found C 52.64%, H8.92%, N 6.62%).

### Preparation of Compound 9: (3-Bromo-2,2,6,6-tetramethyl-piperidine-1,4-diol)

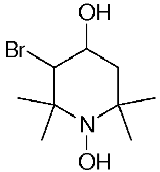

Compound 9 was prepared according to the preparation described in L. A. Krinitskaya et al, Bull.Acad.Sci.USSR Div.Chem.Sci.(Eng.),36(7), 1461-1466(1987). ¹H NMR (300MHz, DMSO), δ 12.53 (br. 1H), 11.86 (br. 1H), 4.70 (s, 1H), 4.10 (m, 1H), 2.09-2.43 (m, 2H), 1.40-1.57(m, 12H). mp. 138.5°C (dec). Elemental analysis: Calcd. (C₉H₁₉BrClNO₂) C37.45%, H 6.64%, N4.85% (Found C 37.75%, H6.89%, N 5.00%),

### Preparation of Compound 10: (1-hydroxy-4-methanesulfonamido-2,2,6,6-tetramethylpiperidine)

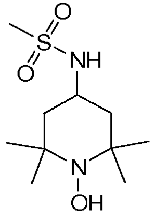

Compound 10 was prepared according to the preparation described in M. C. Krishna et al, J. Med. Chem., 41(18), 3477-3492(1998). ¹H NMR (300MHz, MeOD-D₄), δ 3.81 (m, 1H), 2.98 (s, 3H), 2.22 (m, 2H), 1.94 (m, 2H), 1.47 (s, 12H). ¹³C NMR (300MHz, MeOD-D₄ ), 69.98, 44.78, 44.73, 41.83, 28.41, 20.46. mp. 178.3°C (dec). Elemental analysis: Calcd. (C₁₀H₂₂N₂O₃S.HCl.H₂O) C39.40%, H 8.27%, N9.19% (Found C 39.70%, H8.51%, N 9.19%).

### Preparation of Compound 11: (N-(2,2,6,6-tetramethylpiperidin-1-hydroxyl-4-yl)morpholine-4-carboxamide hydrogen chloride)

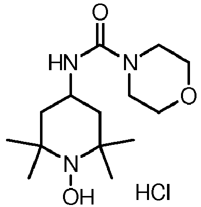

To a solution of N-(2,2,6,6-tetramethylpiperidin-1-oxyl-4-yl)morpholine-4 carboxamide (0.4g, 1.4 mmol) in 2-propanol (10mL) was added a saturated solution containing hydrogen chloride in 2-propanol (5ml) in one portion, and the reaction mixture was stirred at room temperature for 1h. The solvent was removed in vacuum to give a foamlike product (0.23g).The yield is 51%. ¹H NMR (300MHz, MeOD-D₄), δ 4.18 (t, 1H, J=12.3), 2.10 (m, 2H), 1.90 (m, 2H), 1.46 (s, 6H), 1.45 (s, 6H). ¹³C NMR (75MHz, MeOD-D₄ ), δ 158.04, 68.70, 66.19, 43.90, 42.32,40.47, 27.01, 18.98. mp. 158.1°C (dec). Elemental analysis: Calcd. (C₁₄H_{27.}N₃O₃.HCl.0.95H₂O) C49.61%, H 8.89%, N12.40% (Found C 49.69%, H9.21%, N 12.16%).

### Preparation of Compound 12: (4-cyano-1-hydroxyl-2,2,6,6-tetramethylpiperidine hydrogen chloride)

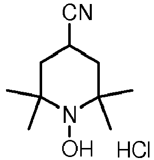

To a solution of 4-cyano-2,2,6,6-tetramethylpiperidin-1-oxyl (0.5g, 2.8 mmol) in 2-propanol (10mL) was added a saturated solution containing hydrogen chloride in 2-propanol (5ml) in one portion, and the reaction mixture was stirred at room temperature for 1h. The solvent was concentrated in vacuum to about 2mL of liquid remaining. The solid was collected by filtration, and dried in vacuum to give a white solid (0.43g). The yield is 71%. ¹H NMR (300MHz, MeOD-D₄), δ 3.484 (t,t 1H, J=12.8, 3.5), 2.38 (d, 2H, J=13.8), 2.32 (t, 2H, J=13.7), 1.54 (s, 6H), 1.47 (s, 6H). ¹³C NMR (75MHz, MeOD-D₄ ), δ 119.83, 67.67, 38.71, 26.38, 19.55, 18.03. mp. 186.0°C (dec). Elemental analysis: Calcd. (C₁₀H₁₉ClN₂O) C54.91%, H 8.76%, N12.81% (Found C 54.94%, H8.64%, N 12.73%).

### Preparation of Compound 13 & Compound 14 Nitroxide precursors

To a solution of t-BuOK (3.4g) in t-BuOH (30mL), TEMPOL (3.45g) was added in one portion. The reaction mixture was allowed to stir for 0.5h, followed by addition of 3,4-dichloro-1,2,5-thiazole (3.1g) and then. stirred at room temperature overnight. TLC (EtOAc/Hexane =1:2) showed 3,4-dichloro-1,2,5-thiazole at Rf 0.8; compound 13 precursor nitroxide at Rf 0.5; compound 14 precursor nitroxide at Rf 0.4. After removal of solvent in vacuum, EtOAc (100mL) was added to the residue. The organic phase was washed with water (2x30mL) and dried over MgSO₄. Upon removal of the solvent in vacuum, brown liquid was obtained. The crude product (0.4g) was applied on preparative TLC (EtOAc/Hexane = 1/10). The OT-314[O] and OT-314[O] spots were collected. OT-314[O] (0.18g) and OT-314[O] (0.06g) were obtained.

### Preparation of Compound 13: (4-(4-chloro-1,2,5-thiadiazol-3-yloxyl)-1-hydroxyl-2,2,6,6-tetramethylpiperidine hydrogen chloride)

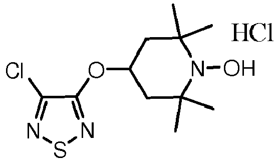

To a solution of 4-(4-chloro-1,2,5-thiadiazol-3-yloxyl)-2,2,6,6-tetramethylpiperidin-1-oxyl (0.18g, 0.71 mmol) in 2-propanol was added a saturated solution containing hydrogen chloride in 2-propanol (5ml) in one portion. The mixture was then heated to 40°C for another 1h and allowed to cool off to room temperature. The solvent was removed in vacuum to give white solid (0.2g). The yield is 97%. ¹H NMR (300MHz, CDCl₃), δ 11.9 (d, 1H, J=5.5), 11.8 (d, 1H, J=5.5) 5.35 (t,t 1H, J=11.6, 4.4), 2.73 (t, 2H, J=12.6), 2.30 (dd, 2H, J=13.7, 4.0), 1.75 (s, 3H), 1.61 (s, 6H), 1.49 (3, 3H). mp. 161.5°C (dec). Elemental analysis: Calcd. (C₁₁H₁₇N₃O₂.1.5HCl) C38.13%, H 5.67%, N12.13% (Found C 38.15%, H5.50%, N 11.84%).

### Preparation of Compound 14: (1-hydroxyl-4-(4-(2,2,6,6-tetramethylpiperidin-1-hydroxyl-4-yloxy)-1,2,5-thiadiazol-3-yloxy)-2,2,6,6-tetramethylpiperidine bis-hydrogen chloride)

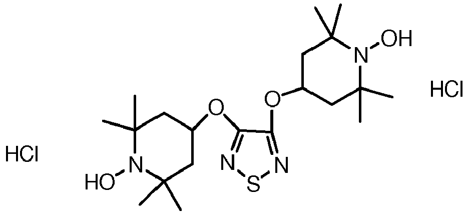

To a solution of 4-(4-(2,2,6,6-tetramethylpiperidin-1-oxyl-4-yloxy)-1,2,5-thiadiazol-3-yloxy)-2,2,6,6-tetramethylpiperidin-1-oxyl (0.06g, 0.14 mmol) in 2-propanol was added a saturated solution containing hydrogen chloride in 2-propanol (5ml) in one portion. The reaction mixture was heated to 40°C for another 1h and then allowed to cool off to room temperature. The solvent was removed in vacuum to give a white solid (0.06g). The yield is 86%. ¹H NMR (300MHz, MeOD-D₄), δ 11.9 (d, 2H, J=5.5), 11.8 (d, 2H, J=-5.5), 5.44 (m, 2H), 2.65 (d, 4H, J=15.8), 2.14 (t, 4H, J=13.5), 1.60 (s, 12H), 1.57 (s, 12H). ¹³C NMR (75MHz, MeOD-D₄), δ 132.98, 70.68, 68.66, 40.67, 27.01, 19.18. mp. 216.2°C (dec). Elemental analysis: Calcd. (C₂₀H₃₈Cl₂N₄O₄S.0.5HCl) C46.22%, H 7.47%, N10.78% (Found C 46.40%, H7.69%, N 10.58%).

### Preparation of Compound 15: (1,1,3,3-tetramethylisoindolin-2-hydroxyl-5-carboxylic acid hydrogen chloride)

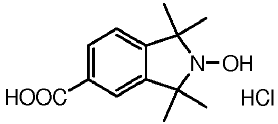

To a solution of 1,1,3,3-tetramethylisoindolin-2-oxyl-5-carboxylic acid (0.2g, 0.85 mmol) in 2-propanol (10mL) was added a saturated solution containing hydrogen chloride in 2-propanol (5ml) in one portion. The reaction mixture was heated to 40°C for another 1h and then allowed to cool off to room temperature. The solvent was removed in vacuum to give a white solid (0.07g). The yield is 31%. ¹H NMR (300MHz, MeOD-D₄), δ 12.88 (br, 1H), 7.85 (d, 2H, J=7.5), 7.66 (d, 1H, J=), 7.34 (d, 1H, J=6.6), 1.33 (s, 12H). ¹³C NMR (75MHz, MeOD-D₄), δ 132.98, 70.68, 68.66, 40.67, 27.01, 19.18. mp. 225.5°C (dec). Elemental analysis: Calcd. (C₁₃H₁₇NO₃.0.15HCl) C64.86%, H 7.19%, N5.82% (Found C 64.78%, H7.54%, N 5.55%).

### Compound 17: (α-phenyl-t-butyl nitrone) ("PBN")

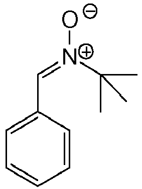

### Preparation of Compound 20: (3,3,5,5-1-hydroxy-tetramethylmorpholine hydrogen chloride)

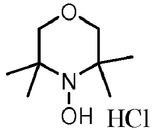

To a solution of 3,3,5,5-tetramethylmorpholin-1-oxy (0.5g, 3.2 mmol) in 2-propanol (10mL) was added a saturated solution containing hydrogen chloride in 2-propanol (5ml) in one portion. The reaction mixture was heated to 40°C for another 2h and then allowed to cool off to room temperature. The solvent was removed in vacuum to give a white solid (0.41g). The yield is 66%. ¹H NMR (300MHz, MeOD-D₄), δ 11.84 (br, 1H,), 11.00 (br, 1H, ), 4.14 (d, 2H, J=12.5), 3.69 (d, 2H, J=12.4), 1.58 (s, 6H), 1.50 (s, 6H). ¹³C NMR (75MHz, MeOD-D₄), δ 74.34, 67.33, 22.43, 19.74. mp. 184.6°C (dec). Elemental analysis: Calcd. (C₈H₁₇NO₂.HCl) C49.10%, H 9.27%, N7.16% (Found C 48.93%, H9.35%, N 7.17%).

### Preparation of Compound 21: (4-chloro-1-hydroxy-2,2,6,6-tetramethylpiperidine hydrogen chloride)

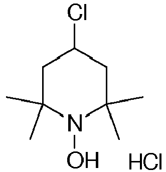

To a solution of 4-chloro-2,2,6,6-tetramethylpiperidin-1-oxy (0.4g, 2.1 mmol) in 2-propanol (15mL) was added a saturated solution containing hydrogen chloride in 2-propanol (5mL) in one portion, and the reaction mixture was stirred at 40°C for half an hour until it became colorless. The solvent was removed in vacuum to give an off-white solid. The crude product was recrystallized from 2-propanol. White solid (0.39g) was obtained. The yield was 81%. ¹H NMR (300MHz, DMSO), δ 4.76 (m, 1H,), 2.29 (m, 4H,), 1.50 (s, 6H), 1.36 (s, 6H). ¹³C NMR (75MHz, DMSO), δ 69.22, 67.52, 42.21, 27.67, 20.67. mp. 201.8°C (dec). Elemental analysis: Calcd. (C₉H₁₉Cl₂NO) C47.38%, H 8.39%, N6.14% (Found C 47.34%, H8.49%, N 5.96%).

### Preparation of Compound 23: (2,5-dihydro-2,2,5,5-tetramethyl-1H-pyrrole-3-carboxamide-1-hydroxy)

To a solution of 2,5-dihydro-2,2,5,5-tetramethyl-1H-pyrrole-3-carboxamide-1-oxy (0.5g, 2.7 mmol) in 2-propanol (10mL) was added a saturated solution containing hydrogen chloride in 2-propanol (5mL) in one portion. The solution was kept stirring at 40°C until it became colorless. The solvent was concentrated in vacuum to about 2mL of liquid remaining. The solid was collected by filtration, and dried in air with vacuum to give a white solid (0. 42g). The yield was 70%. ¹H NMR (300MHz, D₂O), δ 6.51 (s, 1H,), 1.56 (s, 6H), 1.48 (s, 6H). ¹³C NMR (75MHz, D₂O), δ 166.98, 136.50, 136.44, 78.43, 75.98, 22.52.

### Preparation of Compound 24: 1-hydroxy-2,2,5,5-tetramethylpyrrolidine-3-carboxamide)

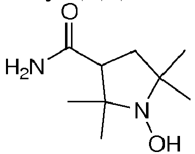

To a solution of 2,2,5,5-tetramethylpyrrolidine-3-carboxamide-1-oxy(0.45 g, 2.4 mmol) in 2-propanol (10mL) was added a saturated solution containing hydrogen chloride in 2-propanol (5mL) in one portion, and the reaction mixture was stirred at 40° C until it became colorless. The solvent was removed in vacuum to give an off-white solid. The crude product was recrystallized from 2-propanol. 0.38 g of white solid was obtained. The yield was 71%. ¹H NMR (300 MHz, DMSO), δ 3.08(t, 1H, J=8.4), 2.24 (d, 2H, J=8.4), 1.47 (s, 3H), 1.45 (s, 3H), 1.39 (s, 3H), 1.31 (s, 3H). ¹³C NMR (75MHz, DMSO), δ 74.14, 71.89, 49.08, 37.36, 24.73, 23.12.

### Preparation of Compound 25: (1-hydroxy-1,2,3,6-tetrahydro-2,2,6,6-tetramethylpyridin-4-yl)methanol hydrogen chloride)

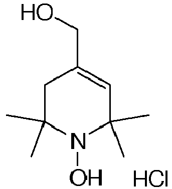

To a solution of (1,2,3,6-tetrahydro-2,2,6,6-tetramethylpyridin-1-oxy-4-yl)methanol (0.4g, 2.2 mmol) in 2-propanol (10mL) was added a saturated solution containing hydrogen chloride in 2-propanol (10mL) in one portion, and the reaction mixture was stirred at room temperature for 1h. Then the mixture was heated to 40 °C for another 0.5h. The mixture was turned to light color and allowed to cool off to room temperature. The solvent was removed in vacuum to give an off-white solid. The crude product was recrystallized from 2-propanol. White solid (0.31g) was obtained. The yield was 64%. ¹H NMR (300MHz, DMSO), δ 12.45 (br, 1H), 11.40 (br, 1H), 5.56 (s, 1H), 3.83 (s, 3H, OH), 2.70 (d, 1H, J=16.8), 2.15 (d, 1H, J=16.8), 1.56 (s, 3H), 1.50 (s, 3H), 1.38 (s, 3H), 1.28 (s, 3H). ¹³C NMR (75MHz, DMSO), δ 123.60, 121.42, 66.95, 65.24, 63.51, 36.71, 27.01, 25.78, 22.63, 21.25. mp. 170.1°C (dec).

### Preparation of Compound 26: (N-(1-hydroxy-2,2,6,6-tetramethylpiperidin-4-yl)-3-morpholinopropanamide hydrogen chloride)

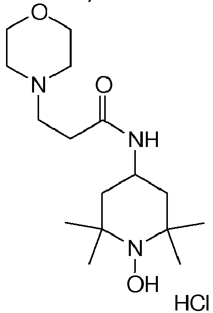

To a solution of N-(2,2,6,6-tetramethylpiperidin-1-oxy-4-yl)-3-morpholinopropanamide (0.4g, 1.3 mmol) in 2-propanol (15mL) was added a saturated solution containing hydrogen chloride in 2-propanol (15mL) in one portion, and the reaction mixture was stirred at 40 °C for half an hour until it turned into colorless. The solvent was removed in vacuum to give an off-white solid. The crude product was recrystallized from 2-propanol. White solid (0.42g) was obtained. The yield was 92%.

### Preparation of Compound 27: (4,5-dihydroxy-2-methyl-N-(I-hydroxy-2,2,6,6-tetramethylpiperidin-4-yl)benzamide hydrogen chloride)

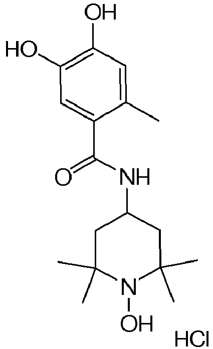

### Step a: Preparation of 4,5-bis(benzyloxy)-2-methyl-N-(2,2,6,6-tetramethylpiperidin-1-oxy-4-yl)benzamide:

To a solution of 4,5-bis(benzyloxy)-2-methylbenzoyl chloride (3.67g, 10 mmol) in toluene (100 mL) at 0-5 °C, 4-amino-TMPO (2,2,6,6-tetramethylpiperidin-4-amine-1-oxy) (1.71g, 10 mmol) in Et₃N (50 mL) was added dropwise. The reaction mixture was then stirred overnight at room temperature. After filtration, the organic phase was washed with 1M hydrogen chloride (50 mL), water (50ml) and dried over MgSO₄. The solvent was removed in vacuum. The crude solid was purified by column ( silica gel, EtOAc/Hexane 1:1) to give a yellow solid (4.2g) of 4,5-bis(benzyloxy)-2-methyl-N-(2,2,6,6-tetramethylpiperidin-1-oxy-4-yl)benzamide. The yield was 84%.

### Step b: Synthesis of 4,5-dihydroxy-2-methyl-N-(2,2,6,6-tetramethylpiperidin-1-oxy-4-yl)benzamide

To the mixture of 10% Pd/C (0.2g) in i-PrOH (5 mL), 4,5-bis(benzyloxy)-2-methyl-N-(2,2,6,6-tetramethylpiperidin-1-oxy-4-yl)benzamide (4.2g, 8.4mmol) in MeOH (25 mL) was added in one portion and the reaction mixture was stirred under H₂ atmosphere overnight. After the solid was filtered off, the filtrate was concentrated in vacuum to give a solid. It was purified by column chromatography (silica gel, methanol) to give a yellow solid (2.3g). The yield was 86%.

### Step c:

To a solution of 4,5-dihydroxy-2-methyl-N-(2,2,6,6-tetramethylpiperidin-1-oxyl-4-yl)benzamide (2.3g, 7.2 mmol) in 2-propanol (50mL) was added a saturated solution containing hydrogen chloride in 2-propanol (25mL) in one portion, and the reaction mixture was stirred at room temperature for 1h. The solvent was concentrated in vacuum to about 2mL of liquid remaining. The solid was collected by filtration, and dried in vacuum. White solid (1.83g) was obtained. The yield was71%.

### Preparation of Compound 29: (N-(3,5-di-tert-butyl-4-hydroxyphenyl)-1-hydroxy-2,2,6,6-tetramethylpiperidine-4-carboxamide hydrogen chloride)

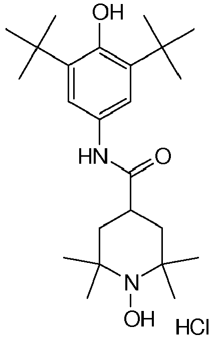

### Step a: Preparation of (3,5-di-tert-butyl-4-hydroxy-N-(2,2,6,6-tetramethylpiperidin-l-oxy-4-yl)benzamide)

To a solution of 3,5-t-butyl-4-hydroxyl benzoic acid (2.50g, 10 mmol), 4-amino-TEMPO (1.55g, 9.1mmol) and DMAP(0.5g, 4mmol) in CH₂Cl₂ (50 mL) At 0-5 °C, DCC(2.30g, 11mmol) in dichloromethane(50mL) was added dropwise. After addition is complete, the mixture was stirred at room temperature overnight. The reaction mixture was filtered and the filtrate was washed with 1N HCl (20mL) and dried over MgS04. After MgS04 was filtered off, the solvent was removed in vacuum to give a solid The solid was purified by column chromatography (silica gel, EtOAc/Hexane). The product is an orange solid (1.3g). The yield was 35%.

### Step b:

To a solution of N-(3,5-di-tert-butyl-4-hydroxyphenyl)-2,2,6,6-tetramethylpiperidin-1-oxyl-4-carboxamide (1.3g, 3.2 mmol) in 2-propanol (50mL) was added a saturated solution containing hydrogen chloride in 2-propanol (25mL) in one portion, and the reaction mixture was stirred at room temperature for 1h. The solvent was concentrated in vacuum to about 2mL of liquid remaining. The solid was collected by filtration, and dried in vacuum. White solid (0.8g) was obtained. The yield was 56%.

### Preparation of Compound 30 : (1-hydroxy-2,2,6,6-tetramethyl-4-(2H-tetrazol-5-yl)piperidine hydrogen chloride)

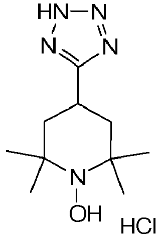

### Step a: Preparation of (2,2,6,6-tetramethyl-4-(2H-tetrazol-5-yl)piperidin-1-oxy)

To the solution of 4-cyano-TMPO (4-cyano-2,2,6,6-tetramethylpiperidin-1-oxy) (1.9g, 10.5 mmol) in 1,4-dioxane(100 mL), Bu₃SnN₃ (3.0 mL)was added in one portion. The mixture was heated at 100° C with stirring overnight. After the solvent was removed in vacuum, 300 mL of ethyl acetate was added to the residue and 20 mL of 2.0 M HCl/ether was added dropwise during a period of 30 minutes. After the addition was complete, the mixture was stirred for an hour. The precipitate was collected via filtration followed with purification by column chromatography (silica gel, CH₂Cl₂/MeOH, 4:1). An orange solid (0.72 g) was obtained. The yield was 30%.

### Step b:

To a solution of 2,2,6,6-tetramethyl-4-(2H-tetrazol-5-yl)piperidin-loxyl (0.72g, 3.2 mmol) in 2-propanol (10mL) was added a saturated solution containing hydrogen chloride in 2-propanol (5mL) in one portion. The reaction mixture was heated to 40°C for another 1h and then allowed to cool off to room temperature. The solvent was removed in vacuum and white solid (0.5g) was obtained. The yield was 59%.

### Preparation of Compound 31: (N-(1-hydroxy-2,2,6,6-tetramethylpiperidin-4-yl)cyclopropanecarboxamide hydrogen chloride)

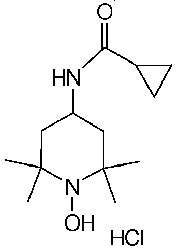

To the solution of N-(2,2,6,6-tetramethylpiperidin-1-oxyl-4-yl)cyclopropanecarboxamide (0.8g, 3.4 mmol) in 2-propanol (10mL) was added a saturated solution containing hydrogen chloride in 2-propanol (5mL) in one portion. The reaction mixture was heated to 40°C for 0.5 h and then allowed to cool off to room temperature. The solvent was removed in vacuum and 5 mL of diisopropyl ether was added. White solid (0.67g) was obtained. The yield was 71%.

### Preparation of Compound 32: (4-(4-(1-hydroxy-2,2,5,5-tetramethylpyrrolidin-3-yloxy)-1,2,5-thiadiazol-3-yl)morpholine hydrogen chloride)

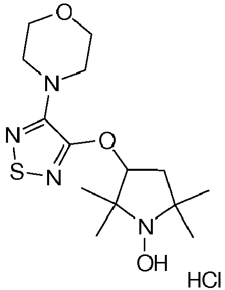

### Step a: Preparation of (4-(4-(2,2,5,5-tetramethylpyrrolidin-1-oxyl-3-yloxy)-1,2,5-thiadiazol-3-yl)morpholine)

To a solution of 3-hydroxypyrroline-1-oxyl (0.87g, 5.5 mmol) in t-BuOH(50mL), t-BuOK(0.81g, 7.2 mmol) was added. After the mixture was stirred for half an hour, 4-(4-chloro-1,2,5-thiadiazol-3-yl)morpholine (1.3g, 6.3 mmol) was added. The mixture was stirred at 40° C for 2 days. Then the reaction was quenched by adding 50 mL of water to the reaction mixture. The mixture was extracted with EtOAc( 3 x 150m1). The combined organic phase was washed with brine (50 mL) and was concentrated in vacuum. The crude solid was purified by column chromatography (silica gel, EtOAc/Hexane: 1:5). 0.36 g of orange solid was obtained. The yield was 20%.

### Step b:

To the solution of 4-(4-(2,2,5,5-tetramethylpyrrolidin-1-oxyl-3-yloxy)-1,2,5-thiadiazol-3-yl)morpholine (0.36g, 1.1 mmol) in 2-propanol (15mL) was added a saturated solution containing hydrogen chloride in 2-propanol (5mL) in one portion. The reaction mixture was stirred at room temperature for 2 hrs. The solvent was removed in vacuum and 5 ml of diisopropyl ether was added. White solid (0.27g) was obtained. The yield was 67%.

### Preparation of Compound 33: (1-hydroxy-2,2,5,5-tetramethylpyrrolidin-3-yl)methanol hydrogen chloride)

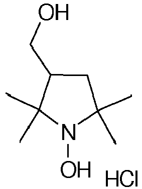

To the solution of (2,2,5,5-tetramethylpyrrolidin-1-oxyl-3-yl)methanol (0.41g, 2.4 mmol) in 2-propanol (10mL) was added a saturated solution containing hydrogen chloride in 2-propanol (5mL) in one portion. The reaction mixture was heated to 40°C for 0.5 h and then allowed to cool off to room temperature. The solvent was removed in vacuum and 2 mL of diisopropyl ether was added. White solid (0.31g) was obtained. The yield was 63%.

### Preparation of Compound 34: ((1-hydroxy-2,2,6,6-tetramethylpiperidin-4-yl)methanol hvdrogen chloride)

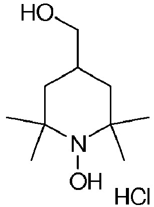

To the solution of 2,2,6,6-tetramethylpiperidin-1-oxyl-4-yl)methanol (0.33g, 1.7 mmol) in 2-propanol (10mL) was added a saturated solution containing hydrogen chloride in 2-propanol (5mL) in one portion. The reaction mixture was heated to 40°C for 0.5 h and then allowed to cool off to room temperature. The solvent was removed in vacuum and 3 mL of diisopropyl ether was added. A white solid (0.26g) was obtained via filtration. The yield was 67%.

### Preparation of Compound 35: (1-hydroxy-1,2,3,6-tetrahydro-2,2,6,6-tetramethylpyridine hydrogen chloride)

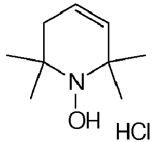

To the solution of 1,2,3,6-tetrahydro-2,2,6,6-tetramethylpyridin-1-oxyl (0.5g, 3.2 mmol) in 2-propanol (10mL) was added a saturated solution containing hydrogen chloride in 2-propanol (5ml) in one portion. The reaction mixture was heated to 40°C for 0.5 h and then allowed to cool off to room temperature. The solvent was removed in vacuum and 5 ml of diisopropyl ether was added. A white solid (0.39g) was obtained via filtration. The yield was 63%.

### Preparation of Compound 36 : (((1-hydroxy-2,5-dihydro-2,2,5,5-tetramethyl-1H-pyrrol-3-yl)methyl)morpholine bis-hydrogen chloride)

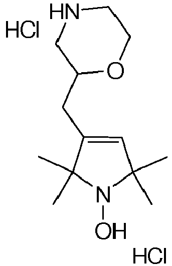

To the solution of 2,5-dihydro-2,2,5,5-tetramethyl-1H-pyrrol-1-oxyl-3-yl)methyl)morpholine (0.41g, 1.7 mmol) in 2-propanol (10mL) was added a saturated solution containing hydrogen chloride in 2-propanol (5mL) in one portion. The reaction mixture was heated to 40oC for 0.5 h and then allowed to cool off to room temperature. The solvent was removed in vacuum and 5 mL of diisopropyl ether was added. A white solid (0.32g) was obtained via filtration. The yield was 59%.

### Preparation of Compound 37: (1-hydroxy-2,2,5,5-tetramethylpyrrolidin-3-one hydrogen chloride)

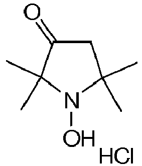

To the solution of 2,2,5,5-tetramethylpyrrolidin-1-oxy-3-one (0.3g, 1.9 mmol) in 2-propanol (10mL) was added a saturated solution containing hydrogen chloride in 2-propanol (5mL) in one portion. The reaction mixture was heated to 40°C for half an hour and then allowed to cool off to room temperature. The solvent was removed in vacuum and 5 mL of diisopropyl ether was added. A white solid (0.22g) was obtained via filtration. The yield was 59%.

### Preparation of Compound 38: (N-(1-hydroxy-2,2,5,5-tetramethylpyrrolidin-3-yl)cyclopropanecarboxamide hydrogen chloride)

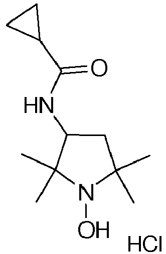

To the solution of the nitroxide compound (0.28g, 1.3 mmol) in 2-propanol (15mL) was added a saturated solution containing hydrogen chloride in 2-propanol (5mL) in one portion. The reaction mixture was heated to 40°C for 0.5 h and then allowed to cool off to room temperature. The solvent was removed in vacuum and 2 mL of diisopropyl ether was added. A white solid (0.19g) was obtained via fiiltration. The yield was 56%.

### Preparation of compound 39: (N-Hydroxyl-3,3,5,5-tetramethylmorpholin-2-one hydrochloride)

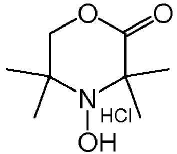

N-Oxyl-3,3,5,5-tetramethylmorpholin-2-one (Sagdeev et al; J.Struct.Chem.(Engl.Transl.),8,625,1967) (0.5 g, 2.9 mmol) was dissolved in saturated hydrogen chloride solution 2-propanol (10 mL) and left at 40 °C for half an hour. After the solvent was evaporated, a white solid (0.42 g) was obtained. The yield was 69. mp 145 °C (dec.). ¹H NMR (300 MHz, MeOD-d₄): δ4.59 (2H, s), 1.79 (6H, s), 1.52 (6H, s). ¹³C NMR (75 MHz, MeOD-d₄): δ170.67, 71.86, 71.75, 65.45, 24.91, 19.47. Anal. Calcd for C₈H₁₆ClNO₃: C, 45.83; H, 7.69. N, 6.68. Found: C, 45.91; H, 7.31; N, 6.64.

### Preparation of compound 40 (N-Hydroxyl-N,N-bis(1-ethoxy-2-methylpropan-2-yl)amine hydrochloride)

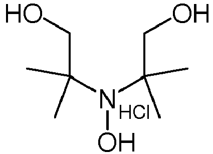

To N-oxyl-N,N-bis(1-ethoxy-2-methylpropan-2-yl)amine (J. T. Lai; Synthesis, 2,122-123,1984) (0.34 g, 1.46 mmol), saturated hydrogen chloride solution in 2-propanol (2.5 mL) was added. Brown color disappeared right away. Two hours later at room temperature, solvent was evaporated to afford a light yellow syrup (0.35 g). Yield was 89%. ¹H NMR (300 MHz, MeOD) δ3.6 (4H, *q*, *J*=6.98), 3.51 (4H, s), 1.49 (12, s), 1.24 (6H, t, *J*=6.85). ¹³C NMR (75 MHz, MeOD) δ163.86, 76.24, 68.0, 63.9, 24.18, 15.49. Anal. Calcd for C₁₂H₂₈ClNO₃: C, 53.42; H, 10.46; N 5.19. Found: C, 53.16; H, 10.82; N 5.51.

### Preparation of compound 41 (1,4-dihydroxy-4-n-butyl-2,2,6,6-tetramethylpiperidine hydrochloride)

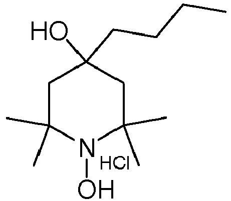

To a solution of 4-oxo-2,2,6,6-tetramethylpiperidine-1-oxyl (5.1g, 0.03 mol) in anhydrous THF (50 mL) at 0-5 °C under nitrogen was added n-butyl lithium in hexane (2.5M, 15 mL, 0.038 mol) dropwise. The mixture was stirred at room temperature for 2 hours. Then water (100 mL) was added to the reaction mixture. The mixture was extracted with ethyl acetate (3 X 50 mL). The combined solution of organic layers was dried over sodium sulphate and concentrated to give a residue, which was purified by column chromatography (silica gel, ethyl acetate/hexanes=10:1). 0.5g of orange oil was obtained. Yield was 7%.

To a solution of above orange oil (0.43g, 1.88mmol) in 2-propanol (10 mL) was added a saturated hydrogen chloride solution in 2-propanol (5 mL) in one portion. The reaction mixture was heated to 40 °C for 0.5 h and then allowed to cool off to room temperature. The solvent was removed in vacuum and 5 mL of isopropyl ether was added. White solid (0.35g) was obtained. Yield was 70.2%. mp 187.0°C (dec.). ¹H NMR (300MHz, MeOD) δ 2.00(m, 4H),1.70(s, 6H)1.48(m, 12H), 0.97(m, 3H). ¹³C NMR (75MHz, MeOD) δ 68.86, 67.50, 45.55, 44.38, 28.10, 24.39, 22.76, 19.76, 12.96. Anal. Calcd for C₁₃H₂₈ClNO₂: C, 58.74; H, 10.62; N, 5.27. Found: C, 58.65; H, 10.69; N, 5.24.

### Preparation of compound 42: (1,4-Dihydroxy-4-phenyl-2,2,6,6-tetrmethylpiperidine hydrochloride)

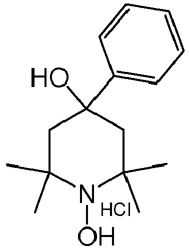

### Step 1

To a solution of 4-oxo-2,2,6,6-tetramethylpiperidine-1-oxyl (2.5g, 15 mmol) in anhydrous THF (50 mL) at room temperature under nitrogen was added phenylmagnesium bromide solution in THF (1.0M, 16.5 mL, 16.5 mmol) dropwise. The mixture was then stirred at room temperature for 2 hours and heated at 50 °C for one hour. After it was cooled to room temperature, saturated ammonium chloride (50 mL) was added to the reaction mixture. It was extracted with ethyl acetate (2 X 150 mL). The combined solution of organic layers was dried over sodium sulphate and concentrated in vacuum to give a residue, which was purified by column chromatography (silica gel, ethy acetate/hexanes=3:1). 1.8g of orange oil was obtained. Yield was 48%.

### Step 2

To a solution of above orange oil (0.41g, 1.65 mmol) in 2-propanol (10 mL) was added a saturated hydrogen chloride solution in 2-propanol (5 mL) in one portion. The reaction mixture was heated to 40 °C for 0.5 h and then allowed to cool off to room temperature. The solvent was removed in vacuum and 5 mL of isopropyl ether was added. White solid (0.28g, 0.98mmol) was obtained. Yield was 59.4%. mp 201.7°C (dec.). ¹H NMR (300MHz, MeOD) δ 7.58(m, 2H), 7.40(m, 2H), 7.30(m, 1H), 2.50(m, 2H), 2.13(d, 2H), 1.82(s, 6H), 1.54(s, 6H). ¹³C NMR (75MHz, MeOD) δ 128.0, 126.91, 124.22, 47.13, 28.17, 19.95. Anal. Calcd for C₁₅H₂₄ClNO₂: C, 63.04; H, 8.46; N, 4.90. Found: C, 63.00; H, 8.78; N, 4.84.

### Preparation of compound 43: (4-Benzyloxy-1-hydroxy-2,2,6,6-tetramethylpiperidine hydrochloride)

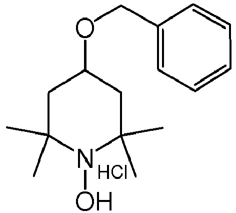

### Step 1

To a solution of 4-hydroxy-2,2,6,6-tetramethylpiperidine-1-oxyl (6.27g, 36 mmol) in DMF (150 mL) at 0-5 °C under nitrogen was added sodium hydride (2.6g, 65 mmol). After it was stirred at the same temperature for 45 minutes, benzyl bromide (6.23g, 36 mmol) was added. The mixture was stirred at room temperature overnight. Water was added to the reaction mixture slowly. It was then extracted with ethyl acetate (3 X 200 mL). The combined organic phase was dried over sodium sulfate and concentrated in vacuum to give a residue, which was purified by column chromatography (silica gel, hex/ethyl acetate=4:1). 5g of orange oil was obtained. Yield was 52%. Used as is in the next step.

### Step 2

To the solution of above orange oil (0.50g, 1.91 mmol) in 2-propanol (15 mL) was added a saturated hydrogen chloride solution in 2-propanol (5 mL) in one portion. The reaction mixture was heated to 40 °C for 0.5 h and then allowed to cool off to room temperature. The solvent was removed in vacuum and 2 mL of diisopropyl ether was added. A white solid (0.48g, 1.60mmol) was obtained via filtration. The yield was 83.77%. mp 181.5°C (dec.). ¹H NMR (300MHz, DMSO) δ 11.98, 11.46(d, 1H), 7.34(m, 5H), 4.55(s, 2H), 4.00(m, 1H), 2.24(m, 2H), 1.95(m, 2H), 1.47(s, 6H), 1.34(s, 6H). ¹³C NMR (75MHz, DMSO) δ 1 38.98, 128.72, 127.91, 69.81, 68.11, 41.60, 27.96, 20.65. Anal. Calcd for C₁₆H₂₆ClNO₂: C, 64.09; H, 8.74; N, 4.67. Found: C, 63.74; H, 8.92; N, 4.57.

### Preparation of Compound 44: (1-hydroxy-4-(4-phenyl-1,2,5-thiadiazol-3-yloxy)-2,2,6,6-tetramethylpiperidine hydrochloride)

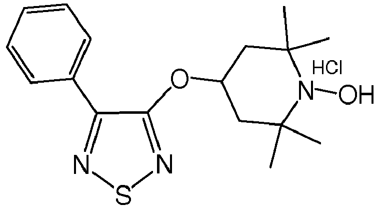

### Step 1

3-phenyl-4-chloro-1,2,5-thiadiazole was synthesized according to the procedure described in the "General procedure A." ¹H NMR (300MHz, CDCl₃) δ 7.53 (3H, m), 7.97 (2H, m). ¹³C NMR (75MHz, CDCl₃, δ), 128.61, 128.65, 130.19, 130.76, 143.42, 157.92.

### Step 2

To a solution of 4-hydroxy-2,2,6,6-tetramethylpiperidine-1-oxyl (2.02g, 11.7 mmol) and t-BuOK (1.7g, 15.2 mmol) in t-BuOH (30 mL) was added 3-phenyl-4-chloro-1,2,5-thiadiazole (1.96g, 10mmol). The dark solution was stirred at room temperature over weekend. The reaction was monitored by TLC (Hex/EtOAc 1/1). Water (10 mL) was added and the mixture was stirred for another 30 min. The mixture was extracted with CH₂Cl₂ (3x20 mL). The organic phase was dried over MgSO₄ and evaporated in vacuum. The residue was separated by column chromatography (silica gel, Hexane (300 mL), Hex/EtOAc (10/1, 1000 mL)). 2.01g of red solid was obtained. The yield was 60.5%. Used as is in the next step.

1.2g of the above red solid was dissolved in 60 mL of 2-propanol at 50°C. Saturated hydrogen chloride solution in 2-propanol was added until the solution became light yellow. The solvent was removed and the residue was washed with CH₂Cl₂ to get white a solid. Yield was 84.1%. mp 220.8°C (dec.). ¹H NMR (300MHz, CD₃OD) δ1.58 (6H, s), 1.62 (6H, s), 2.20 (2H, t, J=13.1Hz), 2.72 (1H, m), 2.77 (1H, m), 5.56 (1H, m).7.49 (3H, m), 8.12 (2H, m). ¹³C NMR (75MHz, CD₃OD) δ19.25, 27.02, 40.95, 68.79, 70.22, 127.31, 128.27, 129.46, 131.25, 147.84, 160.78. Anal. Calcd for C₁₇H₂₄ClN₃O₂S: C, 55.20; H, 6.54; N, 11.36. Found: C, 54.98; H, 6.64; N, 11.16.

### Preparation of compound 45: (4-(2,2,6,6-tetramethylpiperidin-4-yloxy)-1,2,5-thiadiazole-3-carbonitrile hydrochloride)

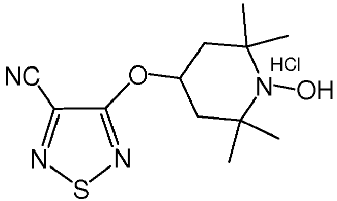

### Step 1

3-cyano-4-chloro-1,2,5-thiadiazole was synthesized according to the proceduredescribed in the "General procedure A." ¹³C NMR (75MHz, CDCl₃, δ), 110.05, 133.35, 149.10.

### Step 2

To a solution of 4-hydroxy-2,2,6,6-tetramethylpiperidine-1-oxyl (1.1g, 6.3 mmol) and t-BuOK (1.0g, 8.3 mmol) in t-BuOH (20 mL) was added 3-cyano-4-chloro-1,2,5-thiadiazole (0.77g, 5.3mmol). The dark solution was stirred at room temperature over weekend. The reaction was monitored by TLC (Hex/EtOAc 1/1). Water (10 mL) was added and the mixture was stirred for another 30 min. The mixture was extracted with CH₂Cl₂ (3x20 mL). The organic phase was dried over MgSO₄ and evaporated. The residue was separated by column chromatography (silica gel, Hexane (300 mL), Hex/EtOAc (10/1, 1000 mL)). 0.45g of red solid was obtained. The yield was 30%.

0.45g of the above red solid was dissolved in 10 mL of 2-propanol at 50 °C. Saturated hydrogen chloride solution in 2-propanol was added until the solution became light yellow. The solvent was removed to almost dryness and the residue was soaked with hexane and filtered. 0.25g of product was obtained. Yield was 49%. mp 179.2°C (dec.). ¹H NMR (300MHz, DMSO) δ1.40 (6H, s), 1.52 (6H, s), 2.33 (2H, m), 2.44 (2H, m), 5.33 (1H, m).11.63 (1H, s), 12.42 (1H, s). Anal. Calcd for C₁₂H₁₉ClN₄O₂S: C, 45.21; H, 6.01; N, 17.57. Found: C, 45.13; H, 5.96; N, 17.21.

### Preparation of compound 46: (5-(2,5,-dihydro-4-(3,4,5-trimethoxyphenyl)-1-hydroxy-2,2,5,5-tetramethyl-1H-pyrrol-3-yl)-2-methoxybenzaldehyde hydrochloride)

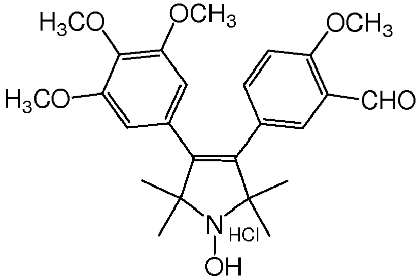

### Step 1

A mixture of 3,4-dibromo-2,5-dihydro-2,2,5,5-tetramethyl-1H-pyrrol-1-oxy (A. V. Chudinov et al, Bull.Acad.Sci.USSR Div.Chem.Sci.(Engl.Transl.), 32(2), 356-360(1983)) (5g, 16.7 mmol), 3,4,5-trimethoxybenzene boronic acid (1.94g, 2.4 mmol), barium hydroxide (2.7g, 17 mmol) and PdCl₂(Ph₃P)₂ (0.58g, 0.83 mmol) in dioxane (150 ml) and water (15 ml) was refluxed at 110°C until the boronic acid was consumed. The solid was filtered off and washed with dioxane (2x5 mL). The filtrate was evaporated and the residue was purified by column chromatography (silica gel, Hex/EtOAc 95/5 (1800 mL), Hex/EtOAc (85/15 1000 mL). 0.61g of 3-bromo-2,5-dihydro-4-(3,4,5-trimethoxyphenyl)-2,2,5,5-tetramethyl-1H-pyrrol-1-oxy was obtained. The yield was 17%. MS⁺: 384. Anal. Cacld for C₁₇H₂₃BrNO₄: C, 53.00; H, 6.02; N, 3.64. Found: C, 53.07; H, 6.09; N, 3.60.

### Step 2

A mixture of 3-bromo-2,5-dihydro-4-(3,4,5-trimethoxyphenyl)-2,2,5,5-tetramethyl-1H-pyrrol-1-oxy (0.4g, 1 mmol), formylmethoxybenzene boronic acid (0.27g, 1.5 mmol), barium hydroxide (0.2g, 1.2mmol) and PdCl₂(Ph₃P)₂ (0.035g, 0.05mmol) in dioxane (8 mL) and water (2 mL) was refluxed at 110 °C until the boronic acid was consumed (monitored by TLC 9/1 EtOAc/MeOH). The solid was filtered off and washed with dioxane (2x5 mL). The filtrate was evaporated and the residue was purified by prep. TLC. There were four bands collected. The 3^{rd} band was the expected product (130mg, 29.5. The 3^{rd} spot (100mg) was converted at room temperature to hydrochloride by dissolving in 2-propanol (10 mL) and saturated hydrogen chloride solution in 2-propanol (2 mL) and warming at 45 °C until the brown color of the solution turned light yellow. The solvent was removed in vacuum and foam was obtained. Yield was 100%. ¹H NMR (300MHz, CDCl3) δ1.72 (12H, s, br), 3.75 (6H, s), 3.80 (3H, s), 3.91 (3H, s), 6.27 (2H, s), 6.89 (1H, m), 7.30( 1H, m), 7.62 (1H, m), 10.40 (1H, s). ¹³C NMR (75MHz, CDCl3) δ14.11, 22.65, 25.31, 31.58, 55.79, 56.28, 77.55, 77.80, 106.79, 112.05, 124.34, 124.66, 127.17, 128.67, 136.92, 138.08, 139.16, 141.37, 153.07, 161.61, 189.04. Anal. Calcd for C₂₅H₃₂ClNO₆.(C₃H₈O)_{0.5}: C, 62.65; H, 7.14; N, 2.76. Found: C, 62.36; H, 6.86; N, 2.28.

### Preparation of compound 47: (3-((1-hydroxy-2,5-dihydro-2,2,5,5-tetramethyl-1H-pyrrol-3-yl)methoxy)-4-methyl-1,2,5-thiadiazole hydrochloride)

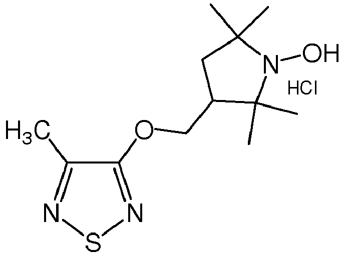

### Step 1

3-methyl-4-hydroxy-1,2,5-thiadiazole was synthesized according to the proceduredescribed in the "General procedure A." ¹H NMR (300MHz, CDCl₃, δ), 2.48 (3H, CH3), 12.16 (1H, OH). 13C NMR (75MHz, CDCl₃, δ),14.58, 149.18, 163.03.

### Step 2

To a stirred solution of 3-methyl-4-hydroxy-1,2,5-thiadiazole (4 mol) and CsF-silica (1.5 mmol) in 50 mL of acetonitrile, 3-(bromomethyl)-2,5-dihydro-2,2,5,5-tetramethyl-1H-pyrrol-1-oxy (H. O. Hankovszky et al; Synthesis, 11, 914-916, 1980) (8.0 mmol) were added. Then the mixture was continued for stirring at room temperature or refluxed up to completion of the reaction, indicated by TLC monitoring. The reaction mixture was filtered, the solvent evaporated in vacuum and the residue dissolved in ethyl acetate. The product was purified, by prep. TLC using dichloromethane/hex (3/1) to afford the pure ether products (0.94g). The yield was 87%.

0.45g of above product was dissolved in 10 mL of 2-propanol. Any insoluble was filtered off. The saturated hydrogen chloride in 2-propanolwas added until the solution became acidic. The solution was warmed in water bath (45°C) until the color disappeared (pale yellow). The solvent was removed and the residue was diluted in 5 mL of EtOAc and 15 mL of Hexane. The solution was kept overnight for crystallization. The white crystal was collected and washed with hexane (2x5 mL). 0.40 grams of product was obtained. Yield was 78.0%. mp 173.2°C. ¹H NMR (300MHz, DMSO-d6) δ1.48 (12H, m), 2.37 (3H, s), 5.02 (2H, s), 7.07 (1H, s), 11.69 (1H, s), 12.35 (1H, s). ¹³C NMR (75MHz, DMSO-d6) δ14.82, 49.06, 65.73, 131.65 (130.70), 139.58 (138.09), 149.10, 162.27. Anal. Calcd for C₁₂H₂₀ClN₃O₂S: C, 47.13; H, 6.59; N, 13.74. Found: C, 47.04; H, 6.81; N, 13.42.

### Preparation of compound 48: (1-Hydroxyl-4-(3,4,5-trimethoxybenzyloxy)-2,2,6,6-tetramethylpiperidine hydrochloride)

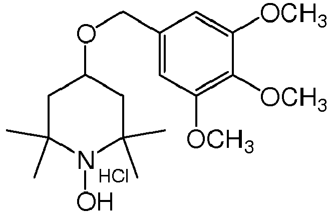

### Step 1

To a solution of 4-hydroxy-2,2,6,6-tetramethylpiperidine-1-oxyl (5 g, 29.0 mmol) in t-butanol (80 mL), potassium *tert*-butoxide (4.88 g, 43.5 mmol) and 3,4,5-Trimethoxybenzyl chloride (6.91 g, 31.9 mmol) were added sequentially and stirred at room temperature overnight. Next day, another portion of potassium tert-butoxide (2.44 g) and 3,4,5-Trimethoxybenzyl chloride (1.4 g) were added and refluxed for 2 hours, both starting materials still remained. The reaction mixture was treated with saturated NH₄Cl (15 mL), filtered, dried with Na₂SO₄ and solvent was evaporated in vacuum. After the residual syrup was purified through column chromatography (silica gel, hexane, hexane : EtOAc (9:1)), pure product, 1-Oxyl-4-(3,4,5-trimethoxybenzyloxy)-2,2,6,6-tetramethylpiperidine (6.1 g) was obtained. The yield was 60%. Used as is in the next step.

### Step 2

To 1-Oxyl-4-(3,4,5-trimethoxybenzyloxy)-2,2,6,6-tetramethylpiperidine (2.0 g, 5.13 mmol) which was cooled down in an ice-water bath, saturated hydrogen chloride solution in 2-propanol (20 mL) was added slowly. Half an hour later, the solvent was removed in vacuum. The solid residue was dissolved in MeOH (30 mL) with heating and diisopropyl ether (50 mL) was added with stirring. White solid was formed. The mixture was left in refrigerator overnight and afforded pure product (1.0 g, 2.56 mmol). Yield was 50%. ¹H NMR (300 MHz, DMSO) δ12.02 (1H, s), 11.43 (1, s), 6.61 (2H, s), 4.44 (2H, s), 3.99-3.92 (1H, m), 3.75 (6H, s), 3.62 (3H, s), 2.23-2.20 (2H, m), 1.99-1.91 (2H, m), 1.49-1.31 (12H, m). ¹³C NMR (75 MHz, DMSO) δ152.76, 136.77, 134.07, 104.82, 69.64, 67.54, 67.4 , 59.96, 55.83, 41.12, 27.48, 20.2. Anal. Calcd for C₁₉H₃₂ClNO₅: C, 58.53; H, 8.27; N 3.59. Found: C, 58.45; H, 8.42; N, 3.54.

### Preparation of compound 49: (5-(1,2-dithialan-3-yl)-N-(1-hydroxy-2,2,6,6-tetramethylpiperidin-4-yl)pentanamide hydrochloride)

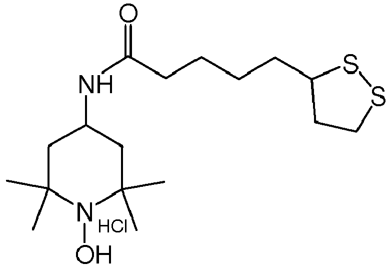

### Step 1

To a solution of (±)-α-Lipoic acid (2.06g, 10 mmol), 4-amino-2,2,6,6-tetramethylpiperidine-1-oxyl (1.71g, 10 mmol) and 4-DIMETHYLAMINOPYRIDINE (DMAP) (0.6g, 5 mmol) in CH₂Cl₂ (50 mL) at 0-5 °C, DDC (2.3g, 11mmol) in dichloromethane (50 mL) was added dropwise. After addition was complete, the mixture was stirred at room temperature for 4 hours. 100 mL of water was added to the reaction mixture and the reaction mixture was kept stirring at room temperature overnight. After filtration, the mixture was washed with water (2x50 mL), 1N HCl (20 mL) and saturated Na₂CO₃ (20 mL) and dried over MgS04. After MgS04 was filtered off, the solvent was removed in vacuum to give a solid. The solid was purified by column chromatography (silica gel, EtOAc/Hexane 1:10). The product is an orange solid (3.58g). The yield was 94.9%. Used as is in the next step.

### Step 2

To a solution of above orange solid (1.8g) in 2-propanol (10 mL) was added a saturated hydrogen chloride solution in methanol (20 mL) in one portion, and the reaction mixture was stirred at 40 °C for 2 hrs. TLC showed that the staroom temperatureing material disappeared. The solution is light yellow. The solvent was removed in vacuum to give a light yellow solid (1.5g). Yield was 75%. mp 164.7°C (dec.). ¹H NMR (300MHz, CDCl3) δ 4.35(m, 1H), 3.62(m, 1H), 3.18(m, 2H), 2.22(m, 4H), 1.94(m, 4H), 1.69(m, 6H), 1.54(s, 12H). ¹³C NMR (75MHz, CDCl3) δ 175.64, 69.97, 57.64, 43.02, 41.37, 40.45, 39.42, 36.79, 29.82, 28.33, 27.03, 26.63, 25.30, 20.41. Anal. Calcd for C₁₇H₃₃ClN₂O₂S₂: C, 51.43; H, 8.38; N, 7.06. Found: C, 51.64; H, 8.51; N, 6.68.

### Preparation of compound 50: (1-Hydroxy-2,3,6-trihydro-4-(3,4,5-trimethoxyphenyl)-2,2,6,6-tetramethylpiperidine hydrochloride)

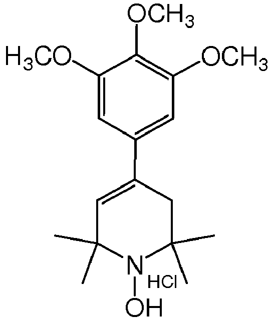

### Step 1

A mixture of 1,2,3,6-tetrahydro-4-iodo-2,2,6,6-tetramethylpyridin-1-oxy (T. Kalai et al; Synthesis, 3, 439-446, 2006) (0.54g, 1.9 mmole), indole-5-boronic acid (0.41g, 1.9 mmole), potassium carbonate (0.69g, 5 mmole), and PdCl₂(Ph₃P)₂ (0.09g, 0.25 mmole) in dioxane (20 mL) and water (5 mL) was stirred and refluxed until the staroom temperatureing compounds were consumed. Four hours later, TLC showed that the staroom temperatureing materials had disappeared. After filtration and the solvent was removed in vacuum. The crude mixture was purified by flash column chromatography (silica gel, EtOAc/Hexane=1:2). 0.43 g of brownish solid was obtained.

### Step 2

To a solution of above brownish solid (0.43g) in 2-propanol (5 mL) was added a saturated hydrogen chloride solution in 2-propanol (10 mL) in one portion, and the reaction mixture was stirred at 40 °C for 2 hrs. TLC showed that the staroom temperatureing material disappeared and the solution turned colorless. The solvent was removed in vacuum to give an off-white solid (0.35g). Yield was 76.7%. mp 159.9°C (dec.). ¹H NMR (300MHz, CDCl3) δ 6.76(s, 2H), 6.10(s, 1H), 3.91(s, 6H), 3.83(s, 3H), 2.98(dd, 2H), 1.66(s, 6H), 1.63(s, 3H), 1.56(s, 3H). ¹³C NMR (75MHz, CDCl₃) δ 125.67, 102.98, 59.74, 55.40, 38.50, 26.10, 24.92, 20.89, 19.59(Dept135).

### Preparation of compound 51: (3-((2,5-dihydro-1-hydroxy-2,2,5,5-tetramethyl-1H-pyrrol-3-yl)methoxy-4-isopropyl-1,2,5-thiadiazole hydrochloride)

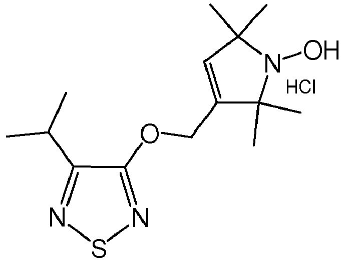

### Step 1

3-isopropyl-4-hydroxy-1,2,5-thiadiazole was synthesized according to the procedure described in the "General procedure A." ¹H NMR (300MHz, CDCl₃, δ), 1.00 (6H, d, J=6.7Hz), 2.22 (1H, m), 2.71 (2H, d, J=6.7Hz). ¹³C NMR (75MHz, CDCl₃, δ), 22.39, 27.57, 37.59, 152.28, 162.86.

### Step 2

To a stirred solution of 3-isopropyl-4-hydroxy-1,2,5-thiadiazole (0.39 g, 2.7 mmol) in acetone (10 mL) were added potassium carbonate (1.2 g, 8.1 mmol) and 3-(bromomethyl)-2,5-dihydro-2,2,5,5-tetramethyl-1H-pyrrol-1-oxy (H.O. Hankovszky et al, Synthesis, 914-916, 1980) (0.7g , 3 mmol). The mixture was heated under reflux at 65°C for 16 h. It was cooled to room temperature, filtered and the filtrate concentrated in vacuum. The residue was dissolved in EtOAc (20 mL) and washed sequentially with 1 M aqueous sodium hydroxide (10 mL) and brine (2x10 mL). The organic layer was dried over magnesium sulfate and concentrated in vacuum. The resultant residue was purified using prep. TLC (Hex./EtOAc (6/1)) to afford a brown oil (0.66g). The yield was 82.5%.

0.45g of above brown oil was dissolved in 10 mL of 2-propanol. Saturated hydrogen chloride solution in 2-propanol was added until the solution became acidic. The solution was warmed in water bath (45 °C) until the color disappeared. The solvent was removed in vacuum and the residue was diluted in 5 mL of EtOAc and 15 mL of Hexane. The solution was kept overnight for crystallization. The white crystal was collected and washed with hexane (2x5 mL). 0.41g of product was obtained. mp 155.7°C. Yield was 80.7%. ¹H NMR (300MHz, DMSO) δ 1.25 (6H, d, J=6.9Hz), 1.49 (12H, m), 3.13 (1H, sep. J=6.9Hz), 5.04 (2H, s), 6.05 (1H, s), 11.73 (1H, s), 12.35 (1H, s). ¹³C NMR (75MHz, DMSO) δ 20.95, 28.92, 65.56, 130.99, 138.23, 157.20, 161.51. Anal. Calcd for C₁₄H₂₄ClN₃O₂S: C, 50.36; H, 7.25; N, 12.59. Found: C, 50.57; H, 7.51; N, 12.33.

### Preparation of compound 52: (4-[(4-methylpiperazin-1-yl)]-3-[(2,2,6,6-tetramethyl-1-Hydroxy piperidinyl)]-1,2,5-thiadiazole dihydrochloride)

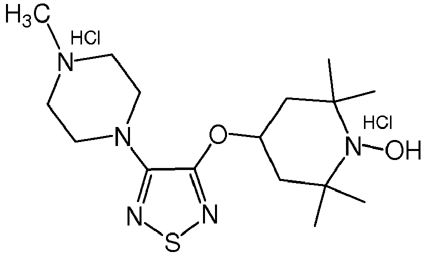

### Step 1

3,4-Dichloro-1,2,5-thiadiazole (4.65g, 30mmol) was added over a 30min period at 105-110°C to 13.3ml (120mmol) of n-methylpiperazine. After addition, the reaction mixture was stirred for 2hr at 105-110°C (monitored by TLC, Hex/EtOAc 1/3). The mixture was cooled to room temperature, aqueous ammonium (20 mL) was added and the mixture was extracted with CH₂Cl₂ (5x20 mL). The combined organic phase was washed with ammonia (10 mL), water (2x10 mL) and dried over MgSO₄. The solvent was removed and the residue was purified (silica gel, EtOAC). 5.9g of 4-(4-methypiperazin-1-yl)-3-chloro-1,2,5-thiadiazole was obtained. The yield was 90.2%. ¹H NMR (300MHz, CDCl₃, δ) 2.37 (3H, s), 2.58 (4H, t, J=5.0Hz), 3.53 (4H, t, J=5.0). 13C NMR (75MHz, CDCl₃, δ), 46.16, 48.80, 54.52, 135.26, 159.15.

### Step 2

To a solution of 4-hydroxy-2,2,6,6-tetramethylpiperidine-1-oxyl (2.02g, 11.7 mmol) and t-BuOK (1.7g, 15.2 mmol) in t-BuOH (30 mL) was added 3-[(4-N-methylpiperazine-1-yl)]-4-chloro-1,2,5-thiadiazole (2.19g, 10 mmol). The dark solution was stirred at room temperature over weekend. The reaction was monitored by TLC (MeOH/EtOAc 1/9). Water (10 mL) was added and the mixture was stirred for another 30 min. It was extracted with CH₂Cl₂ (3x20 mL). The combined dichloromethane layers were dried over MgSO₄ and evaporated in vacuum. The residue was purified by column chromatography (silica gel, Hex/EtOAc (1/1, 1000 mL), EtOAc/MeOH (10/1, 1000 mL)) to give 3.10 g of orange solid. Yield was 87.5%.

1.4g of the above orange solid was dissolved in 20 mL of 2-propanol at 50 °C. Saturated hydrogen chloride solution in 2-propanol was added until the solution became light yellow. The solvent was removed and the residue was triturated in acetone to give a white solid (1.5g). Yield was 97.2%. ¹H NMR (300MHz, DMSO) □ δ 1.406 (s, 3H), 1.545 (s, 3H), 2.326 (2H, t, J=12.2Hz), 2.450 (2H, m), 2.762and 2.777 (3H, two peaks 1/1 ), 3.160(2H, m), 3.442 (4H, m.), 4.090(2H, d, J=13.4Hz), 5.295 (1H, m), 11.387 (1H, s), 11.548 (1H, s), 12.594 (1H, s). ¹³C NMR (75MHz, DMSO) δ 20.79, 23.29, 25.95, 27.69, 42.45, 44.73, 51.85, 67.79, 71.69, 149.55, 152.85. Anal. Calcd for C₁₆H₃₁Cl₂N₅O₂S: C, 44.86; H, 7.29; N, 16.35. Found: C, 44.77; H, 7.40; N, 16.09.

### Preparation of compound 53: (4-(4-phenylpiperazin-1-yl)-3-[(2,2,6,6-tetramethyl-1-hydroxy piperidinyl)-4-oxy]-1,2,5-thiadiazole hydrochloride)

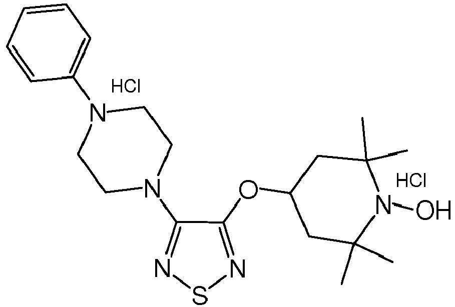

### Step 1

4-(4-phenyl-piperazin-1-yl)-3-chloro-1,2,5-thiadiazole was synthesized according to the procedure described in the "General procedure B." NMR 1H NMR (300MHz, CDCl₃, δ) 2.36 (4H, t, J=5.0Hz), 3.67 (4H, t, J=5.0), 6.96 (3H, m), 7.32 (2H, m). 13C NMR (75MHz, CDCl₃, δ), 48.90, 49.02, 116.48, 120.39, 129.24, 135.39, 151.09, 159.07.

### Step 2

To a solution of 4-hydroxy-2,2,6,6-tetramethylpiperidine-1-oxyl (2.02g, 11.7 mmol) and t-BuOK (1.7g, 15.2 mmol) in t-BuOH (30 mL) was added 3-(4-N-phenylpiperazine-1-yl)-4-chlorothiadiazole (2.81g, 10 mmol). The dark solution was stirred at room temperature over weekend. 20 ml of THF was added in order to dissolve the amine. The reaction was monitored by TLC (Hex/EtOAc 8/1). Water (10 ml) was added and the mixture was stirred for another 30 min. The mixture was extracted with CH₂Cl₂ (3x40 mL). The organic layers were combined, dried over MgSO₄ and evaporated in vacuum. The residue was separated by column chromatography (silica gel, Hexane (300 mL), Hex/EtOAc (85/15, 1500 mL)). 2.63g of orange solid was obtained. Yield was 63.2%. 1.3g of the above orange solid was dissolved in 30 mL of 2-propanol at 50 °C. Saturated hydrogen chloride solution in 2-propanol was added until the solution became light yellow. The volume was reduced to less than 10 mL and acetone (20 mL) was added. The off white precipitate was collected and washed with acetone. The solid was tried to dissolve in 40 mL of 2-propanol at 65°C. Methanol (15 mL) was added to help dissolving. The volume was again reduced to 20 mL and stood overnight. The white solid was collected, washed with acetone and dried in oven (0.82g). The yield was 80%. mp 205.8°C (dec.). ¹H NMR (300MHz, DMSO) δ 1.419 (s, 3H), 1.553 (s, 3H), 2.336 (2H, t, J=12.2Hz), 2.475 (2H, m), 3.461 (4H, s br.), 3.766 (4H, s, br.), 5.325 (1H, m), 7.096 (1H, m), 7.382 (4H, m), 11.564 (1H, s), 12.594 (1H, s). ¹³C NMR (75MHz, DMSO) δ □20.789, 27.693, 46.757, 50.348, 67.941, 71.585, 118.350, 129.837, 150.216.

### Preparation of Example 55: (4-(4-(1-hydroxy-2,2,6,6-tetramethylpiperidin-4-yloxy)-1,2,5-thiadiazol-3-yl)thiomorpholine hydrochloride)

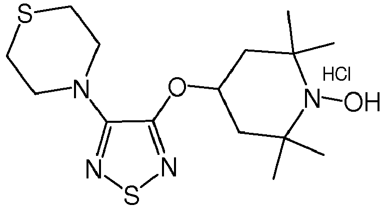

### Step 1

To 21.0 g of thiamorpholine in flask at 109 °C, 7.8 g of 3,4-dichloro-1,2,5-thiadiazole was added dropwise over 5 min. The mixture was kept stirring at 109 °C for 2 hrs. After the mixture was cooled down to room temperature the reaction was quenched by addition of 50 mL of water. It was extracted with EtOAc (3x100 mL). The organic phase was washed with 1N HCl (2x30 mL). The brown oil was purified by column chromatography after work up. 11.6 g of 3-chloro-4-thiamorpholin-yl-1,2,5-thiadiazole was obtained.

### Step 2

To a solution of 4-hydroxy-2,2,6,6-tetramethylpiperidine-1-oxyl (1.72 g, 10mmol) in 50 mL of t-BuOH, t-BuOK(1.36 g, 11 mmol) was added. The solution was kept stirring at room temperature for half an hour. Then 3-chloro-4-thiamorpholin-thiadiazole (1.12g, 20mmol) in 5 mL of THF was added during a period of 10 minutes. The reaction mixture was kept stirring overnight. The reaction mixture was poured into 400 mL of water with stirring. It was extracted with EtOAc (3 x 200 mL) and the organic phase was washed with 200 mL of brine. It was dried over Na₂SO₄. After filtration, the solvent was removed in vacuum to give a solid. The solid was purified by column chromatography (silica gel, EtOAc/Hexane 1:5). An orange solid (3.2g) was obtained and yield was 81.1%.

### Step 3

To a solution of above orange compound (0.8g) in 2-propanol (∼10 mL) was added a saturated solution containing hydrogen chloride in methanol (∼20 mL) in one portion, and the reaction mixture was stirred at 40 °C for 2 hrs. TLC showed that the starting material disappeared. The solution was light yellow. The solvent was removed in vacuum to give a light yellow solid (0.65g). Yield was 64.3%. mp 218.0°C (dec.). ¹H NMR (300MHz, CDCl₃) δ 11.74(b, 1H), 10.91(b, 1H), 5.35(m, 1H), 4.20(m, 4H), 2.73(m, 4H), 2.65(m, 2H), 2.39(m, 2H), 1.78(s, 2H), 1.54(s, 6H). Anal. Calcd for C₁₅H₂₇ClN₄O₂S₂: C, 45.61; H, 6.89; N, 14.18. Found: C, 45.44; H, 6.89; N 13.93.

### Preparation of compound 56: (2-(1-hydroxy 2,2,6,6-tetramethylpiperidin-4-ylidene)-1-(4-phenylpiperazin-1-yl)ethane hydrochloride)

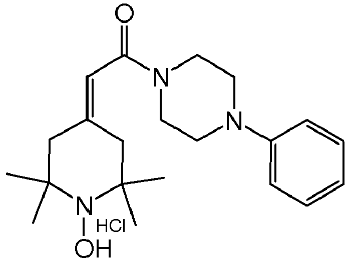

### Step 1

To a solution of triethylamine (2g, 20 mmol), 4-phenylpiperazine (2.55g, 20 mmol) in dichloromethane (100 mL) was added acid chlroride of Diethylphosphonoacetic acid (4.3g, 20 mmol) at 0-5 °C. After the addition was complete, the mixture was stirred at room temperature for 2 hours. The solvent was removed to dryness in vacuum. Water (100 mL) was added to the residue. The mixture was extracted with ethyl acetate (3 X 100 ml). The organic phase was dried and concentrated to give a residue, which was purified by column chromatography (silica gel, ethyl acetate). 4.0 grams of oil was obtained. Yield: 59%. Used as is in the next step.

### Step 2

To a solution of above oil (1.7g, 5 mmol) in THF (50 mL) was added sodium hydride (60; 0.24g, 6 mmol) at 0-5 °C. The mixture was then stirred at room temperature under nitrogen for 30 minutes, followed by adding a solution of 4-oxo-2,2,6,6-tetramethylpiperidine-1-oxyl (0.85g, 5 mmol). The mixture was stirred for 2 hours at room temperature Water (100 mL) was added to the reaction mixture. It was extracted with ethyl acetate (3 X 100 mL). The organic phase was dried and concentrated. The residue was purified by column chromatography (silica gel, ethyl acetate). 1.2 g of orange oil was obtained. Yield was 67%. Used as is in the next step.

### Step 3

Above orange oil (0.38g 1.07mmol) was dissolved in 20 mL of 2-propanol. Saturated hydrogen chloride solution in 2-propanol(10 mL) was added in one portion. The solution was kept stirring at 40 °C for 2 hours. After the solvent was removed in vacuum, isopropyl ether was added and it was stirred at room temperature overnight. The solvent was decanted and the solid was dried in vacuum to give the white solid (0.34g, 0.86mmol). Yield was 80. mp 205.8°C (dec.). Anal. Calcd for C₁₅H₂₇ClN₄O₂S₂: C, 45.61; H, 6.89; N, 14.18. Found: C, 45.44; H, 6.89; N 13.93.

### Preparation of compound 57: (4-(4-Fluorophenyl)-1-hydroxyl-2,2,6,6-tetramethylpiperidin-4-ol hydrochloride)

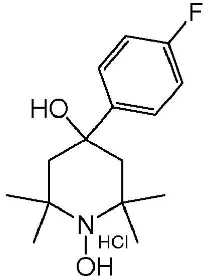

### Step 1

To 4-oxo-2,2,6,6-tetramethylpiperidine-1-oxyl (4.61 g, 27.1 mmol) in dried THF (100 mL) under nitrogen, 4-fluorophenyl magnesium bromide (32.5 mL, 1.0 M solution in THF, 32.5 mmol) was added dropwise. After stirred overnight (red solution) and then heated at 50 °C for 2 hours, the reaction mixture was treated saturated ammonium chloride (50 mL). It was extracted with EtOAc (3X50 mL) and the organic phase was dried over Na₂SO₄ and filtered. The filtrate was concentrated in vacuum to give a red syrup (6.8 g). It was purified by column chromatography (silica gel, Hexane and Hexane:EtOAc (9:1)). 4.4 g of 4-(4-Fluorophenyl)-1-oxyl-2,2,6,6-tetramethylpiperidin-4-ol was obtained. The yield was 61%. Used as is in the next step.

### Step 2

4-(4-Fluorophenyl)-1-oxyl-2,2,6,6-tetramethylpiperidin-4-ol (0.64 g, 2.4 mmol) and saturated hydrogen chloride in 2-propanol (20 mL) were stirred at 40 °C for 2 hour. The solvent was then removed in vacuum. The residual syrup was crystallized in MeOH/diisopropyl ether and provided nice crystal (0.2 g, 0.658 mmol). Yield was 27%. MP: 220 °C (dec.). ¹H NMR (300 MHz, MeOD) δ 7.59-7.54 (2H, m), 7.11-7.05 (2H, m), 2.46 (2H, d, *J*=14.7 Hz), 2.17 (2H, d, *J*=14.9 Hz), 1.77 (6H, s), 1.5 (6H, s). ¹³C NMR (75 MHz, MeOD) δ165.17,145.56,127.97, 127.86, 116.18, 115.89, 72.38, 69.37, 48.72, 29.71, 21.52. Anal. Calcd for C₁₅H₂₃ClFNO₂: C, 59.30; H, 7.63; N, 4.61. Found: C, 59.31; H, 7.75; N, 4.45.

### Preparation of compound 58: ((N-(3,4,5-trimethoxybenzyl)-1-hydroxy-2,2,6,6-tetramethylpiperidin-4-amine dihydrochloride)

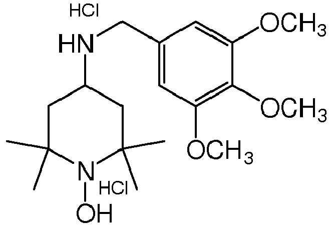

### Step 1

To a solution of 4-amino-2,2,6,6-tetramethylpiperidine-1-oxyl (6.97g, 41 mmol) in dichloromethane (100 mL) was added 3,4,5-trimethoxybenzyl chloride (2.2g, 10 mmol) in five batches. After the mixture was stirred at room temperature overnight, it was poured into water (100 mL). It was extracted with ethyl acetate (3 X 150 mL). The combined ethyl acetate layer was dried and concentrated in vacuum to give a residue, which was purified by column chromatography (silica gel, ethyl acetate). 3g of red oil was obtained. Yield was 85%.

### Step 2

Above red oil (1g, 2.8 mmol) was added to a saturated hydrogen chloride solution in 2-propanol (30 mL). After the mixture was heated at 80 °C for 30 minutes, the solvent was removed in vacuum to give a solid, which was recrystallized in methanol/ether. 0.3g of product was obtained. Yield was 27%. mp 181 °C. ¹H NMR (300 MHz, MeOD) δ 6.96 (s, 2H),4.28(s, 2H), 3.91(s, 6H), 3.85(s, 3H), 3.6-3.5(m, 2H), 2.4-2.2(m, 2H), 1.62(s, 6H), 1.54(s, 6H). □Anal. Calcd for C₁₉H₃₂N₂O₄.2HCl.0.75H₂O: C, 52.00; H, 8.10; N, 6.39. Found: C, 52.02; H, 8.20; N, 6.58.

### Preparation of compound 59: ((4-(4-fluorophenyl)-1-hydroxy-2,5-dihydro-2,2,5,5-tetramethyl-1H-pyrrol-3-yl)methanol hydrochloride)

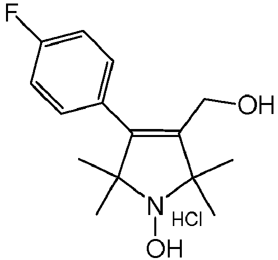

### Step 1

The mixture of (4-bromo-1-oxy-2,5-dihydro-2,2,5,5-tetramethyl-1H-pyrrol-3-yl)methanol (0.7g, 2.8 mmol) (A. V. Chudinov et al, Bull. Acad. Sci. USSR. Div. Chem. Sci., 32(2), 370-375, 1983), dioxane (25 mL), water (6 mL), potassium carbonate (0.42g), 4-fluorophenylboronic acid (0.43g, 3 mmol), PdCl2(Ph3P)2 (0.11g), Pd(Ph3P)4 (0.06g) was refluxed under nitrogen for 3-4 hours. Then dioxane was removed in vacuum. To the residue 20 mL of water and 50 mL of ethyl acetate were added. The organic phase was separated and dried over sodium sulphate. It was concentrated in vacuum and the residue was purified by column chromatography (silica gel, hexane/ethyl acetate 4:1). 0.5g of (4-(4-fluorophenyl)-1-oxy-2,5-dihydro-2,2,5,5-tetramethyl-1H-pyrrol-3-yl)methanol was obtained. The yield was 81%. Used as is in the next step.

### Step 2

To the solution of the (4-(4-fluorophenyl)-1-oxy-2,5-dihydro-2,2,5,5-tetramethyl-1H-pyrrol-3-yl)methanol (0.51g, 2.16 mmol) in 2-propanol (15 mL) was added a saturated solution containing hydrogen chloride in 2-propanol (5 mL) in one portion. The reaction mixture was heated to 40 °C for 0.5 h and then allowed to cool off to room temperature. The solvent was removed in vacuum and 2 mL of diisopropyl ether was added. A white solid (0.45g) was obtained via fiiltration. The yield was 69.0%. MP: 191.7°C (dec.). ¹H NMR (300MHz, MeOD), δ 7.33(d, 2H), 7.25(d, 2H), 4.07(s, 2H), 1.74(s, 6H), 1.62(s, 3H), 1.47(s, 3H). ¹³C NMR (75MHz, MeOD), δ 164.76, 161.48, 139.52, 138.91, 131.14, 127.59, 115.46, 115.17, 77.22, 55.17, 22.51.

### Preparation of Compound 62 (1-Hydroxy-4-(4-Flurophenyl)-2,2,6,6-tetramethylpiperidine hydrochloride)

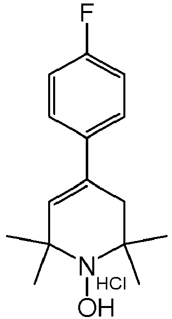

### Step 1

To 4-oxo-2,2,6,6-tetramethylpiperidine-1-oxyl (4.61 g, 27.1 mmol) in dried THF (100 mL) under nitrogen, 4-fluorophenyl magnesium bromide (32.5 mL, 1.0 M solution in THF, 32.5 mmol) was added dropwise. After stirred overnight (red solution) and then heated at 50 ºC for 2 hours, the reaction mixture was treated saturated ammonium chloride (50 mL). It was extracted with EtOAc (3X50 mL) and the organic phase was dried over Na₂SO₄ and filtered. The filtrate was concentrated in vacuum to give a red syrup (6.8 g). It was purified by column chromatography (silica gel, Hexane and Hexane:EtOAc (9:1)). 4.4 g of 4-(4-Fluorophenyl)-1-oxyl-2,2,6,6-tetramethylpiperidin-4-ol was obtained. The yield was 61%. Used as is in the next step.

### Step 2

4-(4-Fluorophenyl)-1-oxyl-2,2,6,6-tetramethylpiperidin-4-ol (0.84g, 3.15mmol) was dissolved in 20 mL of Toluene at room temperature , 5 drops of concentrated sulfuric acid was added. The red solution was heated to reflux overnight. After it was cooled to room temperature the solution was diluted with ether (30 mL) and washed with Na₂CO₃ (10 mL) and brine (10 mL). Solvent was removed and the residue was purified on Prep TLC (hex/EtOAc 85/15). 0.15g of orange solid was obtained. Yield: 19.2%. 0.15g above of orange solid was dissolved in 2-propanol (10 mL). Then hydrogen chloride in ether (2N, 1 mL) was added. The mixture was warmed at 45 °C until the color turned to light yellow. After the solvent was removed in vacuum, A foam was obtained (0.14g). Yield was 13.3%. ¹H NMR (300MHz, CDCl3) δ 1.35 (s, 3H), 1.47 (s, 3H), 1.55 ( 3H, s), 1.62 (3H, s) 2.74(1H, d, J=16.0Hz), 3.12 (1H, d J=16.0Hz), 6.13 (1H, s), 7.23 (2H, t, J=8.8Hz), 7.53 (2H, dd, J= 5.5, 8.5Hz), 11.534(1H, s), 12.512 (1H, s). ¹³C NMR (75MHz, CDCl3) δ 21.182, 22.577, 25.759, 25.972, 26.98, 66.800, 115.651, 115.934, 127.700, 127.808, 129.873, 135.615, 160.735, 163.983. Anal. Calcd for C₁₅H₂₁ClFNO: C, 63.01; H, 7.41; N, 4.90. Found: C, 62.83; H, 7.40; N, 4.80.

### Preparation of Compound 65 (5-Bromo-2-hydroxy-1,1,3,3-tetramethylisoindoline hydrochloride)

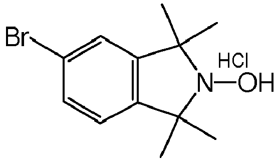

The mixture of 5-bromo-1,1,3,3-tetramethylsioindoline-2-oxyl (A. S. Micallef et al; J. Chem. Soc. Perkin Trans. 2, 1, 65 - 72,1999) (0.5g, 1.9 mmol) in 20 mL of saturated hydrogen chloride in 2-propanol was heated till it became colorless. Then the solvent was removed to give a residue. The residue was dissolved in 2 mL of methanol and ether was added to give a solid, which was washed with ether (3 X 2 mL). It was dried and a white solid of 0.2 grams was obtained. Mp 214.1 °C. Yield was 35 %. ¹H NMR (300 MHz, MeOD) δ 7.67-7.63(m, 2H), 7.41-7.33(m, 1H), 1.96(s, 12H). Anal. Calcd for C₁₂H₁₆NOBr.HCl: C, 47.01; H, 5.58; N, 4.64. Found: C, 47.21; H, 5.58; N, 4.64.

### Preparation of Compound 66 (2-hydroxy-1,1,3,3-tetramethyl-5-morpholinoisoindoline hydrochloride)

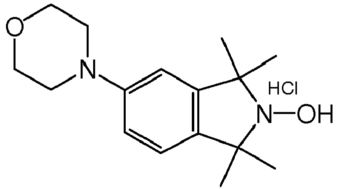

### Step 1

The mixture of 5-bromo-1,1-3,3-tetramethylisoindolin-1-oxy (A. S. Micallef et al, J. Chem. Soc. Perkin Trans. 2, 1,65 - 72(1999)) (1.35g, 5 mmol), morpholine (0.52g, 6 mmol), DMSO (15 mL) and Cesium hydroxide was heated at 120 °C for 30 min. The mixture was poured into water (30 mL). It was extracted with ethyl acetate (3 X 30 mL). The combined organic phase was dried over sodium sulfate and concentrated in vacuum to give a residue, which was purified by column chromatography (silica gel, Hex/EtOAc). 0.35g of orange solid was obtained. Yield was 25%.

### Step 2

0.35g of above orange solid was mixed with saturated hydrogen chloride solution in 2-propanol (20 mL). It was heated at 80 oC for 30 min. It was concentrated in vacuum to give a residue, which was dissolved in methanol (1 mL). To the mixture ether ( 3 mL) was added to give a solid, which was washed with ether (2 X 3 mL). 0.1g of solid was obtained. Yield was 25%. mp 148.9 °C (dec.).
¹H NMR (300 MHz, MeOD) δ 7.77-7.75(m, 2H), 7.62-59(m, 1H), 4.15-4.08(m, 4H), 3.67-3.64(m, 4H), 1.9-1.6(br, 12H). ¹³C NMR(75 Hz, CDCl3) δ 145.38, 141.33, 138.64, 124.22, 121.97, 76.18, 76.00, 64.48, 53.82, 23.81.

### Preparation of Compound 69 (2,2,5,5,-tetramethyl-3-phenyl-pyrrolidine-1-hydroxy hydrochloride)

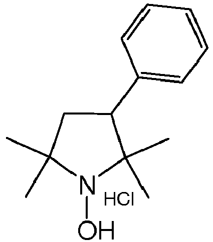

### Step 1

The 5-methyl-5-nitro-4-phenylhexan-2-one (4.70 g, 20 mmol) and NH₄Cl were dissolved in THF/water (3/1, 80ml) and cooled in ice-water. Under vigorous stirring, Zn powder (5.1g, 81 mmol) was added. The reaction mixture was allowed to warm to room temperature and stirred overnight. The solid was filtered off and washed with MeOH (3x5ml). The filtrate was evaporated to ~20 mL and extracted with CH2Cl₂ (3x30 mL) and the combined organic layers dried over MgSO₄. After the solvents were removed, the residue was solidified upon standing. The solid was washed with ether (2x5ml). A pale yellow solid was obtained (3.2g). Yield: 78.8%. M.P.: 84.0-86.9°C.
¹H NMR (300MHz, CDCl3, δ) 0.98 (3H, s), 1.53 (3H, s), 2.15 (3H, t, J=1.53Hz), 2.94 (2H, qd, J=1.54, 8.65), 3.40 (1H, t, J=8.65), 7.22-7.40 (5H, m). ¹³C NMR (75MHz, CDCl3, δ), 13.07, 21.06, 25.87, 35.22, 49.51, 76.16, 127.58, 128.32, 128.58, 127.88, 140.55. Anal. Calcd. for C₁₃H₁₇NO. 0.2H₂O: C, 75.47; H, 8.48; N, 6.77. Found: C, 75.17; H, 8.50; N, 6.76.

### Step 2

A solution of above pale yellow oil, 2,5,5-trimethyl-4-phenyl- 3,4-dihydropyrrole 1-oxide, (2.56g, 12.3 mmol) in 30 mL of THF was added slowly to 10 mL of MeMgCl in THF (3N, 30 mmol) at room temperature. The reaction was stirred at room temperature for 2hrs. Another 3 mL of MeMgCl was added and the reaction mixture was stirred overnight at room temperature. TLC (MeOH/EtOAc 1/9) indicated only small amounts of starting material left. The mixture was diluted with ether (50 mL) and quenched with NH₄Cl (30 mL). The organic solution was washed with brine (2x15 mL). Solvent was removed in vacuum and the residue was taken up in CHCl₃ (30 mL). The yellow solution was well stirred over MgSO₄ and PbO₂ (0.7g) for 2hrs. The mixture was filtered through silica gel (30g) and eluted with EtOAc/hec (1/4, 100 mL) to give an orange crystal (1.57g, 58.5. Above orange solid (0.3g) was dissolved in 2-propanol and hydrogen chloride in ether (1mL, 2N) was added. The clear orange solution was warmed at 45 °C until the color disappeared. Solvent was removed and foam was obtained (0.25g). Yield was 71.1%. ¹H NMR (300MHz, DMSO) δ 1.00(3H, s), 1.39 (3H, s), 1.47 (3H, s), 1.62 (3H, s), 2.10 (1H, dd J=13.3, 6.7Hz), 2.70 (1H, t, J=14.4Hz), 3.36 (1H, dd, J=14.0, 7.0 Hz), 7.36 (5H, m), 11.54 (1H, s), 11.96 (1H, s). ¹³C NMR (75MHz, DMSO) δ 15.88, 23.00, 25.69, 27.93, 47.97, 64.74, 70.95, 74.90, 128.24, 128.50, 128.87, 129.21, 135.66. Anal. Calcd for C₁₄H₂₂ClNO.0.1H2O: C, 65.28; H, 8.69; N 5.44. Found: C, 65.12; H, 8.71; N, 5.32.

### Preparation of Compound 71 (4-(4-ethoxycarbonylpiperidine-1-yl)-3-(1-hydroxyl-2,2,5,5,-tetramethyl-piperidine-4-oxy)-1,2,5-thiadiazole hydrochloride)

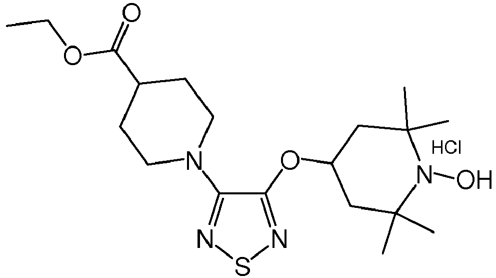

### Step 1

4-[4-ethoxycarbonyl-piperidin-1-yl)]-3-chloro-1,2,5-thiadiazole was synthesized according to the proceduredescribed in the "General procedure B." NMR ¹H NMR (300MHz, CDCl₃, δ), 1.29 (3H, t, J=7.1Hz), 1.94 (2H, m), 2.03 (2H, m), 2.53 (1H, m), 3.02 (2H, m), 3.96 (2H, m), 4.18 (2H, q, J=7.1Hz). 13C NMR (75MHz, CDCl3, δ), 14.23, 27.67, 40.70, 48.61, 60.59, 135.66, 159.46, 174.40.

### Step 2

To a solution of 4-hydroxy-2,2,6,6-tetramethylpiperidine-1-oxyl (3.02g, 17.7mmol) and t-BuOK (2.6g, 23.2mmol) in t-BuOH (60 mL) was added 3-(4-ethoxycarbonylpiperidine-1-yl)-4-chlorothiadiazole (4.12g, 15mmol). The dark solution was stirred at room temperature over weekend. The reaction was monitored by TLC (Hex/EtOAc 1/9). Water (10 mL) was added and the mixture was stirred for another 30 min. The mixture was extracted with CH₂Cl₂ (3x20 mL). The organic layers were combined, dried over MgSO₄ and evaporated. The residue was separated by column chromatography (silica gel, Hex/EtOAc (9/1)). The fourth spot was assumed as the expected nitroxide (0.96g). Yield was 15.8%. Used as is in the next step.

0.3g of the above nitroxide was dissolved in 20 mL of 2-propanol at 50 °C. Hydrogen chloride in 2-propanol was added until the solution became light yellow. The solvent was removed and the residue was dissolved in dichloromethane. The solvent was removed and a foam was obtained (0.25g). Yield: 76.4%. Anal. Calcd. C₁₉H₃₃ClN₄O₄S.0.5H₂O: C, 49.82; H, 7.48; 12.23. Found: C, 49.72; H, 7.35; N, 12.04.

### Preparation of Compound 72 (4-(4-(4-Fluro-Phenyl)piperazin-1-yl)-3-(1-hydroxyl-2,2,5,5,-tetramethyl-piperidine-4-oxy)1,2,5-thiadiazole hydrochloride)

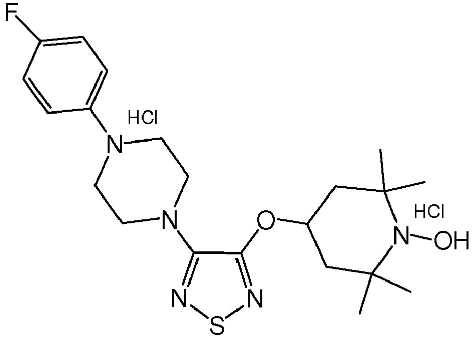

### Step 1

4-[4-(4-fluro-phenyl)-piperazin-1-yl)]-3-chloro-1,2,5-thiadiazole was synthesized according to the proceduredescribed in the "General procedure B." NMR 1H NMR (300MHz, CDCl3, δ) 3.27 (4H, t, J=5.0Hz), 3.67 (4H, t, J=5.0), 6.99 (4H, m). 13C NMR (75MHz, CDCl3, δ), 48.91, 50.01, 115.83, 115.53, 118.30, 118.40, 135.40, 147.73, 147.76, 155.96, 159.01, 159.14.

### Step 2

To a solution of 4-hydroxy-2,2,6,6-tetramethylpiperidine-1-oxyl (2.02g, 11.7 mmol) and t-BuOK (1.7g, 15.2 mmol) in t-BuOH (30 mL) was added 3-(4-N-(4-fluorophenylpiperazine-1-yl)-4-chlorothiadiazole (2.98g, 10 mmol). The dark solution was stirred at room temperature over weekend. 20 mL of THF was added in order to dissolve the amine. The reaction was monitored by TLC (Hex/EtOAc 8/1). Water (10 mL) was added and the mixture was stirred for another 30 min. The precipitate was collected and washed with water (2x 10 mL), t-butanol (8 mL) and hexane (2x8 mL). The red solid was dried in air (2.60g). The yield was 59.9%. Above red solid (0.5g) was suspended in 20 mL of 2-propanol at 40 °C. Hydrogen chloride in ether (2N, 3 mL) was added and warmed at 46 °C until the solution became light yellow. The solvent was removed in vacuum to almost dryness and acetone (5 mL) was added. The solution was diluted with EtOAc (20 mL) and stood for precipitation. An off white precipitate was collected and washed with acetone (2x1mL). The solid was dried in oven (0.45g). Yield was 82.8%. mp 89.4°C (dec.). ¹H NMR (300MHz, DMSO) δ 1.41(6H, s), 1.55 (6H, s),2.34 (2H, m), 2.47 (2H, m), 3.42 (4H, m), 3.76 (4H, m), 5.32 (1H, m), 7.22 (2H, m), 7.42 (2H, m), 11.56 (1H, s), 12.61 (1H, s). ¹³C NMR (75MHz, DMSO) δ 20.76, 27.64, 40.56 (in DMSO), 46.66, 51.03, 67.89, 71.56, 116.23, 116.53, 120.51, 120.55, 150.15, 152.84. Anal. Calcd for C₂₁H₃₂Cl₂FN₅O₂S.1.5H₂O: C, 47.10; H, 6.59; N, 13.08. Found: C, 46.92; H, 6.62; N 12.77.

### Preparation of Compound 73 (4-O-nitro-1-hydroxy-2,2,6,6- tetramethylpiperidine hydrochloride)

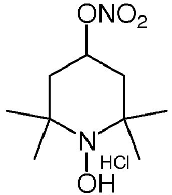

### Step 1

4-hydroxy-2,2,6,6-tetramethylpiperidine-1-oxyl (2.02g, 11.7mmol) was added to 10 mL of concentrated sulfuric acid. The yellow mixture was cooled in an ice water bath (3 °C). Nitric acid (10 mL) was added slowly in 20min. After addition, the mixture was stirred at room temperature for 20min and cooled in the ice water bath again. The mixture was poured into a mixture of crushed ice (100g) and dichloromethane (40 mL). The organic was separated and the aqueous was extracted with dichloromethane (3x15 mL). The combined organic layers were dried over MgS04 and evaporated. The residue was run through a quick column (silica gel, CH₂Cl₂/Pet. Ether (1/1, 500 mL)). 0.81g of red solid was obtained. Yield was 32.4%.

### Step 2

0.61g of above red solid was converted to hydrogen chloride salt by dissolving it in 10 mL of 2-propanol and 2 mL of hydrogen chloride in ether (2N) and warming at 40 °C for 1hrs. The colorless solution was concentrated and EtOAc was added (5 mL). The white crystal was collected, washed with EtOAc (2x2 mL), hexane (2 mL) and dried in oven (0.35g). Yield was 48.8%. mp 168.7-175.3°C(dec).
¹H NMR (300MHz, DMSO-d6) δ 1.39(6H, s), 1.51 (6H, s), 2.29 (4H, m), 5.52 (1H, m), 11.61 (1H, s), 12.52 (1H, s). ¹³C NMR (75MHz, DMSO-d6) δ 20.52, 27.63, 38.58, 67.50, 74.89. Anal. Calcd for C₉H₁₉ClN₂O₄: C, 42.44; H, 7.52; N, 11.00. Found: C, 42.56; H, 7.73; N, 10.81.

### Preparation of Compound 74 (1-Hydroxy-4-(3-hydroxy-4-methoxybenzyl)-2,2,6,6,-tetramethylpiperidine hydrochloride)

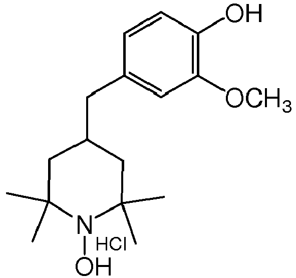

### Step 1

3-benzyloxy4-methoxybenzyl triphosphonium chloride (2.63g, 5 mmol) was suspended in dry THF (30 mL). At room temperature , BuLi (2.5M, hex, 3mL) was added dropwise during a period of 15min. The red solution was stirred for 30 min. until all solid was disappeared. 4-oxo-2,2,6,6-tetramethylpiperidine-1-oxyl (1.28g, 7.5mmol) was added at once. The clear red solution was heated reflux for 4hr. The reaction mixture was diluted with pet ether (20mL) and run through 50g of silica gel eluted with CH2Cl2 (500 mL). The solvent was evaporated. The residue was loaded on Prep. TLC eluted with Hex/EtOAc (15/85). 1.73g of 3-benzyloxy-4-methoxybenzylidene 2,2,6,6-tetramethylpiperidine-1-oxyl was obtained. The yield was 42%. Used as is in the next step.

### Step 2

3-benzyloxy-4-methoxybenzylidene 2,2,6,6-tetramethylpiperidine-1-oxyl (0.21g, 0.55mmol) was dissolved in 10 mL of 2-propanol. Pd/C (60mg, 10%) was added. The mixture was evacuated and subjected to hydrogenation with a balloon. TLC indicated the disappearance of starting material. Catalyst was filtered off through celite and the solid washed with acetone. Hydrogen chloride in ether (2N, 2 mL) was added and the solvents were removed. The residue was dissolved in CH₂Cl₂ and hexane was added until the solution became cloudy. Solvent was removed and foam was obtained (0.12g). Yield was 75%. ¹H NMR (300MHz, DMSO-d6) δ 1.36 (s, 6H), 1.65 (s, 6H), 1.73 (2H, m), 1.95(2H, m), 2.04(1H, m), 2.51(2H, d, J=4.5Hz), 3.90 (3H, s), 5.65 (1H, s), 6.62 (1H, m), 6.77 (2H, m), 10.49 (1H, s), 11.25 (1H, s). Anal. Calcd for C₁₇H₂₇ClNO₃: C, 61.90; H, 8.56; N, 4.25. Found: C, 61.99; H, 8.59; N, 4.07.

### Preparation of Compound 77 ((E)-3-(4-hydroxy-3-methoxyphenyl)-N-(1-hydroxyl-2,2,6,6-tetramethyl-piperidin-4-yl)acrylamide hydrochloride)

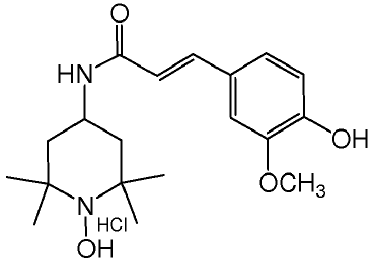

### Step 1

A mixture of dark brown liquid 4- hydroxy -2,2,6,6-tetramethylpiperidine-1-oxyl (2.57 g, 15 mmol), 4-dimethylaminopyridine (DMAP) (0.59 g, 4.8 mmol) and trans-4-acetoxy-3-methoxy-cinnamic acid (3.54 g, 15 mmol), dichloromethane (200 mL) was stirred at room temperature for one hour to allow most of the solid dissolved. N-(3-Dimethylaminopropyl)-N'-ethylcarbodiimide hydrochloride (EDAC) (2.45 g, 25.5 mmol) was then added in five minutes and allowed the mixture stirred for another half an hour. More solid was dissolved and another portion of EDAC (2.45 g, 25.5 mmol) was added. One more hour later, the solvent was evaporated. The residual was redissolved in EtOAc (200 mL), washed with NH₄Cl (three times) and NaHCO₃ (three times), and dried over Na₂SO₄. The crude product was isolated by column chromatography (silica gel, EtOAc/Hexane (1:9, 1:1)). A crude product (5.6 g), (E)-3-(4-acetoxy-3-methoxy-phenyl)-N-(1-oxyl-2,2,6,6-tetramethyl-piperidin-4-yl)acrylamide, was obtained. The yield was 96%. Used as is in the next step.

### Step 2

To a solution of (E)-3-(4-acetoxy-3-methoxy-phenyl)-N-(1-oxyl-2,2,6,6-tetramethyl-piperidin-4-yl)acrylamide (0.2 g, 0.514 mmol) in MeOH (2 mL), aqueous HCl (37%) (1 mL) was added. After the solution was kept in water bath (40 °C) for 1.5 hour, the solvent was evaporated and the crude product was dried in vacuum. The syrup was then loaded on TLC plates and run in methylene chloride : methanol (9:1). The product band was collected and washed with methylene chloride : methanol (9:1). The condensed product was redissolved in MeOH (1 mL), added hydrogen chloride/ether (1 mL) and blown dry, which afforded pure product (110 mg). Yield was 56%. mp 190 ºC (dec.). ¹H NMR (300 MHz , MeOD-d₄): δ7.55
(1H, d, *J*=15. Hz), 7.18 (1H, s), 7.08 (1H, *J*=7.85 Hz), 6.84 (1H, *J*=7.88 Hz), 6.55 (1H, *J*=15.4 Hz), 4.46 (1H, b), 3.9 (3H, s), 2.23-2.08 (4H, m), 1.55-1.52 (12H, d). ¹³C NMR (75 MHz, MeOD-d₄): δ169.24, 150.5, 149.41, 143.93, 127.86, 123.96, 117.16, 116.64, 111.83, 69.9, 56.73, 42.77, 41.33, 28.34, 20.63.

### Preparation of Compound 91 (4-(4-(2-Fluro-Phenyl)piperazin-1-yl)-3-(1-hydroxyl-2,2,5,5,-tetramethyl-piperidine-4-oxy)1,2,5-thiadiazole Hydrochloride)

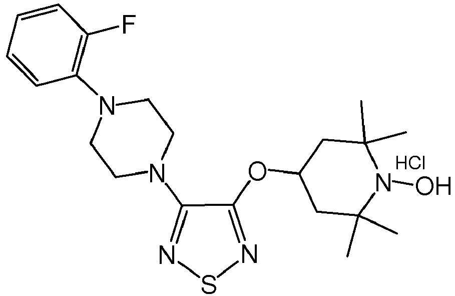

### Step 1

4-[4-(2-fluro-phenyl)-piperazin-1-yl)]-3-chloro-1,2,5-thiadiazole was synthesized according to the procedure described in the "General procedure B." NMR . 1H NMR (300MHz, CDCl3, δ) 3.26 (4H, t, J=5.0Hz), 3.69 (4H, t, J=5.0), 7.04 (4H, m). 13C NMR (75MHz, CDCl3, δ), 49.01, 50.16, 116.13, 116.41, 119.12, 119.16, 122.93, 123.04, 124.52, 124.56, 135.35, 139.70, 139.82, 154.18, 157.44, 159.08.

### Step 2

To a solution of 4-hydroxy-2,2,6,6-tetramethylpiperidine-1-oxyl (3.02g, 17.5mmol) and t-BuOK (2.2g, 22.2mmol) in t-BuOH (45 mL) was added 3-(4-N-(4-Fluorophenyl piperazine-1-yl)-4-chlorothiadiazole (3.5g, 12mmol). The dark solution was stirred at room temperature over weekend. 20 mL of THF was added in order to dissolve the amine. The reaction was monitored by TLC (Hex/EtOAc 8/1). Water (10 mL) was added and the mixture was stirred for another 30 min. The precipitate was collected and washed with water (2x 10 mL), t-butanol (8 mL) and hexane (2x8 mL). The red colored solid was dried in air (2.50g) The yield was 57.6%. Used as is in the next step.

0.5g of the above red solid was suspended in 20 mL of 2-propanol at 40°C. Hydrogen chloride in ether (2N, 3 mL) was added and warmed at 46°C until the solution became light yellow. The solvent was removed to almost dryness and acetone was added (10 mL). The off white precipitate was collected and washed with acetone (2x1mL). The solid was dried in oven. 0.50g of product was obtained. Yield: 85.5%. mp 199.0 (dec.).
¹H NMR (300MHz, DMSO-d6) δ 1.41(6H, s), 1.51 (6H, s),2.24 (2H, m), 2.47 (2H, m in DMSO), 3.13 (4H, m), 3.60 (4H, m), 5.31 (1H, m), 6.99 (1H, m), 7.11 (3H, m), 11.56 (1H, s), 12.27 (1H, s).
Anal. Calcd for C₂₁H₃₂Cl₂FN₅O₂S 0.5H2O: C, 52.43; H, 6.71; N, 14.56. Found: C, 52.55; H, 6.85; N, 14.60.

### Preparation of Compound 92 (1-(4-chloro-1,2,5-thiadiazol-3-1)-N-(1-hydroxy-2,2,6,6-tetramethylpiperidin-4-yl)piperidine-4-carboxamide hydrochloride)

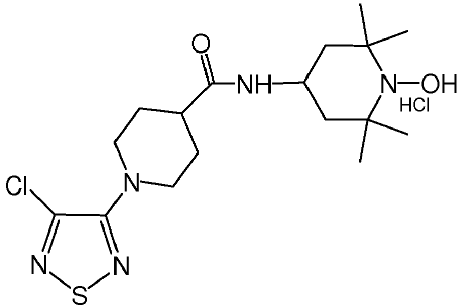

4-Amino-2,2,6,6-tetramethylpiperidine-1-oxyl (0.80g, 4.67mmol) was dissolved in Dry CH₂Cl₂ (20 mL). At room temperature 1-(4-chloro-1,2,5-thiadiazol-3-yl)piperidine-4-carboxylic acid (0.72g, 3mmol ) was added followed by 4-dimethylaminopyridine (DMAP) (0.07g, cat. 5. The mixture was stirred another 20 min at room temperature DCC (0.85, 4.2 mmol ) was added dropwise in once. After addition, the mixture was stirred 3hrs at room temperature TLC indicated the disappearance of acid. (EtOAc/Hex1:1). Water (2 mL) was added. The mixture was stirred for 30 min. The precipitate was filtered off and washed with CH₂Cl₂ (3x5 mL). The combined organic solution was washed with HCl (1N, 2x5 mL), brine (2x5 mL), Na₂CO₃ (sat. 5 mL), brine (2x5 mL) and dried over MgSO₄. Solvent was removed and the residue was purified (silica gel, CH₂Cl₂/EtOAc (8/2, 1000 mL)). 0.96g of pink solid was obtained. The yield was 80.0%. Used as is in the next step.

0.3g of above pink solid was suspended in 2-PrOH (15 mL). Hydrogen chloride in ether (2N, 2 mL) was added. The solution was heated at 40°C until the brown color turned light yellow. 0.24g of solid was obtained. Yield was 73.2%. mp 213.5 (dec.). 1H NMR (300MHz, DMSO-d6) δ 1.34(6H, s), 1.45 (6H, s), 1.75 ( 4H, m), 1.95 (4H, m), 2.36 (1H, m), 2.92 (2H, dt, J=3.6, 11.3Hz), 3.92 (2H, m), 4.11 (1H, m), 8.08, 8.09 (1H, s,s), 11.35 (1H, s), 12.13 (1H, s). 13C NMR (75MHz, DMSO-d6) δ 20.42, 27.66, 28.25, 39.11, 41.50, 41.60, 48.82, 67.78, 135.36, 159.71, 174.00. Anal. Calcd for C₁₇H₂₉Cl₂N₅O₂S: C, 46.57; H, 6.67; N, 15.97. Found: C, 46.28; H, 6.63; N, 15.93.

### Preparation of Compound 93 (1-(4-(2,2,6,6-tetramethylpiperidin-4-yloxy)-1,2,5-thiadiazol-3-yl)piperidine-4-carboxylic acid hydrochloride)

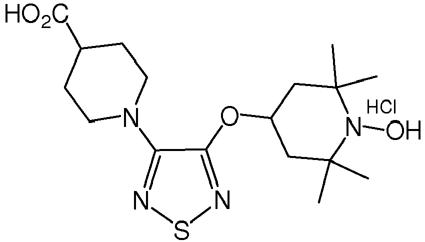

### Step 1

4-[4-ethoxycarbonyl-piperidin-1-yl)]-3-chloro-1,2,5-thiadiazole was synthesized according to the procedure described in the "General procedure B"
NMR 1H NMR (300MHz, CDCl3, δ), 1.29 (3H, t, J=7.1Hz), 1.94 (2H, m), 2.03 (2H, m), 2.53 (1H, m), 3.02 (2H, m), 3.96 (2H, m), 4.18 (2H, q, J=7.1Hz). 13C NMR (75MHz, CDCl3, δ), 14.23, 27.67, 40.70, 48.61, 60.59, 135.66, 159.46, 174.40.

### Step 2

To a solution of 4-hydroxy-2,2,6,6-tetramethylpiperidine-1-oxyl (3.02g, 17.7 mmol) and t-BuOK (2.6g, 23.2 mmol) in t-BuOH (60 mL) was added 3-(4-ethoxycarbonyllpiperidine-1-yl)-4-chlorothiadiazole (4.12g, 15 mmol). The dark solution was stirred at room temperature over weekend. The reaction was monitored by TLC (Hex/EtOAc 1/9). Water (10 mL) was added and the mixture was stirred for another 30 min. It was extracted with CH₂Cl₂ (3x20 mL). The organic phase was dried over MgSO₄ and evaporated. The residue was separated by column chromatography (silica gel, Hex/EtOAc (9/1)). The fourth spot was assumed as product (0.96g). Yield was 15.8%. The 4^{th} spot from (0.51g, 1.23mmol) was dissolved in 15 mL of ethanol and 1 mL of water. NaOH (0.32g, 8mmol) was added. The mixture was heated at 40°C until the starting material disappeared by TLC (1hr, Hex/EtOAc, 4/1). Prep. TLC was used to separate product (developed with EtOAc 2^{nd} spot). 0.47g of product was obtained. The yield was 65.9%.

### Step 3

0.3g of the above product was dissolved in 20 mL of 2-propanol at 50°C. Saturated hydrogen chloride in 2-propanol was added until the solution became light yellow. The solvent was removed and the residue was suspended in CH₂Cl₂. The solid was collected and washed with CH₂Cl₂ (2x3 mL), hexane (2x3 mL) and dried in air.0.14g of 1-(4-(2,2,6,6-tetramethylpiperidin-4-yloxy)-1,2,5-thiadiazol-3-yl)piperidine-4-carboxylic acid hydrochloride was obtained. Yield was 41.2%. mp 181.5°C (dec.).
¹H NMR (300MHz, DMSO-d6) δ 1.40(6H, s), 1.51 (6H, s), 1.59 (2H, m), 1.88 (2H, m), 2.25 (2H, m), 2.44 (2, m), 2.98 (2H, m), 3.96 (2H, m), 5.29 (1H, m), 11.53 (1H, s), 12.34 (1H, s). ¹³C NMR (75MHz, DMSO-d6) δ 20.69, 22.02, 27.67, 27.81, 47.23, 65.38, 67.79, 71.27, 150.67, 152.78, 176.11. Anal. Calcd for C₁₇H₂₉ClN₄O₄S: C, 48.50; H, 6.94; N, 13.31. Found: C, 48.45; H, 7.15; N, 13.03.

### Preparation of Compound 94 (1,4-bis(1-hydroxy-2,26,6-tetramethylpiperidin-4-yloxy)-1,2,5-thiadiazol-3-yl)piperazine hydrochloride)

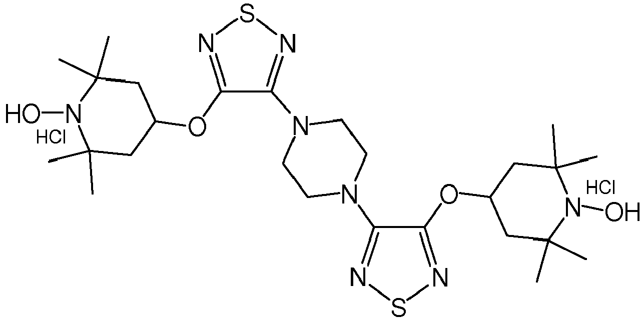

### Step 1

1,4-bis-(3-chloro-1,2,5-thiadiazol-4-yl) piperazine was synthesized according to the proceduredescribed in the "General procedure B"
NMR 1H NMR (300MHz, CDCl3, δ)3.67 (s). 13C NMR (75MHz, CDCl3, δ), 48.39, 135.46, 158.87.

### Step 2

To a solution of 4-hydroxy-2,2,6,6-tetramethylpiperidine-1-oxyl (5.25g, 30 mmol) and t-BuOK (4.24g, 34 mmol) in t-BuOH (100 mL) was added 1,4-bis(4-chloro-1,2,5-thiadiazol-3-yl)piperidine (3.23g, 10 mmol). THF (10 mL) was added to dissolve the starting material. The dark solution was stirred at room temperature over weekend. The reaction was monitored by TLC (Hex/EtOAc 4/1). Water (50 mL) was added and the mixture was stirred for another 30 min. The precipitate was collected and washed with water (2x 10 mL), t-butanol (8 mL) and hexane (2x8 mL). The red colored solid was purified (silica gel, dichloromethane (1.5L)). 0.5g of red solid was obtained. Above red solid (0.5g) was suspended in 2-propanol (30 mL) and was added hydrogen chloride solution in ether (2N, 3 mL). The mixture was heated at 60°C until the clear solution sustained. Solvent was removed as much as possible. The residue was collected and rinsed with dichloromethane. The solid was washed with dichloromethane, acetone and dried in air (0.4g). Yield was 71.0%. mp 215.2°C (dec.). ¹H NMR (300MHz, CD30D) δ 1.58, 1.59(24H, s,s), 2.17 (4H, m), 2.66 (4H, m), 3.62(8H, m), 5.46 (2H, m). ¹³C NMR (75MHz, CD30D) δ 19.22, 23.85, 26.97, 63.35, 68.76, 69.95, 149.97, 152.31. Anal. Calcd for C₂₆H₄₆Cl₂N₈O₄S₂: C, 46.63; H, 6.92; N, 16.73. Found: C, 46.86; H, 7.10; N, 16.26.

### Preparation of Compound 95 (Tert-butyl 4-(1-hydroxy-2,2,6,6-tetramethylpiperidin-4-yloxy)-1,2,5-thiadiazole-3-carboxylate hydrochloride)

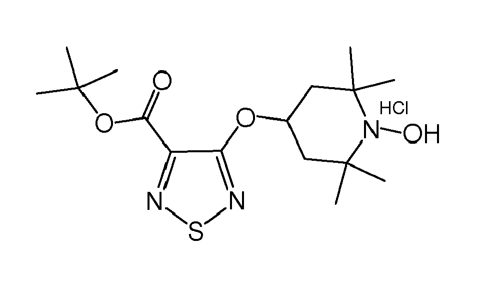

4-hydroxy-2,2,6,6-tetramethylpiperidine-1-oxyl (2.1g, 12 mmol) was dissolved in 30 mL of t-BuOH. At room temperature t-BuOK (1.5g, 12.3 mmol) was added. The mixture was stirred for 1hr or until all dissolved. 4-Ethoxycarbonyl-3-chloro-1,2,5-thiadiazole (1.92g, 10 mmol) was added. The solution became cloudy immediately. The mixture was then stirred overnight. TLC indicated two new spots between both starting materials. Water (10 mL) was added. The mixture was extracted with dichloromethane (4x15 mL). The combined organic layer was dried and evaporated. The residue was purified (silica gel, Hex/EtOAc (90/10)). Two pure compounds were isolated, spot one (0.88g) and spot two (0.34g). Also a mixture (0.4g) of spot one and spot two was obtained. The first spot (0.4g) was dissolved in CH₂Cl₂ (15 mL) and hydrogen chloride in ether (2N, 3 ml) was added. The mixture was warmed in water bath (40 °C). 2-propanol (1 mL) was added and the color disappeared in 10 min. Solvent was removed and the residue was dissolved in acetone (5 mL). The light yellow solution was stood for crystallization. The collected solid was washed with acetone (2x2 mL), hexanes (2x2 mL) and dried in oven. 0. 38g of solid was obtained. Yield was 86.2%. mp 197.0°C (dec.). ¹H NMR (300MHz, DMSO-d6) δ 1.40, 1.52, 1.53(21H, s,s,s), 2.29 (2H, m), 2.43 (2H, m), 5.30 (1H, m), 11.52, 11.59 (1H, s,s), 12.34, 12.38 (1H, s,s). ¹³C NMR (75MHz, DMSO-d6) δ 20.72, 21.46, 27.78, 28.18, 38.22, 67.71, 71.60, 83.20, 140.51, 157.89, 163.66. Anal. Calcd for C₁₆H₂₈ClN₃O₄S: C, 48.78; H, 7.16; N, 10.67. Found: C, 48.98; H, 7.28; N, 10.77.

### Preparation of Compound 103 (4-(1-hydroxy-2,2,6,6-tetrmethylpiperidin-4-yloxy)-1,2,5-thiadiazole-3-carboxylic acid hydrochloride)

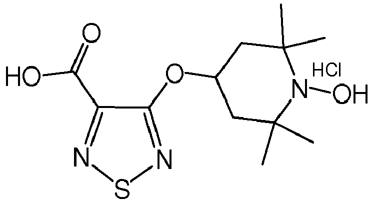

### Step 1

4-ethoxycarbonyl-3-chloro-1,2,5-thiadiazole was synthesized according to the proceduredescribed in the "General procedure A." 1H NMR (300MHz, CDCl3, δ), cont with DMF 1.47 (3H, d, J-7.1Hz), 4.51 (2H, d, J-7.1Hz). 13C NMR (75MHz, CDCl3, δ), 14.11, 62.77, 147.31, 148.63, 158.50.

### Step 2

4-hydroxy 2,2,6,6-tetramethylpiperidine-1-oxyl (2.1g, 12 mmol) was dissolved in 30 mL of t-BuOH. At room temperature, t-BuOK (1.5g, 12.3 mmol) was added. The mixture was stirred for 1hr or until all dissolved. 4-ethoxycarbonyl-3-chloro-1,2,5-thiadiazole (1.92g, 10 mmol) was added. The solution became cloudy immediately. The mixture was then stirred overnight. TLC indicated two new spots between both starting materials. Water (10 mL) was added. The mixture was extracted with CH₂Cl₂ (4x15 mL). The organic phase was dried and evaporated. The residue was purified (silica gel, Hex/EtOAc (90/10)). The first spot was collected in 2L (0.88g,) followed by a mixture of 2 spot (0.4g) and the second spot was then collected (0.34g). Above mixture of spot one and spot two (0.4g) was hydrolyzed with NaOH in methanol and converted to HCl salt (0.25g). mp 209.1°C (dec.). Yield was 74.1%
¹H NMR (300MHz, DMSO-d6) δ 1.42 (6H, s), 1.52(6H, s), 2.89 (2H, m), 2.43 (2H, m), 5.31 (1H, m), 11.50 (1H, s ), 12.29 (1H, s ). ¹³C NMR (75MHz, DMSO-d6) δ 20.75, 27.73, 67.79, 71.60, 140.74, 160.28, 163.69. Anal. Calcd for C₁₂H₁₉N₃O₄S.0.9HCl: C, 43.13; H, 6.00; N, 12.57. Found: C, 43.10: H, 6.03; N, 12.33.

### Preparation of Compound 104 (Ethyl 4-(1-hydroxy-2,2,6,6-tetramethylpiperidin-4-yloxy)-1,2,5-thiadiazole-3-carboxylate Hydrochloride)

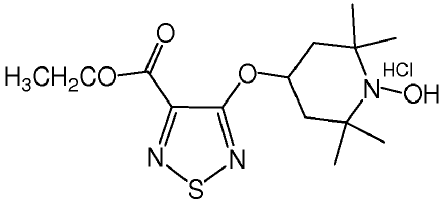

4-hydroxy 2,2,6,6-tetramethylpiperidine-1-oxyl (2.1g, 12 mmol) was dissolved in 30 mL of t-BuOH. At room temperature, t-BuOK (1.5g, 12.3 mmol) was added. The mixture was stirred for 1hr or until all dissolved. 4-ethoxycarbonyl-3-chloro-1,2,5-thiadiazole (example 103, step 1) (1.92g, 10 mmol) was added. The solution became cloudy immediately. The mixture was then stirred overnight. TLC indicated two new spots between both starting materials. Water (10 mL) was added. The mixture was extracted with CH₂Cl₂ (4x15 mL). The organic phase was dried and evaporated. The residue was purified (silica gel, Hex/EtOAc (90/10)). The first spot was collected in 2L (0.88g,) followed by a mixture of 2 spot (0.4g) and the second spot was then collected (0.34g). The second spot was converted to HCl salt (0.20g). mp 183.7°C (dec.). Yield was 86.2%.
Anal. Calcd for C₁₂H₂₄ClN₃O₄S: C, 46.09; H, 6.35; N, 11.52. Found: C, 46.15; H, 6.64; N, 11.61.

### Preparation of Compound 105 (4-(4-(1-hydroxy-2,2,6,6-tetramethylpiperidin-4-yloxy)-1,2,5-thiadiazol-3-yl)piperazin-1-yl)(furan-2-yl)methanone hydrochloride)

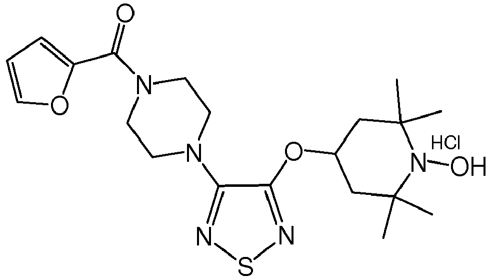

4-hydroxy-2,2,6,6-tetramethylpiperidine-1-oxyl (2.58g, 15 mmol) was dissolved in 40 mL of t-BuOH. At room temperature t-BuOK (2.64g, 22 mmol) was added. The mixture was stirred for 1hr or until all dissolved. (4-(4-chloro-1,2,5-thiadiazol-3-yl)piperazin-1-yl)furan-2-yl)methanone (2.99g, 10 mmol) was added and 30 mL of THF to dissolve the starting material. The mixture was then stirred overnight. Water (15 mL) was added. The mixture was evaporated to remove THF and most of t-BuOH. The aqueous was extracted with CH₂Cl₂ (4x15 mL). The combined organic layer was dried and concentrated to give a residue, which was purified by column chromatography (silica gel, CH₂Cl₂/MeOH/ (99/1, 3L). 1.2g of red solid was obtained. Yield was 50.8%. 0.8g of above red solid was converted to HCl salt by dissolving it in CH₂Cl₂ (25 mL) with 2 mL of i-PrOH, followed by adding hydrogen chloride in ether (2N, 3 mL). The mixture was heated at 40°C till it became colorless. Then the solvents were removed in vacuum and a foam was obtained (0.9g). Yield was100%. mp 198.1°C (dec.). Anal. Calcd for C₂₀H₃₀ClN₅O₄S: C, 50.89; H, 6.41; N, 14.84. Found: C, 50.88; H, 6.50; N, 14.48.

### Preparation of Compound 106 (1-(4-(1-hydroxy-2,2,6,6-tetramethylpiperidin-4-yloxy)-1,2,5-thiadiazol-3-yl)-4-(pyridine-2-yl)piperazine hydrochloride)

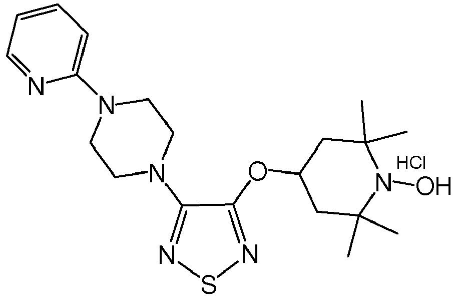

4-hydroxy-2,2,6,6-tetramethylpiperidine-1-oxyl (3.42g, 20 mmol) was dissolved in 40 mL of t-BuOH. At room temperature t-BuOK (2.64g, 22 mmol) was added. The mixture was stirred for 1hr or until all dissolved. 1-(4-chloro-1,2,5-thiadiazol-3-yl)-4-(pyridine-2-yl)piperazine (3.41g, 12.1 mmol) was added and 30 mL of THF to dissolve the starting material. The mixture was then stirred overnight. Water (15 mL) was added. The mixture was evaporated to remove THF and most of t-BuOH. A red solid was collected and washed with water. The solid was then purified by column chromatography (silica gel, EtOAc/Hex (15/85, 3L)). 2.56g of solid was obtained. Yield was 50.8%. 1.3g of above solid was converted to HCl salt by dissolving it in CH₂Cl₂ (25 mL) with 2 mL of i-PrOH, followed by adding hydrogen chloride in ether (2N, 3 mL) and warming the mixture at 40°C. The solvents were removed and a foam was obtained (1.5g). Yield was 96.3%. Anal. Calcd for C₂₀H₃₂Cl₂N₆O₂S.0.5H₂O: C, 48.00; H, 6.65; N, 16.79. Found: C, 48.97; H, 6.87; N, 15.49

### Preparation of Compound 107 (2-(4-(4-(1-hydroxy-2,2,6,6-tetramethylpiperidin-4-yloxy)-1,2,5-thiadiazol-3-yl)piperazin-1-yl)pyrimidine hydrochloride)

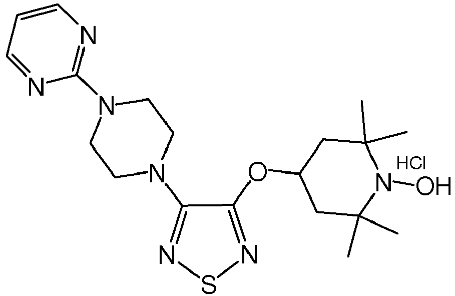

4-hydroxy-2,2,6,6-tetramethylpiperidine-1-oxyl (1.72g, 10 mmol) was dissolved in 30 mL of t-BuOH. At room temperature t-BuOK (1.35g, 11 mmol) was added. The mixture was stirred for 1hr or until all dissolved. 2-(4-(4-chloro-1,2,5-thiadiazol-3-yl)piperazin-1-yl)pyrimidine (1.02g, 3.6 mmol) was added and 10 mL of THF to dissolve the starting material. The mixture was then stirred overnight. Water (10 mL) was added. The mixture was evaporated to remove THF and most of t-BuOH. A red solid was collected and washed with water. The solid was then purified by column chromatography (silica gel, EtOAc/Hex (15/85, 3L)). 0.85g of red solid was obtained. Yield was 56.3%.
0.5g of above red solid was converted to HCl salt by dissolving it in CH₂Cl₂ (25 mL) with 1 mL of i-PrOH, followed by adding hydrogen chloride in ether (2N, 3 mL). The mixture was heated at 40°C until it became colorless. The solvents were then removed and an off white solid was collected and washed with acetone (2 mL), hexane (2x3 mL) and dried in oven. 0.45g of product was obtained. mp 206.5°C (dec.). ¹H NMR (300MHz, CD30D) δ 1.59 (6H, s), 1.61 (6H, s), 2.28 (2H, t, J=13.1Hz), 2.66 (2H, dd, J=13.89, 2.4Hz), 3.75 (4H, m), 4.07 (4H, m), 5.47 (1H, t, J=5.3Hz). 8.66 (2H, d, J=5.3Hz). ¹³C NMR (75MHz, CD30D) δ 219.35, 26.95, 40.81, 44.23, 46.41, 68.78, 70.23, 109.63, 149.30, 152.25, 153.48, 156.51.

### Preparation of Compound 109 (1-Hydroxy-4-(6'-methoxy-benzothiazole-2'-yloxy)-2,2,6,6-tetramethyl-piperidine hydrochloride)

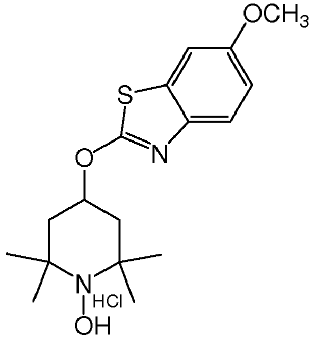

### Step 1

To a solution of 4-hydroxy-2,2,6,6-tetramethylpiperidine-1-oxyl (0.947 g, 5.5 mmol) in DMF (5 mL), NaH (0.24 g, 6 mmol) and 2-Chloro-6-methoxybenzothiazole (0.998 g, 5 mmol) were added sequentially. After the mixture was magnetically stirred at room temperature overnight, EtOAc (80 mL) was added. The organic phase was washed five times with distilled water and dried with sodium sulfate. Upon removal of the solvent in vacuo, the residual was purified through silica gel column chromatography using hexane and EtOAc:Hexane (1:9) as eluent and afforded pure product (1.42 g), 4-(6'-Methoxy-benzothiazole-2'-yloxy)-1-oxyl-2,2,6,6-tetramethylpiperidine. The yield was 84.6 %. Used as is in the next step.

### Step 2

To a solution of 4-(6'-Methoxy-benzothiazole-2'-yloxy)-1-oxyl-2,2,6,6-tetramethylpiperidine (0.5 g, 1.49 mmol) in 2-propanol (4 mL), hydrogen chloride in ether (2M, 5 mL) was added. The solution color was changed from dark brown to colorless right away. The solution was warmed up at water bath (40 ºC) for another 5 minutes. The solvent was evaporated and the product was dried in vacuum, which afforded product (0.56 g). Yield was100%. mp 161 ºC (dec.). ¹H NMR (300 MHz, CDCl₃/DMSO-d₆): δ12.38 (1H, s), 11.41 (1H, s), 7.66-7.58 (2H, m), 7.18 (1H, s), 7-6.97 (1H, d, *J*=8.7 Hz), 5.73-5.51 (1H, m), 3.84 (3H, s), 2.79-2.64 (2H, m), 2.47-2.43 (2H, m), 1.73-1.56 (12H, m). ¹³C NMR (75 MHz, CDCl₃/DMSO-d₆): δ170.27, 156.8, 141.04, 131.65, 121.35, 120.88, 114.51, 114.28, 105.25, 74.32, 72.79, 68.24, 66.6, 55.81, 40.55, 38.54, 28.13, 27.92, 22.36, 21.05. Anal. Calcd for C₁₇H₂₅ClN₂O₃S.0.5 H₂O: C, 53.46; H, 6.86; N, 7.33. Found: C, 53.54; H, 6.69; N, 7.25.

### Preparation of Compound 110 (4- Benzothiazole-2'-yloxy)-1-hydroxy-2,2,6,6-tetramethylpiperidine hydrochloride

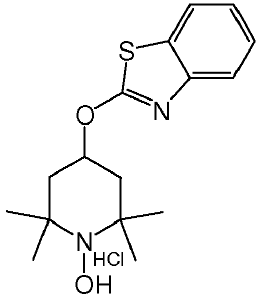

To a solution of 4-hydroxy-2,2,6,6-tetramethylpiperidine-1-oxyl (4- hydroxy - 2,2,6,6-tetramethylpiperidine 1-oxyl) (1.12 g, 6.5 mmol) in DMF (5 mL), sodium hydride (0.28 g, 6 mmol) was added and 2-chlorobenzothiazole (1.02 g, 6 mmol) was followed slowly. The mixture was magnetically stirred at room temperature overnight. To the mixture, ethyl acetate (80 mL) was added. The organic phase was washed five times with distilled water. After dried with sodium sulfate, ethyl acetate was removed under vacuum. Further purification through column using hexane and combination with ethyl acetate afforded 4-(Benzothiazole-2'-yloxy)-1-oxyl-2,2,6,6-tetramethylpiperidine (1.4 g). Yield was76%. Anal. Calcd for C₁₆H₂₁N₂O₂S: C, 62.92; H, 6.93; N, 9.17. Found: C, 62.76; H, 6.98; N, 9.13. mp 109.0 - 110.0 °C.

4-(Benzothiazole-2'-yloxy)-1-oxyl-2,2,6,6-tetramethylpiperidine (0.5 g, 1.6 mmol) was attempted to dissolve in 2-propanol (10 mL) with heating at 55 ºC. Some solid was still not dissolved. Hydrogen chloride in ether (2M, 5 mL) was added with stirring at room temperature Red color was turned lighter and solid disappeared gradually. Two hours later, the solution was dried in vacuum, which afforded white solid (0.57 g). Yield was 100%. mp 160 ºC (dec.).
¹H NMR (300 MHz , CDCl3): δ11.8 (s, 1H), 11.07 (s, 1H), 7.21-7.68 (m, 4H), 5.62 (m, 1H), 2.9-2.69 (m, 4H), 1.23-1.77 (m, 12H). ¹³C NMR (75 MHz, CDCl3): δ171.6, 149.09, 131.97, 126.23, 126.02, 123.9, 123.72, 121.41, 121.16, 120.87, 71.23, 68.82, 67.11, 64.67, 40.78, 38.84, 28.63, 28.32, 25.19, 22.49, 21.25. Anal. Calcd for C₁₆H₂₃ClN₂O₂S·0.3 H₂O: C, 55.08; H, 6.84; N, 8.03. Found: C, 55.43; H, 7.06; N, 7.66.

### Preparation of Compound 111 (1-Hydroxy-4-(6'-fluoro-benzothiazole-2'-yloxy)-2,2,6,6-tetramethylpiperidine hydrochloride)

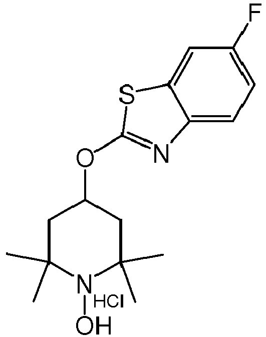

: To a solution of 4-hydroxy-2,2,6,6-tetramethylpiperidine-1-oxyl (1.03 g, 6 mmol) in DMF (5 mL), sodium hydride (0.26 g, 6.5 mmol) was added; 2-chloro-6-fluorobenzothiazole (1.0 g, 5.33 mmol) was followed slowly. The mixture was magnetically stirred at room temperature overnight. To the mixture, ethyl acetate (80 mL) was added. The organic phase was washed 5 times with distilled water. After dried with sodium sulfate, ethyl acetate was removed under vacuum. The syrup was dissolved in ethyl acetate (5 mL) with heating and hexane (30 mL) was followed. After the solution was cooled to room temperature the solution was left in a freezer overnight, which afforded nice red crystal, 1-Oxyl-4-(6'-fluoro-benzothiazole-2'-yloxy)-2,2,6,6-tetramethylpiperidine (1.28 g). The yield was 74%. mp 131-133 ºC. Anal. Calcd for C₁₆H₂₀FN₂O₂S: C, 59.42; H, 6.23; N, 8.66. Found: C, 59.65; H, 6.26; N, 8.74. 1-Hydroxy-4-(6'-fluoro-benzothiazole-2'-yloxy)-2,2,6,6-tetramethyl-piperidine (0.5 g, 1.4 mmol) was attempted to dissolve in 2-propanol (10 mL) with heating at 55 °C. Some solid was still not dissolved. Hydrogen chloride in ether (2M, 5 mL) was added to the mixture with stirring at room temperature Red color was turned lighter and solid disappeared gradually. Two hours later, solvent was removed and expected product (0.558 g) was obtained. Yield was 100%. mp 178 ºC (dec.). ¹H NMR (300 MHz, MeOD-d₄/DMSO-d₆) δ7.72-7.65 (m, 2H), 7.26-7.19 (m, 1H), 5.66-5.57 (m, 1H), 2.63-2.57 (m, 2H), 2.23-2.14 (m, 2H), 1.52 (s, 1H).
¹³C NMR (75 MHz, MeOD-d₄/DMSO-d₆) δ172.52, 162.24, 159.05, 147.14, 147.12, 134.18, 134.03, 123.33, 123.21, 115.75, 115.43, 110.23, 109.86, 73.29, 69.56, 69.54, 41.89, 28.88, 21.18.
Anal. Calcd for C₁₆H₂₂ClFN₂O₂S: C, 53.25; H, 6.14; N, 7.76. Found: C, 53.00; H, 6.15; N, 7.59.

### Preparation of Compound 112 (4-(2-((2,5-dihydro-1-hydroxy-2,2,5,5-tetramethyl-1H-pyrrol-3-yl)methoxy) phenyl)morpholine hydrochloride)

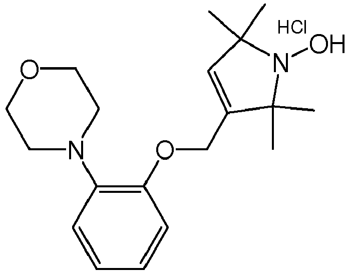

### Step 1

To the mixture of 4-(2-hydroxyphenyl) morpholine (1.79g, 10 mmol) and **potassium** carbonate (4.12g, 40mmol) in 150 ml of acetone, 3.5 g of the 3-(bromomethyl)-2,5-dihydro-2,2,5,5-tetramethyl-1H-pyrrol-1-oxy (H.O. Hankovszky et al, Synthesis, 914-916, 1980) was added in one portion. The mixture was refluxed with stirring for 48 hours. After filtration, acetone was removed in vacuum. The solid was purified by flash column (EtOAc/Hexane 1:10) and 2.47 g of light yellow solid, 4-(2-((2,5-dihydro-1-nitroxy-2,2,5,5-tetramethyl-1H-pyrrol-3-yl)methoxy)phenyl)morpholine, was obtained. Yield was 74.6%.

### Step2

To a solution of 4-(2-((2,5-dihydro-1-nitroxy-2,2,5,5-tetramethyl-1H-pyrrol-3-yl)methoxy)phenyl)morpholine (1.66g, 5mmol) in 2-propanol (~10 mL) was added a saturated solution containing hydrogen chloride in 2-propanol (~20 mL) in one portion, and the reaction mixture was stirred at 40°C for 2 hrs. TLC showed that the starting material disappeared and the solution turned colorless. The solvent was removed in vacuum to give an off-white solid (1.43g). Yield was 77.5%. mp 155.7°C (dec.). ¹H NMR (300MHz, CDCl₃), δ 12.21(s, 1H), 10.9(b, 1H), 8.15(d, 1H), 7.45(d, 1H), 7.10(m, 2H), 6.05(s, 1H), 4.80(m, 2H), 3.94(m, 2H), 3.60(m, 2H), 1.86(s, 3H), 1.77(s, 3H), 1.58(s, 3H), 1.53(s, 3H). ¹³C NMR (75MHz, CDCl₃), δ 131.91(CH), 129.76(CH), 123.95(CH), 123.08(CH), 115.05(CH), 67.00(CH₂), 65.27(CH), 63.86(CH₂), 52.44(CH₂), 25.03(CH₃),23.79(CH₃),23.09(CH₃),22.88(CH₃) (Dept 135). Anal. Calcd. for C₁₉H₂₉ClN₂O₃·1.5H₂O: C, 53.05; H, 7.83; N, 6.26; Found: C, 53.39; H, 8.09; N, 5.91.

### Preparation of Compound 113 (Diethyl (2,2,6,6-tetramethylpiperidin-4-ylcarbamoyl)methylphosphate hydrochloride)

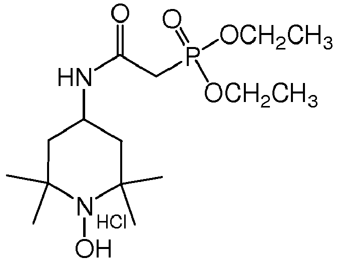

To a mixture of acid (6.6 g, 34 mmol), tempamine (6.08g, 35 mol), dichloromethane (200 mL), 4-dimethylaminopyridine (DMAP) (0.2 g) and diisopropylethylamine (4.78g, 37 mmol) at 0-5 °C was added 1-ethyl-3-(3-dimethylaminopropyl)carbodiimide (EDAC) (7.11g, 37 mmol). The mixture was stirred at room temperature overnight. The solvent was removed in vacuum to give an oil. It was dissolved in ethyl acetate (200 mL). The solution was added into waster (100 mL). The aqueous phase was extracted with ethyl acetate (2X100 mL). The combined ethyl acetate solution was dried and concentrated to give a solid, which was recrystallized in ethyl acetate-hexane three times. An orange solid of 5.5 grams of diethyl (2,2,6,6-tetramethylpiperidin-1-oxyl-4-ylcarbamoyl)methylphosphonate was obtained. Yield was 47%. 0.1g of above orange solid was dissolved in 2 mL of hydrogen chloride in methanol. It was heated at 60 °C for 10 minutes. The solvent was removed in vacuum to give a residue. The residue was treated with anhydrous ether (3 X 1 mL) to afford a semi-solid (0.05g). Anal.Calcd. for C₁₅H₃₁N₂O₅P.HCl.2H₂O: C, 42.60; H, 8.58; N, 6.62; Found: C, 42.74; H, 8.42; N, 6.54.

### Preparation of Compound 114((E)-N-(2,2,6,6-tetramethylpiperidin-1-hydroxyl-4-yl)-3-(4-morpholinophenyl) acrylamide hydrochloride)

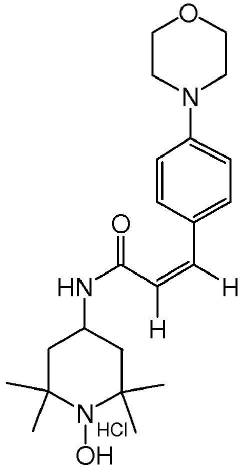

### Step 1

To a mixture of acid (6.6 g, 34 mmol), tempamine (6.08g, 35 mmol), dichloromethane (200 mL), 4-dimethylaminopyridine (0.2 g) and diisopropylethylamine ( 4.78g, 37 mmol) at 0-5 °C was added 1-ethyl-3-(3-dimethylaminopropyl)carbodiimide (EDAC) ( 7.11g, 37 mmol). The mixture was stirred at room temperature overnight. The solvent was removed in vacuum to give oil. It was dissolved in ethyl acetate (200 mL). The solution was added into waster (100 mL). The aqueous phase was extracted with ethyl acetate (2X100 mL). The combined ethyl acetate was dried and concentrated to give a solid, which was recrystallized in ethyl acetate-hexane three times. An orange solid of 5.5 grams of diethyl (2,2,6,6-tetramethylpiperidin-1-oxyl-4-ylcarbamoyl)methylphosphonate was obtained. Yield was 47%. Used as is in the next step.

### Step 2

To a solution of (2,2,6,6-tetramethylpiperidin-1-oxyl-4-ylcarbamoyl) methylphosphonate (1g, 2.9 mmol) in anhydrous THF (30 mL) under nitrogen at 0-5 °C was added sodium hydride (0.14g, 6 mmol). The mixture was stirred at room temperature for 30 minutes. Then 4-(4-morpholinyl)benzaldehyde (0.60g, 3 mmol) was added in one batch. The reaction mixture was stirred at room temperature for one hour. Water was added to the mixture carefully. The solution was extracted with ethyl acetate (3x50 mL). The ethyl acetate layers were combined, dried and concentrated to give a solid, which was purified by column chromatography (silica gel, hexanes/ethyl acetate) to give 0.6 grams of (E)-N-(2,2,6,6-tetramethylpiperidin-1-oxyl-4-yl)-3-(4-morpholinophenyl)acrylamide as an orange solid. Yield was 49%. Used as is in the next step.

### Step 3

(E)-N-(2,2,6,6-tetramethylpiperidin-1-oxyl-4-yl)-3-(4-morpholinophenyl)acrylamide (0.35g, 0.24 mmol) in methanol (1 mL) was heated at 50 °C for 10 minutes till the orange color disappeared. The solvent was removed to give a residue, which was washed with anhydrous ether (3X2 mL) to afford a solid (0.3g). mp 122.2 °C. Yield was 78%.
¹H NMR (300 MHz, MeOD) δ 7.80-7.76(m, 4H), 7.59-7.54(d, J=15Hz, 1H), 6.72-6.67(d, J=15 Hz, 1H), 4.50-4.35(m, 1H), 4.15-4.07(m, 4H), 3.8-3.67(m, 4H), 2.25-1.97(m, 4H), 1.59-1.47(m, 12H).

### Preparation of Compound 116 (3,4-dihydro-6-hydroxy-2,5,7,8-tetramethyl-N-(1-hydroxy-2,2,6,6-tetramethylpiperidin-4-yl)-2H-chromene-2-carboxamide hydrochloride)

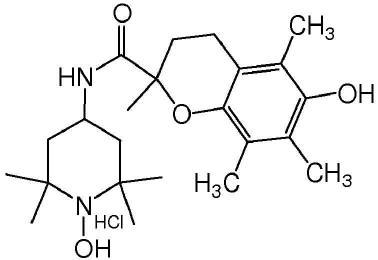

### Step 1

To a solution of 6-Hydroxy-2,5,7,8-tetramethylchromane-2-carboxylic acid (2.50g, 10 mmol), 4-amino-2,2,6,6-tetramethylpiperidine-1-oxyl (1.71g, 10 mmol) and 4-4-Dimethylaminopyridine (DMAP) (0.6g, 5 mmol) in CH₂Cl₂ (50 mL) at 0-5 °C, EDAC (2.14g, 11 mmol) in dichloromethane(50 mL) was added dropwise. After the addition was complete, the mixture was stirred at room temperature overnight. The reaction mixture was washed with water (2x50 mL), 1N HCl (20 mL) and saturated Na₂CO₃ (20 mL) and dried over MgS04. After MgS04 was filtered off, the solvent was removed in vacuum to give a solid. The solid was purified by column chromatography (silica gel, EtOAc/Hexane 1:10). The product, 3,4-dihydro-6-hydroxy-2,5,7,8-tetramethyl-N-(1-nitroxy-2,2,6,6-tetramethylpiperidin-4-yl)-2H-chromene-2-carboxamide, was an orange solid (2.61g). The yield was 64.7%. Used as is in the next step.

### Step 2

To a solution of 3,4-dihydro-6-hydroxy-2,5,7,8-tetramethyl-N-(1-nitroxy-2,2,6,6-tetramethylpiperidin-4-yl)-2H-chromene-2-carboxamide (0.8g, 1.9mmol) in 2-propanol (~10 mL) was added a saturated hydrogen chloride solution in methanol (~20 mL) in one portion, and the reaction mixture was stirred at 40°C for 2 hrs. TLC showed that the starting material disappeared. The color of the solution was light yellow. The solvent was removed in vacuum to give a light yellow solid (0.72g). Yield was 83.9%. mp 174.9 °C (dec.). ¹H NMR (300MHz, CDCl₃) δ 11.59 (b, 1H), 10.82(b, 1H), 6.53(d, 1H), 4.09 (m, 1H), 2.81(m. 2H), 2.61(s, 3H), 2.58(s, 3H), 2.56(m, 2H), 2.53(s, 3H), 2.03(m, 2H), 1.95(m, 2H), 1.69(s, 3H), 1.63(s, 3H), 1.43(s, 6H), 1.35(s, 3H). Anal. Calcd. (C₂₃H₃₇ClN₂O₄·2H₂O) C 57.91; H 8.66; N 5.87% ; Founded C 58.23; H 8.38; N 5.94%.

### Preparation of Compound 117 (4-(4-bromobutoxy)-1-hydroxy-2,2,6,6-tetramethylpiperidine hydrochloride)

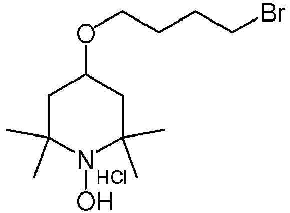

### Step1

4-hydroxy-2,2,6,6-tetramethylpiperidine-1-oxyl (5.24g, 31 mmol) was added to a three-neck flask containing benzene (150 mL). Under Nitrogen, NaH (1.11g, 46 mmol) was added and refluxed with stirring for 24 hours. After cooling in ice-cold water and 100 mL of water was added. The mixture was extracted two times with ethyl acetate (2 X 150 mL). The combined ethyl acetate solution was dried and concentrated to give an oil, which was purified by column chromatography (silica gel, hexanes and then hexanes/ethyl acetate 2:1). 6.6 grams of solid was obtained.

### Step 2

Above solid (0.3g, 1 mmol) was dissolved in 2 mL of methanol and hydrogen chloride solution in methanol (2 mL) was added. The mixture was heated till it became colorless. The solvent was removed in vacuum to give a residue, which was washed with ether (3 X 1 mL). 0.1g of solid was obtained. Yield was 30%. mp 130 °C (dec.). ¹NMR (300 MHz, MeOD) δ 3.92-3.88(m, 1H), 3.61-3.42(m, 4H), 2.35-2.31(m,2H), 2.02-1.88(m, 2H), 1.62-1.55(m, 4H), 1.48(s, 9H). ¹³C NMR (75 MHz, MeOD) δ 66.99, 65.98, 65.78, 40.33, 36.94, 31.30, 27.98, 26.68, 25.91, 25.60, 18.83, 17.69. Anal. Calcd for (C₁₃H₂₆BrNO.HCl) C, 45.3; H, 7.89; N, 4.06. Found: C, 45.55; H, 8.04; N, 4.04.

### Preparation of Compound 119 (2-(4-(1-hydroxy-2,2,6,6-tetramethylpiperidin-4-yloxy)-1,2,5-thiadiazol-3-yl)-1,2,3,4-tetrahydro-6,7-dimethoxyisoquinoline hydrochloride)

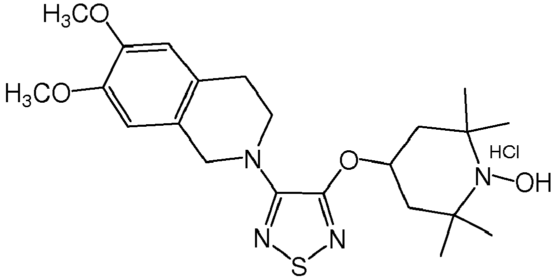

: To a solution of 4-hydroxy-2,2,6,6-tetramethylpiperidine-1-oxyl (2.2g, 12.8 mmol) and t-BuOK (1.9g, 15.5 mmol) in t-BuOH (30 mL) was added 2-(4-chloro-1,2,5-thiadiazol-3-yl)-1,2,3,4-tetrahydro-6,7-dimethoxyisoquinoline (3.3g, 10.6 mmol). The dark solution was stirred at room temperature over weekend. The reaction was monitored by TLC (Hex/EtOAc 1/9). Water (10 mL) was added and the mixture was stirred for another 30 min. The mixture was extracted with CH₂Cl₂ (3x20 mL). The combined organic layer was dried over MgSO₄ and evaporated. The residue was purified by column chromatography (silica gel, Hex/EtOAc (9/1)). 0.96g of red solid was obtained. Yield was 15.8%. 0.3g of the above solid was dissolved in 20 mL of 2-propanol at 50°C. The saturated hydrogen chloride in 2-propanolwas added until the solution became light yellow. The solvent was removed and the residue was dissolved in CH₂Cl₂. Again the solvent was removed and foam was obtained (0.28g). mp 166.9°C (dec.). Yield was 86.1%. Anal. Calcd for C₂₂H₃₃ClN₄O₄S.(CH₃COCH₃): C, 55.29; H, 7.24; N, 10.32. Found: C, 55.38; H, 7.05; N, 10.29.

### Preparation of Compound 120 (4-(2,2,6,6-tetramethylpiperidin-4-yloxy)-1,2,5-thiadiazol-3-yl)morpholino)methanone hydrochloride)

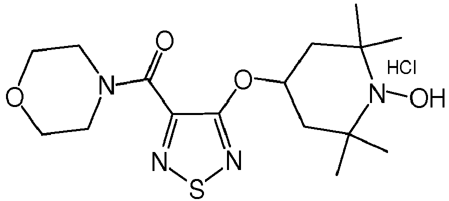

To a solution of 4-HYDROXY-2,2,6,6-tetramethylpiperidine-1-oxyl (1.45g, 8.4 mmol) and t-BuOK (1.0g, 8.1 mmol) in t-BuOH (30 mL) was added (4-chloro-1,2,5-thiadiazol-3-yl)morpholino)methanone (1.2g, 5.1 mmol). The dark solution was stirred at room temperature A solid came out immediately. The reaction was monitored by TLC (CH₂Cl₂/EtOAc 8/2). Solvent was removed to dryness. The residue was separated by prep. TLC with CH₂Cl₂ containing 1% of methanol. 0.73g of red solid was obtained. Yield was 38.8%. 0.38g of above red solid was converted to HCl salt by dissolving in CH₂Cl₂ (20 mL), 2-propanol (0.5 mL) and hydrogen chloride in ether (1N, 1ml). The solution was warmed at 45oC until the red color turned light yellow. The solvent was removed and the residue was taken into acetone (10 mL). The precipitate was collected and washed with acetone (2x3 mL), hexane (2x3 mL) and dried in oven. 0.31g of product was obtained. The precipitate was collected and washed with acetone (2x3 mL), hexane (2x3 mL) and dried in oven (0.31g).
mp 186.9°C (dec.). Anal. Calcd for C₁₆H₂₇ClN₄O₄S: C, 47.22; H, 6.69; N, 13.77. Found: C, 47.44; H, 6.85; N, 13.62.

### Preparation of Compound 122 (4-(Benzo[d]thiazol-2'-amino)-1-hydroxyl-2,2,6,6-tetramethylpiperidine hydrochloride)

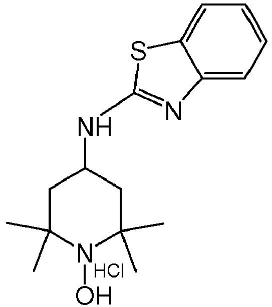

### Step 1

A mixture of 4-Amino-2,2,6,6-tetramethylpiperidine-1-oxyl (1.11 g, 6.5 mmol) and 2-chlorobenzothiazole (1.02 g, 6 mmol) in DMF (5 mL) was magnetically stirred at 110 °C overnight. The mixture was purified using preparatory TLC plates (EtOAc:Hexane 1:1), which afforded product (1 g). Recrystallization in MeOH/EtOAc afforded a red crystal (0.42 g), 4-(Benzo[d]thiazol-2'-amino)-1-oxyl-2,2,6,6-tetramethylpiperidine. Yield was 23%. mp 210 ºC (dec.). Anal. Calcd for C₁₆H₂₂N₃OS: C, 63.12; H, 7.28; N, 13.80. Found: C, 63.11; H, 7.32; N, 13.61.

### Step 2

4-(Benzo[d]thiazol-2'-amino)-1-oxyl-2,2,6,6-tetramethylpiperidine (0.5 g, 1.6 mmol) was put in 2-propanol (10 mL) with heating at 70 ºC. Some solid was still not dissolved. Hydrogen chloride in ether (2M, 5 mL) was added with stirring at room temperature Red color was turned lighter, the solid disappeared gradually. New solid came out, which afforded product (0.19 g). Yield was 31%. mp 183 °C (dec.). ¹H NMR (300 MHz, CDCl3): δ15.62 (1H, s), 12.48 (1H, s), 11.19 (1H, s), 10.77 (1H, s), 8.01-7.99 (1H, m), 7.58-7.55 (1H, m), 7.44-7.39 (1H, m), 7.31-7.27 (1H, m), 5.33 (1H, b), 2.68-2.6 (2H, m), 2.22-2.18 (2H, m), 1.69 (6H, s), 1.66 (6H, s). ¹³C NMR (75 MHz, CDCl3): δ170.71, 132.33, 129.64, 127.92, 126.4, 120.54, 72.57, 52.59, 46.25, 32.55, 25.65. Anal. Calcd for C₁₆H₂₅Cl₂N₃OS·0.7H₂O: C, 49.15; H, 6.81; N, 10.75. Found: C, 49.41; H, 6.66; N, 10.39.

### Preparation of Compoind 123 (4-(6'-Methoxy-benzo[d]thiazol-2'-amino)-1-hydroxyl-2,2,6,6-tetramethyl-piperidine dihydrochloride)

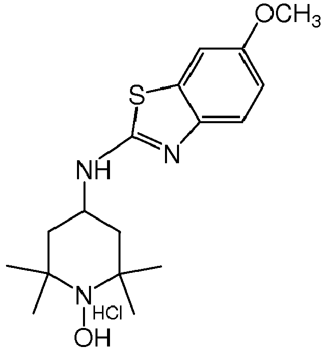

### Step 1

To a solution of 4-Amino-2,2,6,6-tetramethylpiperidine-1-oxyl (4-amino-2,2,6,6-tetramethylpiperidine 1-oxyl) (1.11 g, 6.5 mmol) in DMF (5 mL) was added; 2-chloro-6-methoxybenzothiazole (1.02 g, 6 mmol) was added subsequently. The mixture was magnetically stirred at 110 ºC for 18 hours. After dried over the vacuum, the mixture was purified with preparatory TLC plate (EtOAc:Hexane 1:1). The product was crystallized by dissolving in EtOAc (2 mL) with heating, which afforded a red crystal (0.48 g), 4-(6'-Methoxy-benzo[d]thiazol-2'-amino)-1-oxyl-2,2,6,6-tetramethyl-piperidine. The yield was 24%. mp 170 °C (dec.).
Anal. Calcd for C₁₇H₂₄N₃O₂S: C, 61.05; H, 7.23; N, 12.56. Found: C, 61.09; H, 7.28; N, 12.51.

### Step 2

To a solution of 4-(6'-methoxy-benzo[d]thiazol-2'-amino)-1-oxo-2,2,6,6-tetramethylpiperidine (0.3 g, 0.90 mmol) in 2-propanol (1.5 mL)/dichloromethane (5 mL), Hydrogen chloride in ether (2M, 5 mL) was added with stirring at room temperature Red color was turned lighter. White crystal appeared in one hour. Hexane (15 mL) was added to the liquid. The reaction mixture was allowed to stand for another one hour. After removal of the upper clear pale yellow solution, expected product (0.32 g) was obtained. The yield was 87%. mp 220 °C (dec.).
¹H NMR (300 MHz, CDCl3): δ15.37 (1H, s), 12.4 (1H, s), 11.2 (1H, s), 10.63 (1H, s), 7.87 (1H, d, *J*=8.9 Hz), 7.11 (1H, s), 6.91 (1H, dd, *J₁*=8.93, *J₂*=2.4), 5.23 (1H, b), 3.83 (3H, s), 2.65-2.57 (2H, m), 2.22-2.17 (2H, m), 1.68 (6H, s), 1.65 (6H, s). ¹³C NMR (75 MHz, CDCl3): δ164.67, 156.7, 131.92, 123.94, 115.78, 114.24, 105.67, 67.32, 55.39, 47.08, 40.97, 27.27, 20.31. Anal. Calcd for C₁₇H₂₇Cl₂N₃O₂S·0.5H₂O: C, 48.92; H, 6.76; N, 10.07. Found: C, 48.90; H, 6.72; N, 9.83.

### Preparation of Compound 124 (N-(1-hydroxy-2,2,6,6-tetramethylpiperidin-4-yl)-7-chloro-benzo[c] [1,2,5] oxadiazole-4-sulfonamide)

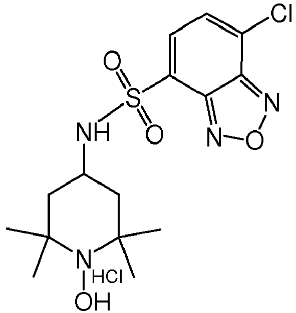

### Step 1

To the solution of 4-amino-2,2,6,6-tetramethylpiperidine-1-oxyl (5.0g, 29 mmol), triethylamine (3.76g) in 100 mL of THF in an ice water bath, 4-Chloro-7-chlorosulfonyl-2,1,3-benzoxadiazole (5.0g, 20 mmol) in 50 ml of THF was added. After the addition was completed, the ice water bath was removed. The mixture was kept stirring at room temperature for 4 hours. 500 ml of EtOAc was added to the reaction mixture, and then the mixture was washed with water (2 x 100 mL), 100 ml of 1N HCl , 100 ml of saturated aqueous sodium carbonate and 100 mL of saturated aqueous brine. After dried over sodium sulfate, and filtration, the solvent was removed in vacuum. The crude solid was purified by flash column chromatography (silica gel, EtOAc/Hexane 1:2) to give 5.8g yellow solid. Yield was 75.6%.

### Step 2

0.5 g of above yellow solid (1.29 mmol) was dissolved into 20 mL of methanol at 50°C for half an hour to form a clear solution and 10 mL of saturated hydrogen chloride in methanol was added in one portion. The solution was kept stirring at 50°C for two more hours. TLC showed that the starting material disappeared and the color of the solution is golden yellow. The solvent was removed in vacuum and 5 mL of i-Pr₂O was added and the residue was kept stirring at room temperature for 3 hours. The solvent was decanted and the residue was washed with i-Pr₂O (2x5 mL). The solid was dried in vacuum to give 0.39 g of yellow solid. Yield was 71.3%. mp 176.0°C (dec.).
¹H NMR (300MHz, CDCl₃) δ 11.36(s, 1H), 10.66(s, 1H), 8.04(d, 2H), 7.58(d, 2H), 4.48(m, 1H), 2.50(m, 2H), 1.99(m, 2H), 1.57(s, 6H), 1.53(s, 6H). ¹³C NMR (75MHz, CDCl₃)(dept) δ 130.0, 129.30, 45.21, 43.22, 27.96, 20.60.

### Preparation of Compound 125 (N-(1-hydroxy-2,2,6,6-tetramethylpiperidin-4-yl)-7-morpholinobenzo[c][1,2,5] oxadiazole-4-sulfonamide)

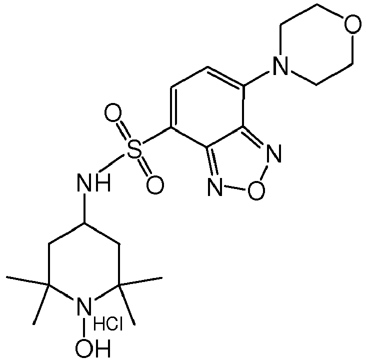

### Step 1

To the solution of 4-amino-2,2,6,6-tetramethylpiperidine-1-oxyl (5.0g, 29 mmol), triethylamine (3.76g) in100 mL of THF in an ice water bath, 4-Chloro-7-chlorosulfonyl-2,1,3-benzoxadiazole (5.0g, 20 mmol) in 50 ml of THF was added. After the addition was completed, the ice water bath was removed. The mixture was kept stirring at room temperature for 4 hours. 500 mL of EtOAc was added to the reaction mixture, and then the mixture was washed with water (2 x 100 mL), 100 mL of 1N HCl , 100 mL of saturated aqueous sodium carbonate and 100 mL of saturated aqueous brine. After dried over sodium sulfate, and filtration, the solvent was removed in vacuum. The crude solid was purified by flash column chromatography ( silica gel, EtOAc/Hexane 1:2) to give 5.8g yellow solid. Yield was 75.6%.

### Step 2

To the mixture of the saturated hydrogen chloride in 2-propanol (26 mg), and t-BuOK (0.8g) in 10 mL of THF, nickel complex (44mg) was added. The mixture was kept stirring at room temperature for half hour. 0.81 g of sulfonylamide was added. After half an hour, 0.21 g of morpholine was added. The mixture was refluxed with stirring for 10 hours. 100 mL of water was added to the mixture and the mixture was extracted with EtOAc (3 x 100 mL). The organic phase was washed with saturated aqueous brine and dried over sodium sulfate. After filtration, the solvent was removed in vacuum. The crude solid was purified by flash column (EtOAc/Hexane 1:2). 0.75 g of orange solid was obtained.

### Step 3

To a solution of above orange solid (0.5g) in 2-propanol (~10 mL) was added a saturated hydrogen chloride solution in 2-propanol (~20 mL) in one portion, and the reaction mixture was stirred at 40°C for 2 hrs. TLC showed that the starting material disappeared and the solution turned colorless. The solvent was removed in vacuum to give an off-white solid (0.43g). Yield was 79.3%. mp 176.0°C (dec.). ¹H NMR (300MHz, CDCl₃) δ 7.97(d, 1H), 6.56(d, 1H), 4.18(m, 1H), 3.69(m, 8H), 2.07(dd, 2H), 1.81(t, 2H), 1.47(s, 6H), 1.41(s, 6H).
The compounds are further characterized in TABLE 1.

**TABLE 1**

| Co. No.- | Recryst Solvent | Melting point °C | Molecular formula | Elemental analysis | | | | | |
|---|---|---|---|---|---|---|---|---|---|
| | | | | Theoretical | | | Found | | |
| | | | | %C | %H | %N | %C | %H | %N |
| 1 | i-PrOH | 198.5(dec.) | C₁₀H₂₁NO₂.HCl | 53.68 | 9.91 | 6.26 | | | |
| 2 | i-PrOH | 180.5(dec.) | C₁₁H₂₂N₂O₂.HCl | 52.69 | 9.24 | 11.17 | | | |
| 3 | i-PrOH | 229.2(dec.) | C₁₃H₂₆N₂O₂.HCl | 56.00 | 9.76 | 10.05 | | | |
| 4 | i-PrOH | 203.3(dec.) | C₁₅H₂₆N₄O₃S.HCl | 47.55 | 7.18 | 14.79 | | | |
| 5 | i-PrOH | 166.0(dec.) | C₉H₁₇NO₂.HCl | 52.05 | 8.74 | 6.74 | | | |
| 6 | i-PrOH | 191.5(dec.) | C₁₁H₂₃NO₂.HCl | 55.57 | 10.17 | 5.89 | | | |
| 7 | i-PrOH | 155.5(dec.) | C₈H₁₇NO₂.HCl.0.08H₂O | 48.74 | 9.29 | 7.11 | 48.59 | 9.18 | 7.44 |
| 8 | i-PrOH | 141.9(dec.) | C₉H₁₈ClNO₂.0.2C₃H₈O | 52.48 | 8.99 | 6.37 | 52.64 | 8.92 | 6.62 |
| 9 | i-PrOH | 138.5(dec.) | C₉H₁₉BrClNO₂ | 37.45 | 6.64 | 4.85 | 37.75 | 6.89 | 5.00 |
| 10 | i-PrOH | 178.3(dec.) | C₁₀H₂₂N₂O₃S.HCl.H₂O | 39.40 | 8.27 | 9.19 | 39.70 | 8.51 | 9.19 |
| 11 | i-PrOH | 158.1(dec.) | C₁₄H₂₇N₃O₃.HCl.0.95H₂ O | 49.61 | 8.89 | 12.40 | 49.69 | 9.21 | 12.16 |
| 12 | i-PrOH | 186.0(dec.) | C₁₀H₁₉ClN₂O | 54.91 | 8.76 | 12.81 | 54.94 | 8.64 | 12.73 |
| 13 | i-PrOH | 161.5(dec) | C₁₁H₁₉ClN₃O₂S 0.5HCl | 38.13 | 5.67 | 12.13 | 38.15 | 5.50 | 11.84 |
| 14 | i-PrOH | 216.2(dec.) | C₂₀H₃₈Cl₂N₄O₄S | 47.90 | 7.64 | 11.17 | | | |
| 15 | i-PrOH | 225.5(dec.) | C₁₃H₁₈ClNO₃ | 57.46 | 6.68 | 5.15 | | | |
| 20 | i-PrOH | 184.6(dec.) | C₈H₁₈ClNO₂ | 49.10 | 9.27 | 7.16 | 48.93 | 9.35 | 7.17 |
| 21 | i-PrOH | 201.8(dec.) | C₉H₁₉Cl₂NO | 47.38 | 8.39 | 6.14 | 47.34 | 8.49 | 5.96 |
| 26 | i-PrOH | 193.3 | C₁₆H₃₃Cl₂N₃O₃ | 49.74 | 8.61 | 10.88 | 50.06 | 8.95 | 10.64 |
| 27 | | 168.7(dec.) | C₁₇H₂₇ClN₂O₄ | 56.90 | 7.58 | 7.81 | | | |
| 28 | | 214.8(dec.) | C₁₇H₂₇NO₄ | 65.99 | 8.80 | 4.53 | 65.91 | 9.17 | 4.49 |
| 29 | | 218.0(dec.) | C₂₄H₄₀N₂O₃.HCl | 65.36 | 9.37 | 6.35 | | | |
| 30 | | 166.0(dec.) | C₁₀H₁₉N₅O.HCl | 45.89 | 7.70 | 26.76 | | | |
| 31 | | 230.0(dec.) | C₁₃H₂₅ClN₂O₂ | 56.41 | 9.10 | 10.12 | 56.52 | 8.83 | 9.82 |
| 32 | | 178.6(dec.) | C₁₄H₂₅ClN₄O₃S | 46.08 | 6.91 | 15.35 | 46.17 | 7.15 | 15.05 |
| 33 | | 152.3(dec.) | C₉H₂₀ClNO₂ | 51.54 | 9.61 | 6.68 | 51.67 | 9.38 | 6.55 |
| 34 | | 220.8(dec.) | C₁₀H₂₂ClNO₂ | 53.68 | 9.91 | 6.26 | 53.91 | 9.73 | 6.15 |
| 35 | | 163.2(dec.) | C₉H₁₈ClNO | 56.39 | 9.46 | 7.31 | 56.48 | 9.15 | 7.17 |
| 36 | | 172.4(dec.) | C₁₃H₂₆Cl₂N₂O₂·0.25H₂O | 49.14 | 8.41 | 8.82 | 49.42 | 8.11 | 8.53 |
| 37 | | 157.8(dec.) | C₈H₁₅NO₂.HCl | 49.61 | 8.33 | 7.23 | | | |
| 38 | | 127.1(dec.) | C₁₂H₂₂N₂O₂.HCl | 54.85 | 8.82 | 10.66 | | | |
| 39 | | 145.0(dec.) | C₈H₁₆ClNO₃ | 45.83 | 7.69 | 6.68 | 45.91 | 7.31 | 6.64 |
| 40 | | oil | C₁₂H₂₈ClNO₃ | 53.42 | 10.46 | 5.19 | 53.16 | 10.8 2 | 5.51 |
| 41 | | 187.0(dec.) | C₁₃H₂₈ClNO₂ | 58.74 | 10.62 | 5.27 | 58.65 | 10.6 9 | 5.24 |
| 42 | | 201.7(dec.) | C₁₅H₂₄ClNO₂ | 63.04 | 8.46 | 4.90 | 63.00 | 8.78 | 4.84 |
| 43 | | 181.5(dec.) | C₁₆H₂₆ClNO₂ | 64.09 | 8.74 | 4.67 | 63.74 | 8.92 | 4.57 |
| 44 | | 220.8(dec) | C₁₇H₂₄ClN₃O₂S | 55.20 | 6.54 | 11.36 | 54.98 | 6.64 | 11.16 |
| 45 | | 179.2(dec.) | C₁₂H₁₉ClN₄O₂S | 45.21 | 6.01 | 17.57 | 45.13 | 5.96 | 17.21 |
| 46 | | foam | C₂₅H₃₂ClNO₆ | 62.82 | 6.75 | 2.93 | | | |
| 47 | EtOAc/Hexane | 173.2 | C₁₂H₂₀ClN₃O₂S | 47.13 | 6.59 | 13.74 | 47.04 | 6.81 | 13.42 |
| 48 | MeOH /i-Propyl ether | 186.0(dec.) | C₁₉H₃₂ClNO₅ | 58.53 | 8.27 | 3.59 | 58.45 | 8.42 | 3.54 |
| 49 | | 164.7(dec.) | C₁₇H₃₃ClN₂O₂S₂ | 51.43 | 8.38 | 7.06 | 51.64 | 8.51 | 6.68 |
| 50 | | 159.9(dec.) | C₁₈H₂₇NO_{4.}HCl | 60.41 | 7.89 | 3.91 | 59.51 | 8.14 | 3.65 |
| 51 | | 155.7(dec.) | C₁₄H₂₄ClN₃O₂S | 50.36 | 7.25 | 12.59 | 50.57 | 7.51 | 12.33 |
| 52 | | | C₁₆H₃₁Cl₂N₅O₂S | 44.86 | 7.29 | 16.35 | 44.77 | 7.40 | 16.09 |
| 53 | MeOH/i-PrOH | 205.8(dec.) | C₂₁H₃₁N₅O₂S.2HCl | 51.42 | 6.78 | 14.28 | | | |
| 55 | | 218.0(dec.) | C₁₅H₂₇ClN₄O₂S₂ | 45.61 | 6.89 | 14.18 | 45.44 | 6.89 | 13.93 |
| 56 | | 205.8(dec.) | C₂₁H₃₃Cl₂N₃O₂·1.25H₂O | 55.69 | 7.90 | 9.26 | 56.08 | 8.08 | 8.84 |
| 57 | MeOH/ i-Propyl | 220.0(dec.) | C₁₅H₂₃ClFNO₂ | 59.30 | 7.63 | 4.61 | 59.31 | 7.75 | 4.45 |

| | Ether | | | | | | | | |
|---|---|---|---|---|---|---|---|---|---|
| 58 | MeOH/ ether | 181.0 | C₁₉H₃₂N₂O₄.2HCl.0.75H₂ O | 52.00 | 8.10 | 6.39 | 52.02 | 8.20 | 6.58 |
| 59 | | 191.7(dec.) | C₁₅H₂₀FNO₂.HCl | 59.70 | 7.01 | 4.64 | | | |
| 61 | heptane | 87.0-88.4 | C₁₆H₂₅NO₂ | 72.96 | 9.57 | 5.32 | 72.66 | 9.61 | 5.33 |
| 62 | | Foam | C₁₅H₂₁ClFNO | 63.01 | 7.41 | 4.90 | 62.83 | 7.40 | 4.80 |
| 64 | | 109.8-111.8 | C₁₅H₂₆N₄O₂S | 55.19 | 8.03 | 17.16 | 55.01 | 8.19 | 16.87 |
| 65 | MeOH/ ether | 214.1 | C₁₂H₁₆NOBr.HCl | 47.01 | 5.58 | 4.64 | 47.21 | 5.58 | 4.64 |
| 66 | MeOH/ ether | 148.9(dec.) | C₁₆H₂₄N₂O₂. HCl | 61.43 | 8.05 | 8.95 | | | |
| 69 | | Foam | C₁₄H₂₂ClNO.0.1H2O | 65.28 | 8.69 | 5.44 | 65.12 | 8.71 | 5.32 |
| 71 | | Foam | C₁₉H₃₃ClN₄O₄S.0.5H₂O | 49.82 | 7.48 | 12.23 | 49.72 | 7.35 | 12.04 |
| 72 | EtOAc | 89.4(dec.) | C₂₁H₃₂Cl₂FN₅O₂S.1.5H₂O | 47.10 | 6.59 | 13.08 | 46.92 | 6.62 | 12.77 |
| 73 | | 168.7-175.3(dec.) | C₉H₁₉ClN₂O₄ | 42.44 | 7.52 | 11.00 | 42.56 | 7.73 | 10.81 |
| 74 | | Foam | C₁₇H₂₇ClNO₃ | 61.90 | 8.56 | 4.25 | 61.99 | 8.59 | 4.07 |
| 76 | | 46.3-47.7 | C₈H₁₅NO₃·H₂O | 54.84 | 9.78 | 7.99 | 55.09 | 9.74 | 7.95 |
| 77 | | 190.0(dec.) | C₁₉H₂₈N₂O₄.HCl | 59.29 | 7.59 | 7.28 | | | |
| 78 | | 262.7 | C₁₆H₂₁NO₂.0.3H₂O.HCl | 63.80 | 7.56 | 4.65 | 63.87 | 7.52 | 4.67 |
| 83 | EtOAc | 149.3 | C₁₅H₂₄N₂O.1.1H₂O | 67.18 | 9.85 | 10.45 | 66.99 | 9.81 | 10.30 |
| 91 | | 199.0(dec.) | C₂₁H₃₂Cl₂FN₅O₂S 0.5H2O | 52.43 | 6.71 | 14.56 | 52.55 | 6.85 | 14.60 |
| 92 | | 213.5(dec.) | C₁₇H₂₉Cl₂N₅O₂S | 46.57 | 6.67 | 15.97 | 46.28 | 6.63 | 15.93 |
| 93 | | 181.5(dec.) | C₁₇H₂₉ClN₄O₄S | 48.50 | 6.94 | 13.31 | 48.45 | 7.15 | 13.03 |
| 94 | | 215.2(dec.) | C₂₆H₄₆Cl₂N₈O₄S₂ | 46.63 | 6.92 | 16.73 | 46.86 | 7.10 | 16.26 |
| 95 | acetone | 197.0(dec.) | C₁₆H₂₈ClN₃O₄S | 48.78 | 7.16 | 10.67 | 48.98 | 7.28 | 10.77 |
| 99 | | 233.7(dec.) | C₁₅H₂₃NO.0.5H₂O.HCl | 64.62 | 9.04 | 5.02 | 64.77 | 8.93 | 5.03 |
| 100 | | 162.6(dec.) | C₁₄H₂₂N₂O₂.0.15H₂O | 66.45 | 8.88 | 11.07 | 66.42 | 8.77 | 10.93 |
| 101 | | 100.0(dec.) | C₁₃H₁₉NS.HCl | 60.56 | 7.82 | 5.43 | | | |
| 103 | | 209.1(dec.) | C₁₂H₁₉N₃O₄S.0.9HCl | 43.13 | 6.00 | 12.57 | 43.10 | 6.03 | 12.33 |
| 104 | | 183.7(dec.) | C₁₄H₂₄ClN₃O₄S | 46.09 | 6.35 | 11.52 | 46.15 | 6.64 | 11.61 |
| 105 | | 198.1(dec.) | C₂₀H₃₀ClN₅O₄S | 50.89 | 6.41 | 14.84 | 50.88 | 6.50 | 14.48 |
| 106 | | Foam | C₂₀H₃₂Cl₂N₆O₂S.0.5H₂O | 48.00 | 6.65 | 16.79 | 48.97 | 6.87 | 15.49 |
| 107 | | 206.5(dec.) | C₁₉H₃₁Cl₂N₇O₂S.H₂O | 44.70 | 6.52 | 19.21 | 44.49 | 6.70 | 18.89 |
| 108 | | 107.0(dec.) | C₁₆H₂₈N₄O₂S | 56.44 | 8.29 | 16.46 | 56.42 | 8.37 | 16.34 |
| 109 | | 161.0(dec.) | C₁₇H₂₅ClN₂O₃S.0.5 H₂O | 53.46 | 6.86 | 7.33 | 53.54 | 6.69 | 7.25 |
| 110 | | 160.0(dec.) | C₁₆H₂₃ClN₂O₂S·0.3 H₂O | 55.08 | 6.84 | 8.03 | 55.43 | 7.06 | 7.66 |
| 111 | | 178.0(dec.) | C₁₆H₂₂ClFN₂O₂S | 53.25 | 6.14 | 7.76 | 53.00 | 6.15 | 7.59 |
| 112 | | 155.7(dec.) | C₁₉H₂₉ClN₂O₃·1.5H₂O | 53.05 | 7.83 | 6.26 | 53.39 | 8.09 | 5.91 |
| 113 | | Semi-solid | C₁₅H₃₁N₂O₅P.HCl.2H₂O | 42.60 | 8.58 | 6.62 | 42.74 | 8.42 | 6.54 |
| 114 | | 122.2 | C₂₂H₃₃N₃O₃.HCl | 62.32 | 8.08 | 9.91 | | | |
| 116 | | 174.9(dec.) | C₂₃H₃₇ClN₂O₄·2H₂O | 57.91 | 8.66 | 5.87 | 58.23 | 8.38 | 5.94 |
| 117 | | 130.0(dec.) | C₁₃H₂₆BrNO₂.HCl | 45.30 | 7.89 | 4.06 | 45.55 | 8.04 | 4.04 |
| 119 | | 166.9(dec.) | C₂₂H₃₃ClN₄O₄S | 54.48 | 6.86 | 11.55 | | | |
| 120 | | 186.9(dec.) | C₁₆H₂₇ClN₄O₄S | 47.22 | 6.69 | 13.77 | 47.44 | 6.85 | 13.62 |
| 122 | | 183.0(dec.) | C₁₆H₂₅Cl₂N₃OS·0.7H₂O | 49.15 | 6.81 | 10.75 | 49.41 | 6.66 | 10.39 |
| 123 | | 220.0(dec.) | C₁₇H₂₇Cl₂N₃O₂S·0.5H₂O | 48.92 | 6.76 | 10.07 | 48.90 | 6.72 | 9.83 |
| 124 | | 196.2(dec.) | C₁₅H₂₂Cl₂N₄O₄S | 42.36 | 5.21 | 13.17 | | | |
| 125 | | 176.0(dec.) | C₁₉H₃₁Cl₂N₅O₅S | 44.53 | 6.10 | 13.67 | | | |

### Biological Data

### Whole blood TNF α:

Compounds at different concentrations (0, 1, 2.5 and 10 uM) were incubated with 100 ul freshly collected heparinized blood for 10 mintes. LPS (25 ng/mL) was added and blood was incubated at room temperature for 3 hrs. Following incubation with LPS, PBS was added (800 uL) and samples were spun for 10 minutes at 1500 g. Compounds at different concentrations (0, 1, 2.5 and 10 uM) were incubated with 100 ul freshly collected heparinized blood for 10 ***mintes.*** TNFα protein concentrations were measured using R&D Systems high sensitivity ELISA kit.

### Measurement of LPS induced-TNFG α in THP-1 cells

The lipid peroxidation assay method of Ohkawa, H.; Ohishi, N.; Yaki, K. Anal. Biochem. 1979, 95, 351 was used to evaluate TNF α. Cells from a human acute monocyte leukemia cell line, THP-1 cells (0.5 x 10⁶ cells/mL), were incubated with the compounds (0, 1, 2.5 and 10 uM) for 3 hrs in humidified chamber, 37 °C, 5%CO₂, 2%FBS in RPMI medium. Cells were induced with 25 ng/mL LPS for another 3 hrs. Cells were collected and spun at 1500 g for 10 minutes. Supernatant was collected and analyzed for TNFα.

Mean Percent Inhibition of TNF-alpha by compounds of the present invention is set forth in Table 2. TNF-alpha inhibition data is set forth in Table 2a. OT-551 was used in both native form and in the form of nanoparticles. Improved efficacy when this material was used in nanoparticular form is shown. Other nitrogenous heterocyclic species of the invention are expected to show similar improvement in efficacy when disposed in nanoparticulate form.
Lipid peroxidation inhibition data for compounds of the present invention is set forth in Table 3.

**TABLE 2**

| **Compound No. (10 uM)** | **Mean Percent Inhibition of TNF-alpha using 5ng/mL LPS** |
|---|---|
| 1 | 50±9 |
| 2 | 64±5 |
| 3 | 60±2 |
| 4 | 61±10 |
| 5 | 66±7 |
| 6 | 54±6 |
| 7 | 69±2 |
| 8 | 55±6 |
| 9 | 48±14 |
| 11 | 24±5 |
| 12 | 35± |
| 13 | 36± |
| 14 | 63±1 |
| 15 | 67±7 |
| TEMPOL-H | 74±8 |
| OT-551 HCl | 68±6 |
| OT-551 nanoparticles | 54±0 |

**Table 2a TNF Alpha Inhibition Data**

| Compound Number | TNFalpha IC₅₀ (uM) |
|---|---|
| 1 | 16.8 |
| 2 | 17 |
| 3 | 11.6 |
| 4 | 6.7 |
| 5 | 22.2 |
| 6 | 29.44 |
| 7 | 44.9 |
| 8 | 20.3 |
| 9 | 18.9 |
| 10 | 100 |
| 11 | 20.7 |
| 12 | 21.5 |
| 13 | 16.4 |
| 14 | 11.4 |
| 20 | 100 |
| 21 | 21.7 |
| 23 | 87.4 |
| 24 | 100 |
| 25 | 30.9 |
| 26 | 100 |
| 27 | 100 |
| 29 | 45.5 |
| 30 | 100 |
| 31 | 38.2 |
| 32 | 100 |
| 33 | 55.7 |
| 34 | 2.4 |
| 35 | 3.8 |
| 36 | 4 |
| 37 | 100 |
| 38 | 100 |
| 39 | 100 |
| 40 | 100 |
| 41 | 0.9 |
| 42 | 1 |
| 43 | 100 |
| 44 | 100 |
| 45 | 30 |
| 46 | 1 |
| 47 | 100 |
| 52 | 3 |
| 56 | 12 |
| 57 | 100 |
| 62 | 100 |
| 66 | 100 |

**Table 3 Lipid Peroxidation Inhibition Data**

| **Compound No. (10 uM)** | **% Inhibition** |
|---|---|
| PQQ* (100 uM) | 93.7 |
| 1 | 90.8 |
| 2 | 81.0 |
| 3 | 77.8 |
| 4 | 91.4 |
| 5 | 86.3 |
| 6 | 89.0 |
| 7 | 82.9 |
| 8 | 81.6 |
| 9 | 87.9 |
| 10 | 85.8 |
| 11 | 82.7 |
| 12 | 92.1 |
| 13 | 93.4 |
| 14 | 95.2 |
| 15 | 94.6 |
| 17 | 0.4 |
| 20 | 90.2 |
| 21 | 94.1 |
| 2-Methoxy estradiol | 92.3 |

| | |
|---|---|
| *PQQ = pyrroloquinoline quinone | |

**Table 3a Lipid Peroxidation Inhibition Data**

| Compound number | % Lipid Peroxidation inhibition (at 1 uM) |
|---|---|
| 1 | 74.1 |
| 4 | 87.0 |
| 5 | 71.0 |
| 6 | 70.5 |
| 9 | 57.2 |
| 13 | 82.9 |
| 14 | 88.6 |
| 15 | 88.0 |
| 21 | 83.8 |
| 24 | 37.0 |
| 25 | 42.0 |
| 27 | 53.4 |
| 29 | 92.3 |
| 33 | 54.9 |
| 35 | 64.4 |
| 39 | 44.8 |
| 40 | 46.8 |
| 41 | 81.8 |
| 42 | 83.8 |
| 43 | 88.5 |
| 44 | 83.3 |
| 45 | 91.5 |
| 46 | 80.1 |
| 47 | 60.3 |

### Neovascularization on the CAM and Microscopic Analysis of CAM Sections

In vivo neovascularization was examined by the method previously described by Auerbach et al. (J. Dev. Biol. 41:391-394 (1974)). Ten-day old chicken embryos were purchased from Spafas, Inc. (Preston, Conn.) and were incubated at 37°C with 55% relative humidity. In the dark, with the aid of a candling lamp, a small hole was punctured in the shell concealing the air sac with a hypodermic needle. A second hole was punctured in the shell on the broadside of the egg directly over an vascular portion of the embryonic membrane, as observed during candling. An artificial air sac was created beneath the second hole by applying gentle vacuum to the first hole using a small rubber squeeze bulb. The vacuum caused the chorioallantoic membrane (CAM) to separate from the shell.

A window, approximately 1.0 cm², was cut in the shell over the dropped CAM with the use of a small crafts grinding wheel (Dremel, Division of Emerson Electric Company Racine, WI). The window allowed direct access to the underlying CAM.

A pro-angiogenic agent was added to induce new blood vessel branches on the CAM of 10-day old embryos. Filter disks of #1 filter paper (Whatman International, United Kingdom) were punched using a small puncher and were soaked in 3 mg/mL cortisone acetate (Sigma, St. Louis, Mo.) in a solution (95% ethanol and water). The disks were subsequently air dried under sterile conditions. The disks were then suspended in PBS (Phosphate Buffered Saline) and placed on growing CAMs. Filters treated with TP-H (TEMPOL-H) or TEMPOL and/or H₂O₂ or TP-H and/or bFGF or VEGF were placed on the first day of the 3-day incubation.

For inducing angiogenesis, sterile filter disks were saturated with bFGF (1µg/ml) (Life Technologies, Gaithersburg, Md.) or other pro-angiogenesis factors and control disks were saturated with PBS without Calcium and Magnesium. Control disks were saturated with PBS without Calcium and Magnesium.

Using sterile forceps one filter/CAM was placed from the window. The window was sealed with Highland brand transparent tape.

After 24-48 hr, 10 -25 ul of test agent was injected intravenously or added topically into the CAM membrane. Eight - Ten eggs/treatment group were used.

CAM tissue directly beneath filter disk was harvested from embryos treated 48 hours prior with compound or control. Tissues were washed three times with PBS. Sections were placed in a 35-mm petri dish (Nalge Nunc, Rochester, N.Y.) and examined under a SV6 stereomicroscope (Karl Zeiss, Thornwood, N.Y.) at 50x magnification.

CAM sections from Petri dish were examined using SV6 stereomicroscope (Karl Zeiss) at 50X magnification. Digital images of CAM sections from Petri dish were collected using a 3-CCD color video camera system (Toshiba America, New York, N.Y.). These images were analyzed using Image-Pro Plus software (Media Cybernetics, Silver Spring, Md.).

The number of branch points in blood vessels within the circular region superimposed to the area of a filter disk was counted for each section. After incubation at 37°C with 55% relative humidity for 3 days, the CAM tissue directly beneath each filter disk was resected from control and treated CAM samples. Tissues were washed three times with PBS. Sections were placed in a 35-mm Petri dish (Nalge Nunc; Rochester, NY) and were examined under a SV6 stereomicroscope (Karl Zeiss; Thornwood, NY) at 50X magnification. Digital images of CAM sections adjacent to filters were collected using a 3-CCD color video camera system (Toshiba America; New York, NY) and analyzed with the Image-Pro Plus software (Media Cybernetics; Silver Spring, MD).

The number of vessel branch points contained in a circular region equal to the area of a filter disk was counted for each section. Percent inhibition data are expressed as the quotient of the experimental value minus the negative control value divided by the difference between the positive control value and the negative control value. One image was counted in each CAM preparation, and findings from eight CAM preparations were analyzed for each treatment condition. In addition, each experiment was performed three times. The resulting angiogenesis index is the mean ± SEM (Standard Error of Measurement) of new branch points in each set of treatment. Statistical analysis of blood vessel branching patterns are performed by 1-way analysis of variance (ANOVA) comparing experimental with corresponding control groups. Statistical significance differences are assessed at P value of < 0.05. Results are given in Table 4 and 4a.

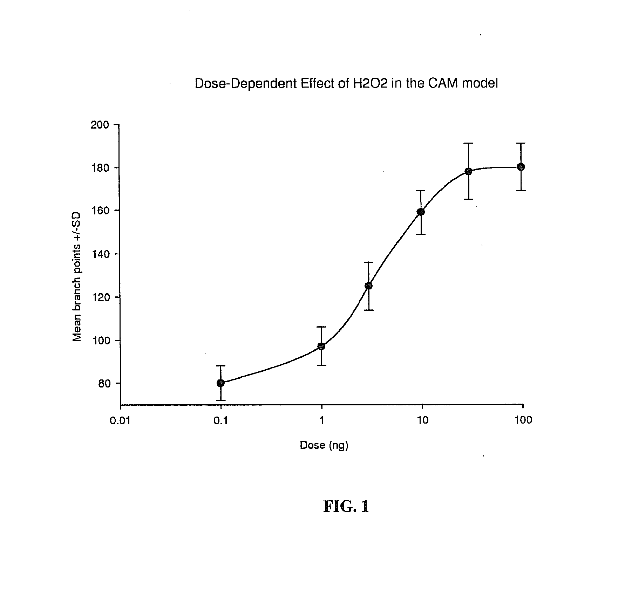

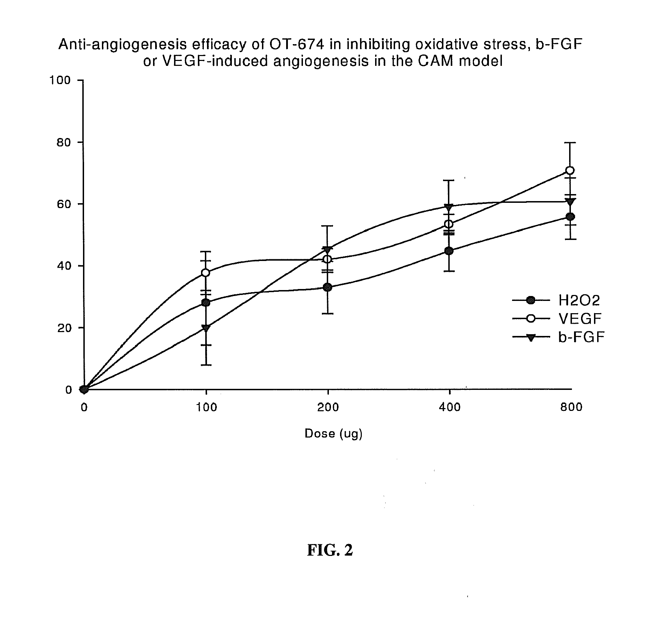

A dose dependent effect of H₂O₂ in the CAM model was observed for TEMPOL-H. This effect is depicted in FIG. 1. The anti-angiogenesis efficacy of TEMPOL-H inhibiting oxidative stress, b-FGF, and VEG-F induced angiogenesis in the CAM model is depicted in FIG. 2.

**Table 4 CAM data**

| **Compound No. (30 ug)** | **% Inhibition** |
|---|---|
| OT-551 HCl | 75.2 ± 9.7 |
| | 79.6 ± 7.9 |
| | 74±4 |
| TEMPOL-H HCl | 43 ± 10 |
| Vitamin E (300ug) | 22 ± 6 |
| Vitamin C (300ug) | 15±7 |
| 1 | 89.9 ± 18.0 |
| 2 | 76.4 ± 9.9 |
| 3 | 54.7 ± 7.9 |
| 4 | 92.8 ± 16.5 |
| 6 | 40.4 ± 9.3 |
| 7 | 124.2 ± 8.4 |
| 8 | 88.1 ± 7.6 |
| 9 | 82.2 ± 6.7 |
| 10 | 78.2 ± 9.9 |
| 11 | 71.0 ± 8.8 |
| 12 | 92.1 |
| 13 | 93.4 |
| 14 | 95.2 |
| 15 | 94.6 |
| 20 | 90.2 |
| 21 | 94.1 |
| 2-methoxyestradiol | 92.3 |

**Table 4a CAM data**

| Compound number | Angiogenesis in CAM model IC₅₀ at 30ug |
|---|---|
| 1 | 89.9 |
| 2 | 76.0 |
| 3 | 54.7 |
| 4 | 92.8 |
| 6 | 40.4 |
| 7 | 100 |
| 8 | 88.0 |
| 9 | 82.2 |
| 10 | 78.2 |
| 11 | 71.0 |
| 13 | 51.2 |
| 14 | 75.1 |
| 15 | 71.8 |
| 20 | 78.6 |
| 21 | 60.9 |
| 23 | 48.8 |
| 24 | 65.8 |
| 25 | 62.9 |
| 26 | 72.0 |
| 27 | 88.3 |
| 29 | 100 |
| 30 | 90.4 |
| 31 | 38.8 |
| 33 | 77.8 |
| 38 | 62.8 |
| 39 | 96.9 |
| 40 | 73.8 |
| 41 | 84.4 |
| 42 | 61.9 |
| 43 | 85.5 |
| 44 | 71.2 |
| 45 | 50.0 |
| 46 | 45.1 |
| 47 | 55.2 |
| 50 | 92.3 |
| 51 | 43.0 |
| 52 | 68.3 |
| 56 | 66.1 |
| 57 | 100 |
| 58 | 39.0 |
| 59 | 53.0 |
| 62 | 43.8 |
| 63 | 100 |
| 64 | 87.8 |

### Assessment of angiogenesis in the CAM model using bFGF stimulus.

The CAM model protocol was modified to include stimulus with bFGF. The effect of injected OT 551 was assayed. Results are displayed in Table 5.

**TABLE 5**

| **Treatment** | **Branch pts ± SEM** | **% Inhibition ± SEM** |
|---|---|---|
| FGF2 (1ug) + PBS | 147 ± 7.5 | |
| FGF2 (1ug) + PBS injected | 139 ± 8 | 9 ± 5 |
| FGF2 (1ug) + OT-551 (30ug) injected | 87 ± 3 | 74 ± 4 |

The effect of OT-551 nanoparticles in LPS, angiotension II, and Bradykini-stimulated CAM model is depicted in Table 6.

**TABLE 6**

| **Treatment** | **Branch pts ± SEM** | **% Inhibition ± SEM** |
|---|---|---|
| PBS | 45.6 ± 2.9 | |
| LPS (5 ug/mL) | 106 ± 9.3 | |
| | | |
| LPS + OT-551 nanoparticle-PLGA (30 ug) | 58.8 ± 11.7 | 76.9 ± 19.1 |
| Angiotension II (5 ug/mL) | 103.2 ± 25.93 | |
| | | |
| Angiotension II + OT-551 nanoparticle-PLGA (30 ug) | 74.8 ± 9.2 | 48.5 ± 15.6 |
| Bradykinin (5 ug/mL) | 106.7 ± 4.8 | |
| | | |
| Bradykinin + OT-551 nanoparticle-PLGA (30 ug) | 61 ± 8.4 | 73.6 ± 19.2 |

### Evaluation of Chemoresistance

A method for evaluating a compound's propensity to overcome cancer cell chemoresistance is described in "Caspase Inhibition Switches Doxorubicin-Induced Apoptosis To Senescence", Abdelhadi Rebbaa, Xin Zheng, Pauline M Chou and Bernard L Mirkin, Oncogene (2003) 22, 2805-2811. Resistant human cancer cells to doxorubicin were selected by stepwise exposure to drug concentrations ranging from 10⁻⁹ M-10⁻⁶ M over 3 months. They were then subjected to treatment with the doxorubicin alone or in combination with hydroxylamine compounds 1-12, 16, 20, and 21 (Table 4). The cells were incubated with the drugs for 72 hours, and cell viability was measured by the MTT assay. Additionally, molecular pathways that might be associated with increased chemo-responsiveness with these compounds were investigated.

Cancer Cell Viability Data for certain hydroxylamines in the presence or absence of Doxorubicin is listed in Table 4. These data show the effect on drug resistance of a series of hydroxylamines that apear capable of targeting simultaneously the survival pathways mediated by NFkB, the oxidative stress mediated by NADH oxidase, and angiogenesis. Cellular treatment with Compound 4 alone enhanced the killing of both doxorubicin-sensitive (P<0.001) and doxorubicin-resistant cells (P<0.001), suggesting that these inhibitors are able to bypass drug resistance. When combined with doxorubicin, Compound 4 displayed a strong ability to reverse drug resistance in osteosarcoma, breast cancer, and neuroblastoma. Similar reversal of chemoresistance with Compound 4 was shown with other chemotherapeutic agents. The underlying mechanism appeared to be mediated through acceleration of cell cycle arrest and induction of apoptosis, as evidenced by increased expression of p21/WAF1 and caspase-3 activation.

A method for evaluating a compound's propensity to overcome cancer cell chemoresistance is described in "Caspase Inhibition Switches Doxorubicin-Induced Apoptosis To Senescence", Abdelhadi Rebbaa, Xin Zheng, Pauline M Chou and Bernard L Mirkin, Oncogene (2003) 22, 2805-2811. Human neuroblastoma SKN-SH cells (ATCC Cat. No. HTB-11) were cultured in Dulbecco's Modified Eagles Medium (DMEM; Gibco, Grand Island, New York) supplemented with 10% fetal bovine serum (FBS; Sigma-Aldrich, St. Louis, MO) at 37°C in a 95% Air / 5% CO₂ atmosphere. Resistant human cancer cells to doxorubicin were selected by stepwise exposure to drug concentrations ranging from 10⁻⁹M-10⁻⁶M over 3 months. They were then subjected to treatment with the doxorubicin alone or in combination with hydroxylamine compounds. The cells were incubated with the drugs for 72 hours, and cell viability was measured by the MTT assay. This consists of adding 10 µl/well of MTT (5 mg/ml solution) and incubation for 4h at 37°C. The precipitate formed is then solubilized by addition of 100 µl of HCL 0.5 N/Isopropanol and incubation for 15 hours at 37°C. The optical density is measured at 570 nm and cell survival is estimated by comparison to untreated cells. Each point represents 4 wells data represent average +/- SE. Additionally, molecular pathways that might be associated with increased chemo-responsiveness with these compounds were investigated.

Cytotoxic activity of doxorubicin and hydroxylamine analogs were quantitatively determined by a colorimetric assay utilizing 3-(4, 5-dimethyl-2-thiazolyl) 2, 5-diphenyl tetrazolium bromide (MTT; Sigma-Aldrich, St. Louis, MO). Briefly, cells were seeded at 10⁴ cells/well in 96-well plates and maintained in culture for 24 hours at 37°C in DMEM supplemented with 10% FBS. Drugs were added to designated wells and cells were incubated for 96 hours, following which MTT (10 µL of 5 mg/ml solution) was added to each 100 µl well and incubated for 4 hours at 37°C. The cells were solubilized by incubation with 100 µl of HCl 0.5N in isopropanol for 15 hours at 37°C. The optical density of this solution was measured at 570 nm and the percentage of viable cells estimated by comparison with untreated control cells.

Cancer Cell Viability Data for certain hydroxylamines in the presence or absence of Doxorubicin is listed in Table 7. These data show the effect on drug resistance of a series of hydroxylamines that apear capable of targeting simultaneously the survival pathways mediated by NFkB, the oxidative stress mediated by NADH oxidase, and angiogenesis. Cellular treatment with Compound 4 alone enhanced the killing of both doxorubicin-sensitive (P<0.001) and doxorubicin-resistant cells (P<0.001), suggesting that these inhibitors are able to bypass drug resistance. When combined with doxorubicin, Compound 4 displayed a strong ability to reverse drug resistance in osteosarcoma, breast cancer, and neuroblastoma. Similar reversal of chemoresistance with Compound 4 was shown with other chemotherapeutic agents. The underlying mechanism appeared to be mediated through acceleration of cell cycle arrest and induction of apoptosis, as evidenced by increased expression of p21/WAF1 and caspase-3 activation.

**Table 7 Chemoresistance Data: Effect of Compounds on Cancer Cell Viability in the presence or absence of Doxorubicin**

| **Compound No. (50 ug/mL)** | **Dox. (10⁻⁶ M)** | **Mean OD (x10⁻³)** | **+/- SEM** |
|---|---|---|---|
| Control | - | 800.25 | 6.17 |
| | + | 339.00 | 18.7 |
| 1 | - | 524.75 | 46.71 |
| | + | 246.50 | 34.61 |
| 2 | - | 496.33 | 10.21 |
| | + | 249.25 | 18.54 |
| 3 | - | 640.25 | 18.80 |
| | + | 289.50 | 2.55 |
| 4 | - | 468.50 | 18.65 |
| | + | 88.00 | 10.31 |
| Control | - | 758.25 | 6.25 |
| | + | 307.75 | 22.36 |
| 5 | - | 556.50 | 20.48 |
| | + | 245.50 | 15.50 |
| 6 | - | 367.00 | 19.00 |
| | + | 185.25 | 4.21 |
| 7 | - | 540.25 | 26.44 |
| | + | 241.25 | 9.82 |
| 8 | - | 392.50 | 14.31 |
| | + | 197.25 | 17.99 |
| 9 | - | 509.00 | 12.49 |
| | + | 234.75 | 2.72 |
| 10 | - | 456.00 | 15.24 |
| | + | 250.00 | 17.27 |
| 11 | - | 470.25 | 9.29 |
| | + | 267.25 | 11.71 |
| Control | - | 829.50 | 18.84 |
| | + | 383.50 | 7.14 |
| 12 | - | 539.25 | 20.28 |
| | + | 287.25 | 10.04 |
| Control | - | 824.75 | 35.32 |
| | + | 446.75 | 21.43 |
| 20 | - | 515.50 | 29.24 |
| | + | 107.5 | 3.07 |
| 21 | - | 492.75 | 19.78 |
| | + | 99.25 | 9.48 |
| Control | - | 1426.25 | 14.76 |
| | + | 421.50 | 22.02 |
| 23 | - | 1432.50 | 12.31 |
| | + | 416.25 | 6.56 |
| 24 | - | 1166.50 | 5.44 |
| | + | 362.00 | 11.42 |
| 25 | - | 1137.50 | 2.22 |
| | + | 325.00 | 11.50 |
| 26 | - | 1056.33 | 34.97 |
| | + | 261.50 | 12.73 |
| 27 | - | 1038.33 | 56.26 |
| | + | 403.75 | 13.68 |
| 28 | - | 1253.75 | 58.14 |
| | + | 162.25 | 8.12 |
| 29 | - | 1328.00 | 60.36 |
| | + | 105.75 | 9.03 |
| Ctl | - | 1387.25 | 59.06 |
| | + | 384.00 | 10.68 |
| 30 | - | 1301.25 | 30.18 |
| | + | 310.00 | 22.73 |
| 31 | - | 1175.25 | 59.53 |
| | + | 322.25 | 11.21 |
| 32 | - | 985.67 | 17.57 |
| | + | 231.50 | 5.95 |
| 33 | - | 1237.50 | 65.29 |
| | + | 300.00 | 20.75 |
| 34 | - | 1168.25 | 35.42 |
| | + | 302.75 | 26.14 |
| 35 | - | 1128.00 | 11.46 |
| | + | 352.75 | 11.18 |
| 36 | - | 1110.75 | 9.28 |
| | + | 297.00 | 7.33 |
| Ctl | - | 1334.25 | 39.97 |
| | + | 381.00 | 28.47 |
| 37 | - | 1329.75 | 23.98 |
| | + | 366.25 | 21.28 |
| 38 | - | 1202.75 | 36.55 |
| | + | 378.50 | 11.95 |
| 39 | - | 1201.00 | 44.91 |
| | + | 311.00 | 21.22 |
| 40 | - | 1177.25 | 64.03 |
| | + | 338.75 | 19.63 |
| 41 | - | 824.75 | 41.20 |
| | + | 261.25 | 27.15 |
| 42 | - | 1068.67 | 22.19 |
| | + | 311.00 | 30.95 |
| 43 | - | 1159.00 | 84.89 |
| | + | 324.67 | 12.24 |
| Ctl | - | 1363.25 | 69.12 |
| | + | 477.00 | 6.94 |
| 44 | - | 23.00 | 7.15 |
| | + | 13.25 | 1.44 |
| 45 | - | 1063.33 | 34.19 |
| | + | 276.50 | 10.60 |
| 46 | - | 79.00 | 15.26 |
| | + | 32.75 | 3.30 |
| 47 | - | 1168.33 | 15.30 |
| | + | 261.00 | 28.10 |
| 48 | - | 1118.00 | 30.02 |
| | + | 50.67 | 12.20 |
| 49 | - | 1330.25 | 62.79 |
| | + | 195.00 | 32.19 |
| 50 | - | 1226.33 | 53.35 |
| | + | 54.50 | 12.84 |
| Ctl | - | 1364.25 | 49.40 |
| | + | 447.75 | 24.62 |
| 51 | - | 1208.25 | 41.98 |
| | + | 71.00 | 5.82 |
| 52 | - | 1186.33 | 68.86 |
| | + | 195.00 | 9.61 |
| 53 | - | 1333.75 | 42.83 |
| | + | 170.00 | 9.16 |
| 54 | - | 1309.00 | 62.56 |
| | + | 65.50 | 11.57 |
| 55 | - | 1037.25 | 38.02 |
| | + | 285.75 | 12.80 |
| 56 | - | 1153.00 | 44.60 |
| | + | 308.75 | 9.68 |
| 57 | - | 1078.50 | 23.21 |
| | + | 426.25 | 31.98 |
| Ctl | - | 1412.50 | 30.97 |
| | + | 562.75 | 60.08 |
| 58 | - | 1207.00 | 19.47 |
| | + | 291.50 | 21.03 |
| 59 | - | 1105.00 | 25.53 |
| | + | 352.00 | 20.42 |
| 60 | - | 1300.25 | 46.76 |
| | + | 507.00 | 35.71 |
| 61 | - | 1366.75 | 21.27 |
| | + | 576.33 | 64.96 |
| 62 | - | 184.75 | 22.96 |
| | + | 40.25 | 9.78 |
| 63 | - | 382.75 | 40.64 |
| | + | 54.50 | 17.65 |
| 64 | - | 1051.00 | 33.51 |
| 64 | + | 153.50 | 10.41 |

As shown herein, OT-551 HCl is an agent with antioxidant and anti-angiogenic activity that has good permeability through the cornea and achieves good levels in the retina after topical administration. It is believed that systemic or topical administration of OT-551 can reduce retinal cell death in persons afflicted or like to develop retinitis pigmentosa. Scotopic and photopic ERGs will be done on 35 *rd10* mice and then 5 mice will be euthanized and the outer nuclear layer will be measured in one eye and cone density will be quantified in the fellow eye. The remainder of the mice will be divided into 3 groups. Group 1 (n=10) will be given daily intraperitoneal injections of 100 mg/kg of OT-551. (2) Group 2 (n=10) will be given daily intraperitoneal injections of vehicle. (3) Group 3 (n=10) will be given 3% eye drops of OT-551 three times a day in one eye and vehicle in the fellow eye. Scotopic and photopic ERGs will be done at P25 and then 5 mice in each group will be euthanized and the outer nuclear layer will be measured in one eye and cone density will be quantified in the fellow eye. The remaining 5 mice in each group will continue treatment and ERGs will be done at P35 after which the mice will be euthanized and the outer nuclear layer will be measured in one eye and cone density will be quantified in the fellow eye. Data are expected to exhibit the following pattern, showing efficacy of the compound in potentiating cone cell death.

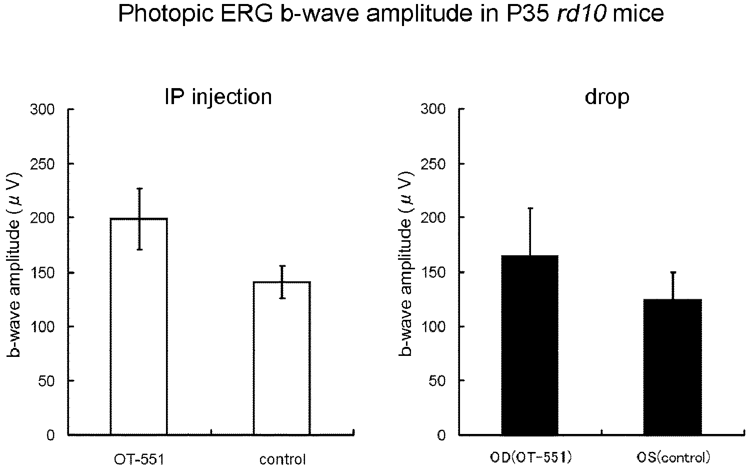

The synthetic methods and biological assays noted herein are easily practiced by those skilled in the art directed as above. Additional information regarding methods, analysis, and assays may be found, for example in the following articles, each incorporated by reference herein in its entirety: Endocrinology. 2006; 147(4):1602-7; J Cardiovascular Pharmacology 2005; 45(5): 462-467; Circulation Research 2004; 94(11):1500-1506; Anticancer Res. 2005; 25(1A):197-206; Cancer Research 63: 2020-2023, 2003; J Thrombosis Haemostasis 1 (1); 164-173, 2003; Clin Exp Metastasis 19(2):145-153, 2002.

In the foregoing specification, the concepts have been described with reference to specific embodiments. Many aspects and embodiments have been described above and are merely exemplary and not limiting. After reading this specification, skilled artisans appreciate that other aspects and embodiments are possible without departing from the scope of the invention. Moreover, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

When ranges are used herein for physical properties, such as molecular weight, or chemical properties, such as chemical formulae, all combinations and subcombinations of ranges and specific embodiments therein are intended to be included.

The disclosures of each patent, patent application and publication cited or described in this document are hereby incorporated herein by reference, in their entirety.

Those skilled in the art will appreciate that numerous changes and modifications can be made to the preferred embodiments of the invention and that such changes and modifications can be made without departing from the spirit of the invention. It is, therefore, intended that the appended claims cover all such equivalent variations as fall within the true spirit and scope of the invention.

## Claims

1. A compound of formula I: 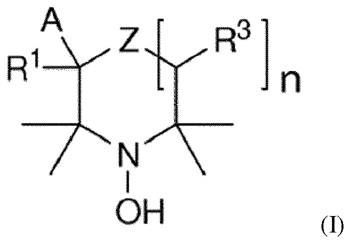 or a pharmaceutically acceptable salt thereof, wherein
A and B are each H;
Z is -C(B)(R²)-;
R¹ and R³ are each independently H;
R² is -OR⁴, -N(R⁵)R⁶, or C[(R⁷)(R⁸)]ₘR⁹;
m is 1 and n is 1;
R⁴ is 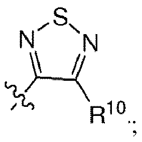 R⁵ is H;
R⁶ is alkyl, 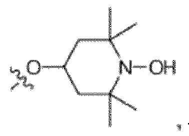 -C(=O)-R¹¹, or -S(=O)₂-R¹¹;
R⁷ and R⁸ is H;
R⁹ is -OH, -CH₂-C(=O)-morpholin-4-yl; or -N(R⁵)(R⁶);
R¹⁰ is H, morpholinyl, halo, or 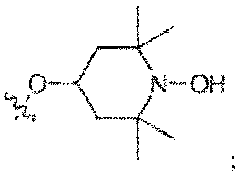 and
R¹¹ is alkyl, cycloalkyl, -NH(3,5-di-tertiary-butyl-4-hydroxyphenyl), -NH-(4,5-dihydroxy-2-methylphenyl), or

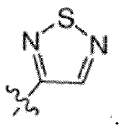

2. A compound according to claim 1 that is 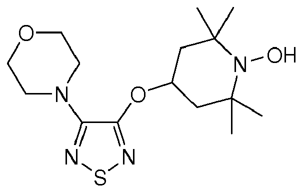 4-(4-(2,2,6,6-tetramethylpiperidin-1-hydroxyl-4-yloxy)-1,2,5-thiazol-3-yl)morpholine (Compound 4); 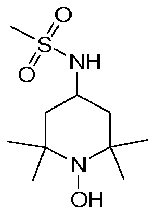 1-hydroxy-4-methanesulfonamido-2,2,6,6-tetramethylpiperidine (compound 10); 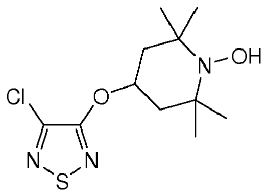 4-(4-chloro-1,2,5-thiadiazol-3-yloxyl)-1-hydroxyl-2,2,6,6-tetramethylpiperidine (compound 13); 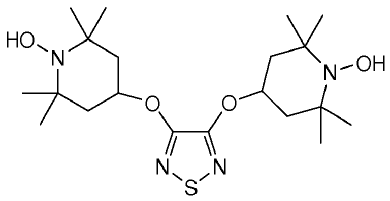 1-hydroxyl-4-(4(2,2,6,6-tetramethylpiperidin-1-hydroxyl-4-yloxy)-1,2,5-thiadiazol-3-yloxy)-2,2,6,6-tetramethylpiperidine (compound 14);
or 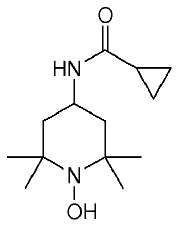 N-(1-hydroxy-2,2,6,6-tetramethylpiperidin-4-yl)cyclopropanecarboxamide (compound 31).

3. A compound according to claim 1 that is 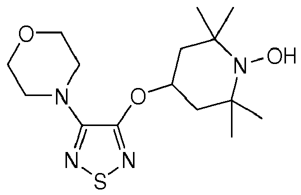 4-(4-(2,2,6,6-tetramethylpiperidin-1-hydroxyl-4-yloxy)-1,2,5-thiazol-3-yl)morpholine (Compound 4).
